# EUROPEAN PATENT SPECIFICATION

(11) **EP 2 336 365 B1**
(45) Date of publication and mention of the grant of the patent: **15.10.2014**
(21) Application number: 10181604.9
(22) Date of filing: 04.11.1997
(51) Int. Cl.: C12Q 1/68

(54) **L. monocytogenes specific DNA probes and amplification primers to rapidly detect and identify in clinical specimens**
L. monocytogenes spezifische DNA-Sonden und Amplifikationsprimer zur schnellen Detektion und Identifizierung in klinischen Proben
Sondes d'ADN spécifiques de L. monocytogenes et amorces d'amplification pour détecter dans les spécimens cliniques

(30) Priority: 04.11.1996 US 743637
(43) Date of publication of application: 22.06.2011
(62) Divisional of application: 09163281.0
(73) Proprietor: Geneohm Sciences Canada, Inc., Sainte-Foy, QC G1V 2K8 (CA)
(72) Inventor: Bergeron, Michel G., Sillery Québec G1T 1G2 (CA); Picard, François, Cap-Rouge Québec G1Y 1A1 (CA); Ouellette, Marc, Sillery Québec G1S 3S1 (CA); Roy, Paul H., Loretteville Québec G2A 2X8 (CA)
(74) Representative: Pohlman, Sandra M.

(56) References cited:
- EP-A- 0 761 815
- EP-A2- 0 314 294
- WO-A-96/08582
- WO-A-96/18745
- WO-A1-89/06699
- FR-A- 2 699 539
- US-A- 5 523 205
- FLEISCHMANN R D ET AL: "WHOLE-GENOME RANDOM SEQUENCING AND ASSEMBLY OF HAEMOPHILUS INFLUENZAE RD", SCIENCE, vol. 269, no. 5223, 28 July 1995 (1995-07-28), page 496, XP000517090,
- QUELETTE M. ET AL.,: "Precise insertion of antibiotic resistance determinants into Tn21-like transposons: nucleotide sequence of the OXA-1 b-lactamase gene", PROC: NATL. ACAD. SCI. USA, vol. 84, November 1987 (1987-11), pages 7378-7382, XP002066855,
- EVERS S. ET AL.,: "Sequence of the vanB and ddl genes encoding D-alanine:D-lactate and D-alanine ligases in vancomycin-resistant Enterococcus faecalis V583", GENE, vol. 140, 1994, pages 97-102, XP002066856,
- DUTKA-MALEN S. ET AL.,: "Sequence of the vanC gene of Enterococcus gallinarum BM4174 encoding a D-alanine:D-alanine ligase related protein necessary for vancomycin resistance", GENE, vol. 112, 1992, pages 53-58, XP002066857, & DATABASE EMPRO EMBL;
- LOECHEL S. ET AL.,: "Nucleotide sequence of the tuf gene from Mycoplasma genitalium", NUCLEIC ACID RESEARCH, vol. 17, no. 23, 1989, page 10127, XP002066858, & DATABASE EMPRO EMBL;
- PORCELLA S. ET AL.,: "Identification of an EF-Tu protein that is priplasm-associated and processed in Neisseria gonorrhoeae", MICROBIOLOGY, vol. 142, September 1996 (1996-09), pages 2481-2489, XP002066859,
- BREMAUD L. ET AL.,: "genetic and molecular analysis of the tRNA-tufB operon of the mycobactreium Stigmatella aurantiaca", NUCLEIC ACID RESEARCH, vol. 23, no. 10, 1995, pages 1737-1743, XP002067242,
- DATABASE EMPRO EMBL; 2 July 1986 (1986-07-02), MURPHY E. ET AL.,: XP002067252,
- EAST A.K. & DYKE K.G.H.: "Cloning and sequence dtermination of six staphylococcus aureus b-lactamases and their expression in E. coli and S. aureus", J. GEN. MICROBIOL., vol. 135, 4 April 1989 (1989-04-04), pages 1001-1015, XP002067243,
- BRISSON-NOEL A. ET AL.,: "Evidence for natural gene transfer from gram-positive cocci to E. coli", J. BACTERIOL, vol. 170, April 1988 (1988-04), pages 1739-1745, XP002067244,
- AN G. & FRIESEN J.D.: "The nucleotide sequence of tufB and four nearby tRNA structural genes of E. coli", GENE, vol. 12, December 1980 (1980-12), pages 33-39, XP002067245,
- OHAMA T. ET AL.,: "Organization and codon usage of the streptomycin operon in micrococcus luteus, a bacterium with a high genomic G+C content", J. BACTERIOL., vol. 169, October 1987 (1987-10), pages 4770-4777, XP002067246,
- MONOD M. ET AL.,: "Sequence and properties of pIM13, a macrolide-lincosamide-steptogramin B resistance plasmid from bacillus subtilis", J. BACTERIOL., vol. 167, July 1986 (1986-07), pages 138-147, XP002067247,
- CARLIN N. ET AL.,: "Monoclonal antibodies specific for elongation factor Tu and complete nucleotide sequence of the tuf gene in M. tuberculosis", INFECT. IMMUN., vol. 60, August 1992 (1992-08), pages 3136-3142, XP002067248,
- ZHANG Y-X. ET AL.,: "Cloning, sequencing, and expression in E. coli of the gene encoding a 45-kilodalton protein, elongation factor Tu, from chlamydia trachomatis serovar F", J. BACTERIOL, vol. 176, no. 4, February 1994 (1994-02), pages 1184-1187, XP002067249,
- DATABASE EMPRO embl; 13 August 1995 (1995-08-13), PERLEE L. & SCHWARTZ I.: XP002067253,
- DATABASE EMPRO EMBL; 27 October 1996 (1996-10-27), YOSHIKAWA H.: XP002067254,
- DATABASE EMPRO EMBL; thesis 8 June 1994 (1994-06-08), KAMLA V.: XP002067255,
- ANDERSSON S.G.E.: "Unusual organization of the rRNA genes in Rickettsia prowazekii", J. BACTERIOL., vol. 177, no. 4, July 1995 (1995-07), pages 4171-4175, XP002067250,
- SHAW K.J. ET AL.,: "Isolation, characterization and DNA sequence analysis of an AAC(6')_II gene from Pseudomonas aeruginosa", ANTIMICROBIAL. AGNETS AND CHEMOTHERAPY, vol. 33, no. 12, December 1989 (1989-12), pages 2052-2062, XP002067251,
- DATABASE EMPRO EMBL; LUDWIG W. ET AL.,: "Phylogenetic relationships of bacteria based on comparative sequence analysis of elongation factor Tu and ATP-synthase-beta subunit genes; Antonie van leeuwenhoek 64; 285-305 (1993)", XP002067256,
- DOMANN E ET AL: "A NOVEL BACTERIAL VIRULENCE GENE IN LISTERIA MONOCYTOGENES REQUIREDFOR HOST CELL MICROFILAMENT INTERACTION WITH HOMOLOGY TO THE PROLINE-RICH REGION OF VINCULIN", EMBO JOURNAL, OXFORD UNIVERSITY PRESS, SURREY, GB, vol. 11, no. 5, 1 January 1992 (1992-01-01), pages 1981-1990, XP002071288, ISSN: 0261-4189
- SCHWAN WILLIAM R ET AL: "Detection and characterization by differential PCR of host eukaryotic cell genes differentially transcribed following uptake of intracellular bacteria", INFECTION AND IMMUNITY, vol. 64, no. 1, 1 January 1996 (1996-01-01), pages 91-99, XP002633365, ISSN: 0019-9567

## Description

### BACKGROUND OF THE INVENTION

### Classical methods for the identification and susceptibility testing of bacteria

Bacteria are classically identified by their ability to utilize different substrates as a source of carbon and nitrogen through the use of biochemical tests such as the API20E™ system (bioMérieux). For susceptibility testing, clinical microbiology laboratories use methods including disk diffusion, agar dilution and broth microdilution. Although identifications based on biochemical tests and antibacterial susceptibility tests are cost-effective, at least two days are required to obtain preliminary results due to the necessity of two successive overnight incubations to identify the bacteria from clinical specimens as well as to determine their susceptibility to antimicrobial agents. There are some commercially available automated systems (i.e. the MicroScan system from Dade Diagnostics Corp. and the Vitek system from bioMérieux) which use sophisticated and expensive apparatus for faster microbial identification and susceptibility testing (Stager and Davis, 1992, Clin. Microbiol. Rev. 5:302-327). These systems require shorter incubation periods, thereby allowing most bacterial identifications and susceptibility testing to be performed in less than 6 hours. Nevertheless, these faster systems always require the primary isolation of the bacteria as a pure culture, a process which takes at least 18 hours for a pure culture or 2 days for a mixed culture. The fastest identification system, the autoSCAN-Walk-Away™ system (Dade Diagnostics Corp.) identifies both gram-negative and gram-positive bacterial species from standardized inoculum in as little as 2 hours and gives susceptibility patterns to most antibiotics in 5.5 hours. However, this system has a particularly high percentage (i.e. 3.3 to 40.5%) of non-conclusive identifications with bacterial species other than *Enterobactericaceae* (Croizé J., 1995, Lett. Infectiol. 10:109-113; York et al., 1992, J. Clin. Microbiol. 30:2903-2910). For *Enterobacteriaceae,* the percentage of non-conclusive identifications was 2.7 to 11.4%.

A wide variety of bacteria and fungi are routinely isolated and identified from clinical specimens in microbiology laboratories. Tables 1 and 2 give the incidence for the most commonly isolated bacterial and fungal pathogens from various types of clinical, specimens. These pathogens are the most frequently associated with nosocomial and community-acquired human infections and are therefore considered the most clinically important.

### Clinical specimens tested in clinical microbiology laboratories

Most clinical specimens received in clinical microbiology laboratories arc urine and blood samples. At the microbiology laboratory of the Centre Hospitalier de l'Université Laval (CHUL), urine and blood account for approximately 55% and 30% of the specimens received, respectively (Table 3). The remaining 15% of clinical specimens comprise various biological fluids including sputum, pus, cerebrospinal fluid, synovial fluid, and others (Table 3). Infections of the urinary tract, the respiratory tract and the bloodstream are usually of bacterial etiology and require antimicrobial therapy. In fact, all clinical samples received in the clinical microbiology laboratory are tested routinely for the identification of bacteria and susceptibility testing.

### Conventional pathogen identification from clinical specimens

### Urine specimens

The search for pathogens in urine specimens is so preponderant in the routine microbiology laboratory that a myriad of tests have been developed. However, the gold standard remains the classical semi-quantitative plate culture method in which 1 µL of urine is streaked on plates and incubated for 18-24 hours. Colonies are then counted to determine the total number of colony forming units (CFU) per liter of urine. A bacterial urinary tract infection (UTI) is normally associated with a bacterial count of 10⁷ CFU/L or more in urine. However, infections with less than 10⁷ CFU/L in urine are possible, particularly in patients with a high incidence of diseases or those catheterized (Stark and Maki, 1984, N. Engl. J. Med. 311:560-564). Importantly, approximately 80% of urine specimens tested in clinical microbiology laboratories are considered negative (i.e. bacterial count of less than 10⁷ CFU/L; Table 3). Urine specimens found positive by culture are further characterized using standard biochemical tests to identify the bacterial pathogen and are also tested for susceptibility to antibiotics. The biochemical and susceptibility testing normally require 18-24 hours of incubation.

Accurate and rapid urine screening methods for bacterial pathogens would allow a faster identification of negative specimens and a more efficient treatment and care management of patients. Several rapid identification methods (Uriscreen™, UTIscrceen™, Flash Track™ DNA probes and others) have been compared to slower standard biochemical methods, which are based on culture of the bacterial pathogens. Although much faster, these rapid tests showed low sensitivities and poor specificities as well as a high number of false negative and false positive results (Koening et al., 1992, J. Clin. Microbiol. 30:342-345; Pezzlo et al., 1992, J. Clin. Microbiol. 30:640-684).

### Blood specimens

The blood specimens received in the microbiology laboratory arc always submitted for culture. Blood culture systems may be manual, semi-automated or completely automated. The BACTEC system (from Becton Dickinson) and the BacTAlert system (from Organon Teknika Corporation) are the two most widely used automated blood culture systems. These systems incubate blood culture bottles under optimal conditions for bacterial growth. Bacterial growth is monitored continuously to detect early positives by using highly sensitive bacterial growth detectors. Once growth is detected, a Gram stain is performed directly from the blood culture and then used to inoculate nutrient agar plates. Subsequently, bacterial identification and susceptibility testing are carried out from isolated bacterial colonies with automated systems as described previously. The bottles are normally reported as negative if no growth is detected after an incubation of 6 to 7 days. Normally, the vast majority of blood cultures are reported negative. For example, the percentage of negative blood cultures at the microbiology laboratory of the CHUL for the period February 1994-January 1995 was 93.1 % (Table 3).

### Other clinical samples

Upon receipt by the clinical microbiology laboratory, all body fluids other than blood and urine that are from normally sterile sites (i.e. cerebrospinal, synovial, pleural, pericardial and others) are processed for direct microscopic examination and subsequent culture. Again, most clinical samples are negative for culture (Table 3).

Regarding clinical specimens which are not from sterile sites such as sputum or stool specimens, the laboratory diagnosis by culture is more problematic because of the contamination by the normal flora. The bacterial pathogens potentially associated with the infection are purified from the contaminants and then identified as described previously. Of course, the universal detection of bacteria would not be useful for the diagnosis of bacterial infections at these non sterile sites. On the other hand, DNA-based assays for species or genus detection and identification as well as for the detection of antibiotic resistance genes from these specimens would be very useful and would offer several advantages over classical identification and susceptibility testing methods.

### DNA-based assays with any clinical specimens

There is an obvious need for rapid and accurate diagnostic tests for bacterial detection and identification directly from clinical specimens. DNA-based technologies are rapid and accurate and offer a great potential to improve the diagnosis of infectious diseases (Persing et al., 1993, Diagnostic Molecular Microbiology: Principles and Applications, American Society for Microbiology, Washington, D.C.). The DNA probes and amplification primers which are described and/or claimed herein are applicable for bacterial or fungal detection and identification directly from any clinical specimens such as blood cultures, blood, urine, sputum, cerebrospinal fluid, pus and other type of specimens (Table 3). The DNA-based tests proposed in this invention are superior in terms of both rapidity and accuracy to standard biochemical methods currently used for routine diagnosis from any clinical specimens in microbiology laboratories. Since these tests are performed in around only one hour, they provide the clinicians with new diagnostic tools which should contribute to increase the efficiency of therapies with antimicrobial agents. Clinical specimens from organisms other than humans (e.g. other primates, birds, plants, mammals, farm animals, livestock and others) may also be tested with these assays.

### A high percentage of culture negative specimens

Among all the clinical specimens received for routine diagnosis, approximately 80% of urine specimens and even more (around 95%) for other types of clinical specimens are negative for the presence of bacterial pathogens (Table 3). It would also be desirable, in addition to identify bacteria at the species or genus level in a given specimen, to screen out the high proportion of negative clinical specimens with a test detecting the presence of any bacterium (i.e. universal bacterial detection). Such a screening test may be based on the DNA amplification by PCR of a highly conserved genetic target found in all bacteria. Specimens negative for bacteria would not be amplified by this assay. On the other hand, those that are positive for bacteria would give a positive amplification signal with this assay.

### Towards the development of rapid DNA-based diagnostic tests

A rapid diagnostic test should have a significant impact on the management of infections. DNA probe and DNA amplification technologies offer several advantages over conventional methods for the identification of pathogens and antibiotic resistance genes from clinical samples (Persing et al., 1993, Diagnostic Molecular Microbiology: Principles and Applications, American Society for Microbiology, Washington, D.C.; Ehrlich and Greenberg, 1994, PCR-based Diagnostics in Infectious Disease, Blackwell Scientific Publications, Boston, MA). There is no need for culture of the bacterial pathogens, hence the organisms can be detected directly from clinical samples, thereby reducing the time associated with the isolation and identification of pathogens. Furthermore, DNA-based assays are more accurate for bacterial identification than currently used phenotypic identification systems which are based on biochemical tests. Commercially available DNA-based technologies are currently used in clinical microbiology laboratories, mainly for the detection and identification of fastidious bacterial pathogens such as *Mycobacterium tuberculosis, Chlamydia trachomatis, Neisseria gonorrhoeae* as well as for the detection of a variety of viruses (Podzorski and Persing, Molecular detection and identification of microorganisms, In : P. Murray et al., 1995, Manual of Clinical Microbiology, ASM press, Washington D.C.). There are also other commercially available DNA-based assays which are used for culture confirmation assays.

Others have developed DNA-based tests for the detection and identification of bacterial pathogens: *Staphylococcus* spp. (US patent application serial No. US 5 437 978), *Neisseria* spp. (US patent application serial No. US 5 162 199 and European patent application serial No. EP 0 337 896 131) and *Listeria monocytogenes* (US patent applications serial Nos US 5 389 513 and US 5 089 386). However, the diagnostic tests described in these patents are based either on rRNA genes or on genetic targets different from thosc described in the present invention.

Although there are diagnostic kits or methods already used in clinical microbiology laboratories, there is still a need for an advantageous alternative to the conventional culture identification methods in order to improve the accuracy and the speed of the diagnosis of commonly encountered bacterial infections. Besides being much faster, DNA-based diagnostic tests are more accurate than standard biochemical tests presently used for diagnosis because the bacterial genotype (e.g. DNA level) is more stable than the bacterial phenotype (e.g. metabolic level).

Knowledge of the genomic sequences of bacterial and fungal species continuously increases as testified by the number of sequences available from databases. From the sequences readily available from databases, there is no indication therefrom as to their potential for diagnostic purposes. For determining good candidates for diagnostic purposes, one could select sequences for DNA-based assays for (i) the species-specific detection and identification of commonly encountered bacterial or fungal pathogens, (ii) the genus-specific detection and identification of commonly encountered bacterial or fungal pathogens, (iii) the universal detection of bacterial or fungal pathogens and/or (iv) the specific detection and identification of antibiotic resistance genes. All of the above types of DNA-based assays may be performed directly from any type of clinical specimens or from a microbial culture.

In our co-pending U.S. (N.S. 08/526,840) and PCT (PCT/CA/95/00528) patent applications, we described DNA sequences suitable for (i) the species-specific detection and identification of 12 clinically important bacterial pathogens, (ii) the universal detection of bacteria, and (iii) the detection of 17 antibiotic resistance genes. This co-pending application described proprietary DNA sequences and DNA sequences selected from databases (in both cases, fragments of at least 100 base pairs), as well as oligonucleotide probes and amplification primers derived from these sequences. All the nucleic acid sequences described in this patent application enter the composition of diagnostic kits and methods capable of a) detecting the presence of bacteria, b) detecting specifically the presence of 12 bacterial species and 17 antibiotic resistance genes. However, these methods and kits need to be improved, since the ideal kit and method should be capable of diagnosing close to 100% of microbial pathogens and antibiotic resistance genes. For example, infections caused by *Enterococcus faecium* have become a clinical problem because of its resistance to many antibiotics. Both the detection of these bacteria and the evaluation of their resistance profiles are desirable. Besides that, novel DNA sequences (probes and primers) capable of recognizing the same and other microbial pathogens or the same and additional antibiotic resistance genes are also desirable to aim at detecting more target genes and complement our earlier patent application.

### STATEMENT OF THE INVENTION

It is an object of the invention to provide a method for determining or detecting the presence and/or amount of one or more *tuf* nucleic acids from the *Listeria monocytogenes* bacterial species in a sample, said method comprising:
a) i) contacting said sample with at least one oligonucleotide having at least 12 nucleotides in length derived from and which hybridizes specifically to SEQ ID NO: 138 or a complementary sequence thereof, so as to perform an hybridization assay; or
   ii) contacting said sample with at least two oligonucleotides, wherein at least one oligonucleotide has at least 12 nucleotides in length and is derived from and hybridizes specifically to SEQ ID NO: 138 or a complementary sequence thereof, so as to perform an amplification reaction; and
b) detecting the presence, amount or both of hybridized oligonucleotides or amplified products in a) as an indication of the presence, amount or both of said one or more *tuf* nucleic acids from *Listeria monocytogenes.*

In a further aspect of the invention, an isolated oligonucleotide having at least 12 nucleotides in length derived from and which hybridizes specifically to: SEQ ID NO:138, or a sequence complementary thereof, is provided for determining the presence and/or amount of one or more tuf nucleic acids from the *Listeria monocytogenes* species. In a further aspect of the invention, an isolated tuf nucleic acid from *Listeria monocytogenes* having the nucleotide sequence defined in SEQ ID NO: 138, a part thereof or a complementary sequence thereof is also provided. The invention also encompasses a recombinant plasmid comprising the isolated oligonucleotide or the nucleic acid of the invention, as well as a recombinant host which has been transformed by such recombinant plasmid.

The invention further provides a diagnostic kit for determining or detecting the presence and/or amount of one or more nucleic acids from the *Listeria monocytogenes* bacterial species in a sample, comprising any suitable combination of species-specific probes and/or primers having at least 12 nucleotides in length derived from and which hybridize specifically to SEQ ID NO:138 or a complementary sequence thereof for determining or detecting the presence and/or amount of one or more *tuf* nucleic acids from the *Listeria monocytogenes* species.

Further described herein is a specific, ubiquitous and sensitive method using probes and/or amplification primers for determining the presence and/or amount of nucleic acids:
- from specific microbial species or genera selected from the group consisting of *Streptococcus* species, *Streptococcus agalactine, Staphylococcus* species, *Staphylococcus saprophyticus. Enterococcus* species, *Enterococcus faecium, Neisseria* species, *Neisseria. meningitidis, Listeria monocytogenes, Candid* species and *Candida albicans*
- from an antibiotic resistance gene selected from the group consisting of *blaₜₑₘ, bla_{rob}, blaₛₕᵥ, blaₒₓₐ, blaZ, aadB, aacC1, aacC2, aacC3, aacA4, aac6'-IIa, ermA, ermB, ermC, mecA, vanA, vanB, vanC, satA, aac(6')-aph(2"), aad(6'), vat, vga, msrA, sul* and *int,* and optionally,
- from any bacterial species
in any sample suspected of containing said nucleic acids,
wherein each of said nucleic acids or a variant or part thereof comprises a selected target region hybridizable with said probe or primers;
said method comprising the steps of contacting said sample with said probes or primers and detecting the presence and/or amount of hybridized probes or amplified products as an indication of the presence and/or amount of said any bacterial species, specific microbial species or genus and antibiotic resistance gene.

Further described herein is, a similar method directed to each specific microbial species or genus detection and identification, antibiotic resistance genes detection, and universal bacterial detection, separately.

The method makes use of DNA fragments (proprietary fragments and fragments obtained from databases), selected for their capacity to sensitively, specifically and ubiquitously detect the targeted bacterial or fungal nucleic acids.

Also described herein are oligonucleotides of at least 12 nucleotides in length which have been derived from the longer DNA fragments, and are used in the present method as probes or amplification primers.

The proprietary oligonucleotides (probes and primers) are also described herein.

Diagnostic kits comprising probes or amplification primers for the detection of a microbial species or genus selected from the group consisting of *Streptococcus* species, *Streptococcus agalactiae, Staphylococcus* species, *Staphylococcus saprophyticus, Enterococcus* species, *Enterococcus faecium, Neisseria* species, *Neisseria meningitidis, Listeria monocytogenes, Candida* species and *Candida albicans* are also described herein.

Diagnostic kits further comprising probes or amplification primers for the detection of an antibiotic resistance gene selected from the group consisting of *blaₜₑₘ, bla_{rob}, blaₛₕᵥ, blaₒₓₐ, blaZ, aadB, aacC1, aacC2, aacC3, aacA4, acre6'-IIa, ermA, ermB, ermC, mecA, vanA, vanB, vanC, satA, aac(6')-aph(2"), aad(6'), vat, vga, msrA, sul* and *int* are also described herein.

Diagnostic kits further comprising probes or amplification primers for the detection of any bacterial or fungal species, comprising or not comprising those for the detection of the specific microbial species or genus listed above, and further comprising or not comprising probes and primers for the antibiotic resistance genes listed above, are also described herein.

Such a kit allows for the separate or the simultaneous detection and identification of the above-listed microbial species or genus, antibiotic resistance genes and for the detection of any bacterium.

In the above methods and kits, amplification reactions may include a) polymerase chain reaction (PCR), b) ligase chain reaction, c) nucleic acid sequence-based amplification, d) self-sustained sequence replication, e) strand displacement amplification, f) branched DNA signal amplification, g) transcription-mediated amplification, h) cycling probe technology (CPT) i) nested PCR, or j) multiplex PCR.

In a preferred embodiment, a PCR. protocol is used as an amplification reaction.

In a particularly preferred embodiment, a PCR protocol is provided, comprising, for each amplification cycle, an annealing step of 30 seconds at 45-55°C and a denaturation step of only one second at 95°C, without any time allowed specifically for the elongation step. This PCR protocol has been standardized to be suitable for PCR reactions with all selected primer pairs, which greatly facilitates the testing because each clinical sample can be tested with universal, species-specific, genus-specific and antibiotic resistance gene PCR primers under uniform cycling conditions. Furthermore, various combinations of primer pairs may be used in multiplex PCR assays.

We aim at developing a rapid test or kit to discard rapidly all the samples which are negative for bacterial cells and to subsequently detect and identify the above bacterial and/or fungal species and genera and to determine rapidly the bacterial resistance to antibiotics. Although the sequences from the selected antibiotic resistance genes are available from databases and have been used to develop DNA-based tests for their detection, our approach is unique because it represents a major improvement over current gold standard diagnostic methods based on bacterial cultures. Using an amplification method for the simultaneous bacterial detection and identification and antibiotic resistance genes detection, there is no need for culturing the clinical sample prior to testing. Moreover, a modified PCR protocol has been developed to detect all target DNA sequences in approximately one hour under uniform amplification conditions. This procedure will save lives by optimizing treatment, will diminish antibiotic resistance because less antibiotics will be prescribed, will reduce the use of broad spectrum antibiotics which are expensive, decrease overall health care costs by preventing or shortening hospitalizations, and decrease the time and costs associated with clinical laboratory testing.

In the methods and kits described herein below, the oligonucleotide probes and amplification primers have been derived from larger sequences (i.e. DNA fragments of at least 100 base pairs). All DNA fragments have been obtained either from proprietary fragments or from databases. DNA fragments selected from databases are newly used in a method of detection according to the present invention, since they have been selected for their diagnostic potential.

It is clear to the individual skilled in the art that other oligonucleotide sequences appropriate for (i) the universal bacterial detection, (ii) the detection and identification of the above microbial species or genus and (iii) the detection of antibiotic resistance genes other than those listed in Annex VI may also be derived from the proprietary fragments or selected database sequences. For example, the oligonucleotide primers or probes may be shorter or longer than the ones we have chosen; they may also be selected anywhere else in the proprietary DNA fragments or in the sequences selected from databases; they may be also variants of the same oligonucleotide. If the target DNA or a variant thereof hybridizes to a given oligonucleotide, or if the target DNA or a variant thereof can be amplified by a given oligonucleotide PCR primer pair, the converse is also true; a given target DNA may hybridize to a variant oligonucleotide probe or be amplified by a variant oligonucleotide PCR primer. Alternatively, the oligonucleotides may be designed from any DNA fragment sequences for use in amplification methods other than PCR. Consequently, the core of this disclosure is the identification of universal, species-specific, genus-specific and resistance gene-specific genomic or non-genomic DNA fragments which are used as a source of specific and ubiquitous oligonucleotide probes and/or amplification primers. Although the selection and evaluation of oligonucleotides suitable for diagnostic purposes requires much effort, it is quite possible for the individual skilled in the art to derive, from the selected DNA fragments, oligonucleotides other than the ones listed in Annex VI which are suitable for diagnostic purposes. When a proprietary fragment or a database sequence is selected for its specificity and ubiquity, it increases the probability that subsets thereof will also be specific and ubiquitous.

Since a high percentage of clinical specimens are negative for bacteria (Table 3), DNA fragments having a high potential for the selection of universal oligonucleotide probes or primers were selected from proprietary and database sequences. The amplification primers were selected from a gene highly conserved in bacteria and fungi, and are used to detect the presence of any bacterial pathogen in clinical specimens in order to determined rapidly (approximately one hour) whether it is positive or negative for bacteria. The selected gene, designated *tuf*, encodes a protein (EF-Tu) involved in the translational process during protein synthesis. The *tuf* gene sequence alignments used to derive the universal primers include both proprietary and database sequences (Example 1 and Annex I). This strategy allows the rapid screening of the numerous negative clinical specimens (around 80% of the specimens received, see Table 3) submitted for bacteriological testing. Tables 4, 5 and 6 provide a list of the bacterial or fungal species used to test the specificity of PCR primers and DNA probes. Table 7 gives a brief description of each species-specific, genus-specific and universal amplification assays. Tables 8, 9 and 10 provide some relevant information about the proprietary and database sequences selected for diagnostic puposes.

### DETAILED DESCRIPTION OF THE INVENTION

### Development of species-specific, genus-speciric, universal and antibiotic resistance gene-specific DNA probes and amplification primers for microorganisms Selection from databases of sequences suitable for diagnostic purposes

In order to select sequences which are suitable for species-specific or genus-specific detection and identification of bacteria or fungi or, alternatively, for the universal detection of bacteria, the database sequences (GenBank, EMBL and Swiss-Prot) were chosen based on their potential for diagnostic purposes according to sequence information and computer analysis performed with these sequences. Initially, all sequence data available for the targeted microbial species or genus were carefully analyzed. The gene sequences which appeared the most promising for diagnostic purposes based on sequence information and on sequence comparisons with the corresponding gene in other microbial species or genera performed with the Genetics Computer Group (GCG, Wisconsin) programs were selected for testing by PCR. Optimal PCR amplification primers were chosen from the selected database sequences with the help of the Oligo™ 4.0 primer analysis software (National Biosciences Inc., Plymouth, Minn.). The chosen primers were tested in PCR assays for their specificity and ubiquity for the target microbial species or genus. In general, the identification of database sequences from which amplification primers suitable for species-specific or genus-specific detection and identification were selected involved the computer analysis and PCR testing of several candidate gene sequences before obtaining a primer pair which is specific and ubiquitous for the target microbial species or genus. Annex VI provides a list of selected specific and ubiquitous PCR primer pairs. Annexes I to V and Examples 1 to 4 illustrate the strategy used to select genus-specific, species-specific and universal PCR primers from *tuf* sequences or from the *rec*A gene.

### Oligonucleotide primers and probes design and synthesis

The DNA fragments sequenced by us or selected from databases (GenBank and EMBL) were used as sources of oligonucleotides for diagnostic purposes. For this strategy, an array of suitable oligonucleotide primers or probes derived from a variety of genomic DNA fragments (size of more than 100 bp) selected from databases were tested for their specificity and ubiquity in PCR and hybridization assays as described later. It is important to note that the database sequences were selected based on their potential for being species-specific, genus-specific or universal for the detection of bacteria or fungi according to available sequence information and extensive analysis and that, in general, several candidate database sequences had to be tested in order to obtain the desired specificity, ubiquity and sensitivity.

Oligonucleotide probes and amplification primers derived from species-specific fragments selected from database sequences were synthesized using an automated DNA synthesizer (Perkin-Elmer Corp., Applied Biosystems Division). Prior to synthesis, all oligonucleotides (probes for hybridization and primers for DNA amplification) were evaluated for their suitability for hybridization or DNA amplification by polymerase chain reaction (PCR) by computer analysis using standard programs (i.e. the Genetics Computer Group (GCG) programs and the primer analysis software Oligo™ 4.0). The potential suitability of the PCR primer pairs was also evaluated prior to the synthesis by verifying the absence of unwanted features such as long stretches of one nucleotide and a high proportion of G or C residues at the 3' end (Persing et al., 1993, Diagnostic Molecular Microbiology: Principles and Applications, American Society for Microbiology, Washington, D.C.).

The oligonucleotide primers or probes may be derived from either strand of the duplex DNA. The primers or probes may consist of the bases A, G, C, or T or analogs and they may be degenerated at one or more chosen nucleotide position(s). The primers or probes may be of any suitable length and may be selected anywhere within the DNA sequences from proprietary fragments or from selected database sequences which are suitable for (i) the universal detection of bacteria, (ii) the species-specific detection and identification of *Enterococcus faecium, Listeria monocytogenes, Neisseria meningitidis, Slaphylococcus saprophyticus, Streptococcus agalactiae* and *Candida albicans* (iii) the genus-specific detection of *Streptococcus* species, *Enterococcus* species, *Staphylococcus* species and *Neisseria* species or (iv) the detection of the 26 above-mentioned clinically important antibiotic resistance genes.

Variants for a given target bacterial gene are naturally occurring and are attributable to sequence variation within that gene during evolution (Watson et al., 1987, Molecular Biology of the Gene, 4th ed., The Benjamin/Cummings Publishing Company, Menlo Park, CA; Lewin, 1989, Genes IV, John Wiley & Sons, New York, NY). For example, different strains of the same bacterial species may have a single or more nucleotide variation(s) at the oligonucleotide hybridization site. The person skilled in the art is well aware of the existence of variant bacterial or fungal DNA sequences for a specific gene and that the frequency of sequence variations depends on the selective pressure during evolution on a given gene product. The detection of a variant sequence for a region between two PCR primers may be demonstrated by sequencing the amplification product. In order to show the presence of sequence variants at the primer hybridization site, one has to amplify a larger DNA target with PCR primers outside that hybridization site. Sequencing of this larger fragment will allow the detection of sequence variation at this site. A similar strategy may be applied to show variants at the hybridization site of a probe. Insofar as the divergence of the target sequences or a part thereof does not affect the specificity and ubiquity of the amplification primers or probes, variant bacterial DNA is under the scope of this invention. Variants of the selected primers or probes may also be used to amplify or hybridize to a variant DNA.

### Sequencing of tuf sequences from a variety of bacterial and fungal species

The nucleotide sequence of a portion of *tuf* genes was determined for a variety of bacterial and fungal species. The amplification primers SEQ ID NOs: 107 and 108, which amplify a *tuf* gene portion of approximately 890 bp, were used for the sequencing of bacterial *tuf* sequences. The amplification primers SEQ ID NOs: 109 and 172, which amplify a *tuf* gene portion of approximately 830 bp, were used for the sequencing of fungal *tuf* sequences. Both primer pairs can amplify *tufA* and *tufB* genes. This is not surprising because these two genes are nearly identical. For example, the entire *tuf* and *tufB* genes from *E*. *coli* differ at only 13 nucleotide positions (Neidhardt et al., 1996, Escherichia coli and Salmonella: Cellular and Molecular Biology, 2nd ed., American Society for Microbiology Press, Washington, D.C.). These amplification primers are degenerated at several nucleotide positions and contain inosines in order to allow the amplification of a wide range of *tuf* sequences. The strategy used to select these amplification primers is similar to that illustrated in Annex I for the selection of universal primers. The amplification primers SEQ ID NOs: 107 and 108 could be used to amplify the *tuf* genes from any bacterial species. The amplification primers SEQ ID NOs: 109 and 172 could be used to amplify the *tuf* genes from any fungal species.

The *tuf* genes were amplified directly from bacterial or yeast cultures using the following amplification protocol: One µL of cell suspension was transferred directly to 19 uL of a PCR reaction mixture containing 50 mM KCl, 10 mM Tris-HCl (pH 9.0), 0.1% Triton X-100, 2.5 mM MgCl₂, 1 µM of each of the 2 primers, 200 µM of each of the four dNTPs, 0.5 unit of *Taq* DNA polymerase (Promega Corp., Madison, WI). PCR reactions were subjected to cycling using a MJ Research PTC-200 thermal cycler (MJ Research Inc., Watertown, Mass.) as follows: 3 min at 96°C followed by 30-35 cycles of 1 min at 95°C for the denaturation step, 1 min at 30-50°C for the annealing step and 1 min at 72°C for the extension step. Subsequently, twenty microliters of the PCR-amplified mixture were resolved by electrophoresis in a 1.5% agarose gel. The gel was then visualized by staining with methylene blue (Flores et al., 1992, Biotechniques, 13:203-205). The size of the amplification products was estimated by comparison with a 100-bp molecular weight ladder. The band corresponding to the specific amplification product (i.e. approximately 890 or 830 bp for bacterial or fungal *tuf* sequences, respectively) was excised from the agarose gel and purified using the QIAquick™ gel extraction kit (QIACTEN Inc., Chatsworth, CA). The gel-purified DNA fragment was then used directly in the sequencing protocol. Both strands of the *tuf* genes amplification product were sequenced by the dideoxynucleotide chain termination sequencing method by using an Applied Biosystems automated DNA sequencer (model 373A) with their PRISM™ Sequenase® Terminator Double-stranded DNA Sequencing Kit (Perkin-Elmer Corp., Applied Biosystems Division, Foster City, CA). The sequencing reactions were all performed by using the amplification primers (SEQ ID NOs: 107 to 109 and 172) and 100 ng per reaction of the gel-purified amplicon. In order to ensure that the determined sequence did not contain errors attributable to the sequencing of PCR artefacts, we have sequenced two preparations of the gel-purified *tuf* amplification product originating from two independent PCR amplifications. For all target microbial species, the sequences determined for both amplicon preparations were identical. Furthermore, the sequences of both strands were 100% complementary thereby confirming the high accuracy of the determined sequence. The *tuf* sequences determined using the above strategy are all in the Sequence Listing (i.e. SEQ ID NOs:118 to 146). Table 13 gives the originating microbial species and the source for each *tuf* sequence in the Sequence Listing.

The alignment of the *tuf* sequences determined by us or selected from databases reveals clearly that the length of the sequenced portion of the *tuf* genes is variable. There may be insertions or deletions of several amino acids. This explains why the size of the sequenced *tuf* amplification product was variable for both bacterial and fungal species. Among the *tuf* sequences determined by our group, we found insertions and deletions adding up to 5 amino acids or 15 nucleotides. Consequently, the nucleotide positions indicated on top of each of Annexes I to V do not correspond for *tuf* sequences having insertions or deletions.

It should also be noted that the various *tuf* sequences determined by us occasionally contain degenerescences. These degenerated nucleotides correspond to sequence variations between *tufA* and *tufB* genes because the amplification primers amplify both *tuf* genes. These nucleotide variations were not attributable to nucleotide misincorporations by the *taq* DNA polymerase because the sequence of both strands were identical and also because the sequences determined with both preparations of the gel-purified *tuf* amplicons were identical.

### The selection of amplification primers from tuf sequences

The *tuf* sequences determined by us or selected from databases were used to select PCR primers for (i) the universal detection of bacteria, (ii) the genus-specific detection and identification of *Enterococcus* spp. and *Staphylococcus* spp. and (iii) the species-specific detection and identification of *Candida albicans.* The strategy used to select these PCR primers was based on the analysis of multiple sequence alignments of various *tuf* sequences. For more details about the selection of PCR primers from *tuf* sequences, please refer to Examples 1 to 3 and Annexes I to IV.

### The selection of amplification primers from recA

The comparison of the nucleotide sequence for the *recA* gene from various bacterial species including 5 species of streptococci allowed the selection of *Streptococcus-specific* PCR primers. For more details about the selection of PCR primers from *recA,* please refer to Example 4 and Annex V.

### DNA fragments isolation from Staphylococcus saprophyticus by arbitrarily primed PCR

DNA sequences of unknown coding potential for the species-specific detection and identification of *Staphylococcus saprophyticus* were obtained by the method of arbitrarily primed PCR (AP-PCR).

AP-PCR is a method which can be used to generate specific DNA probes for microorganisms (Fani et al., 1993, Mol. Ecol. 2:243-250). A description of the AP-PCR protocol used to isolate a species-specific genomic DNA fragment from *Staphylococcus saprophyticus* follows. Twenty different oligonucleotide primers of 10 nucleotides in length (all included in the AP-PCR kit OPAD (Operon Technologies, Inc., Alameda, CA)) were tested systematically with DNAs from 3 bacterial strains of *Staphylococcus saprophyticus* (all obtained from the American Type Culture Collection (ATCC): numbers 15305, 35552 and 43867) as well as with DNA from four other staphylococcal species (*Staphylococcus aureus* ATCC 25923, *Staphylococcus epidermidis* ATCC 14990. *Staphylococcus haemolyticus* ATCC 29970 and *Staphylococcus hominis* ATCC 35982). For all bacterial species, amplification was performed from a bacterial suspension adjusted to a standard 0.5 McFarland which corresponds to approximately 1.5 x 10⁸ bacteria/mL. One µL of the standardized bacterial suspension was transferred directly to 19 µL of a PCR reaction mixture containing 50 mM KCl, 10 mM Tris-HCl (pH 9.0), 0.1% Triton X-100, 2.5 mM MgCl₂, 1.2 µM of only one of the 20 different AP-PCR primers OPAD, 200 µM of each of the four dNTPs and 0.5 unit of *Taq* DNA polymerase (Promega Corp., Madison, WI). PCR reactions were subjected to cycling using a MJ Research PTC-200 thermal cycler (MJ Research Inc.) as follows: 3 min at 96°C followed by 35 cycles of 1 min at 95°C for the denaturation step, 1 min at 32°C for the annealing step and 1 min at 72°C for the extension step. A final extension step of 7 min at 72°C was made after the 35 cycles to ensure complete extension of PCR products. Subsequently, twenty microliters of the PCR amplified mixture were resolved by electrophoresis in a 2% agarose gel containing 0.25 µg/mL of ethidium bromide. The size of the amplification products was estimated by comparison with a 50-bp molecular weight ladder.

Amplification patterns specific for *Staphylococcus saprophyticus* were observed with the AP-PCR primer OPAD-9 (SEQ ID NO: 25). Amplification with this primer consistently showed a band corresponding to a DNA fragment of approximately 450 bp for all *Staphylococcus saprophyticus* strains tested but not for any of the four other staphylococcal species tested. This species-specific pattern was confirmed by testing 10 more clinical isolates of *S*. *saprophyticus* selected from the culture collection of the microbiology laboratory of the CHUL as well as strains selected from the gram-positive bacterial species listed in Table 5.

The band corresponding to the approximately 450 bp amplicon which was specific and ubiquitous for *S*. *saprophyticus* based on AP-PCR was excised from the agarose gel and purified using the QIAquick™ gel extraction kit (QIAGEN Inc.). The gel-purified DNA fragment was cloned into the T/A cloning site of the pCR 2.1™ plasmid vector (Invitrogen Inc.) using T4 DNA ligase (New England BioLabs). Recombinant plasmids were transformed into *E*. *coli* DH5α competent cells using standard procedures. Plasmid DNA isolation was done by the method of Birnboim and Doly (Nucleic Acids Res. 7:1513-1523) for small-scale preparations. All plasmid DNA preparations were digested with the *Eco*RI restriction endonuclease to ensure the presence of the approximately 450 bp AP-PCR insert into the recombinant plasmids. Subsequently, a large-scale and highly purified plasmid DNA preparation was performed from two selected clones shown to carry the AP-PCR insert by using the QIAGEN plasmid purification kit. These plasmid preparations were used for automated DNA sequencing.

Both strands of the AP-PCR insert from the two selected clones were sequenced by the dideoxynucleotide chain termination sequencing method with SP6 and T7 sequencing primers, by using an Applied Biosystems automated DNA sequencer as described previously. The analysis of the obtained sequences revealed that the DNA sequences for both strands from each clone were 100% complementary. Furthermore, it showed that the entire sequence determined for each clone were both identical. These sequencing data confirm the 100% accuracy for the determined 438 bp sequence (SEQ ID NO: 29). Optimal amplification primers have been selected from the sequenced AP-PCR *Staphylococcus saprophyticus* DNA fragment with the help of the primer analysis software Oligo™ 4.0. The selected primer sequences have been tested in PCR assays to verify their specificity and ubiquity (Table 7). These PCR primers were specific since there was no amplification with DNA from bacterial species other than *S*. *saprophyticus* selected from Tables 4 and 5. Furthermore, this assay was ubiquitous since 245 of 260 strains of *S*. *saprophyticus* were efficiently amplified with this PCR assay. When used in combination with another *S*. *saprophyticus*-specific PCR assay, which is an object of our co-pending U.S. (N.S. 08/526,840) and PCT (PCT/CA/95/00528) patent applications, the ubiquity reaches 100% for these 260 strains.

### DNA amplification

For DNA amplification by the widely used PCR (polymerase chain reaction) method, primer pairs were derived from proprietary DNA fragments or from database sequences. Prior to synthesis, the potential primer pairs were analyzed by using the Oligo™ 4.0 software to verify that they are good candidates for PCR amplification.

During DNA amplification by PCR, two oligonucleotide primers binding respectively to each strand of the heat-denatured target DNA from the bacterial genome are used to amplify exponentially *in vitro* the target DNA by successive thermal cycles allowing denaturation of the DNA, annealing of the primers and synthesis of new targets at each cycle (Persing et al, 1993, Diagnostic Molecular Microbiology: Principles and Applications, American Society for Microbiology, Washington, D.C.).

Briefly, the PCR protocols were as follow: Treated clinical specimens or standardized bacterial or fungal suspensions (see below) were amplified in a 20 µL PCR reaction mixture containing 50 mM KCl, 10 mM Tris-HCl (pH 9.0), 2.5 mM MgCl₂, 0.4 µM of each primer, 200 µM of each of the four dNTPs and 0.5 unit of *Taq* DNA polymerase (Promega) combined with the TaqStart™ antibody (Clontech Laboratories Inc., Palo Alto, CA). The TaqStart™ antibody, which is a neutralizing monoclonal antibody to *Taq* DNA polymerase, was added to all PCR reactions to enhance the specificity and the sensitivity of the amplifications (Kellogg et al., 1994, Biotechniques 16:1134-1137). The treatment of the clinical specimens varies with the type of specimen tested, since the composition and the sensitivity level required are different for each specimen type. It consists in a rapid protocol to lyse the bacterial cells and eliminate the PCR inhibitory effects (see example 11 for urine specimen preparation). For amplification from bacterial or fungal cultures, the samples were added directly to the PCR amplification mixture without any pre-treatment step (see example 10). Primer sequences derived from highly conserved regions of the bacterial 16S ribosomal RNA gene were used to provide an internal control for all PCR reactions. Alternatively, the internal control was derived from sequences not found in microorganisms or in the human genome. The internal control was integrated into all amplification reactions to verify the efficiency of the PCR assays and to ensure that significant PCR inhibition was absent. The internal control derived from rRNA was also useful to monitor the efficiency of bacterial lysis protocols.

PCR reactions were then subjected to thermal cycling (3 min at 95°C followed by 30 cycles of 1 second at 95°C for the denaturation step and 30 second at 55°C for the annealing-extension step) using a PTC-200 thermal cycler (MJ Research Inc.) and subsequently analyzed by standard ethidium bromide-stained agarose gel electrophoresis. The number of cycles performed for the PCR assays varies according to the sensitivity level required. For example, the sensitivity level required for microbial detection directly from clinical specimens is higher for blood specimens than for urine specimens because the concentration of microorganisms associated with a septicemia can be much lower than that associated with a urinary tract infection. Consequently, more sensitive PCR assays having more thermal cycles are required for direct detection from blood specimens. Similarly, PCR assays performed directly from bacterial or fungal cultures may be less sensitive than PCR assays performed directly from clinical specimens because the number of target organisms is normally much lower in clinical specimens than in microbial cultures.

It is clear that other methods for the detection of specific amplification products, which may be faster and more practical for routine diagnosis, may be used. Such methods may be based on the detection of fluorescence after amplification (e.g. TaqMan™ system from Perkin Elmer or Amplisensor™ from Biotronics). Methods based on the detection of fluorescence are particularly promising for utilization in routine diagnosis as they are very rapid, quantitative and can be automated (Example 14).

Microbial pathogens detection and identification may also be performed by solid support or liquid hybridization using species-specific internal DNA probes hybridizing to an amplification product. Such probes may be generated from any species-specific or genus-specific DNA amplification products which are described and/or claimed herein. Alternatively, the internal probes for species or genus detection and identification may be derived from the amplicons produced by the universal amplification assay. The oligonucleotide probes may be labeled with biotin or with digoxigenin or with any other reporter molecules.

To assure PCR efficiency, glycerol, dimethyl sulfoxide (DMSO) or other related solvents can be used to increase the sensitivity of the PCR and to overcome problems associated with the amplification of a target DNA having a high GC content or forming strong secondary structures (Dieffenbach and Dveksler, 1995, PCR Primer : A Laboratory Manual, Cold Spring Harbor Laboratory Press, Plainview, New York). The concentration ranges for glycerol and DMSO are 5-15% (v/v) and 3-10% (v/v), respectively. For the PCR reaction mixture, the concentration ranges for the amplification primers and MgCl₂ are 0.1-1.5 µM and 1.5-3.5 mM, respectively. Modifications of the standard PCR protocol using external and nested primers (i.e. nested PCR) or using more than one primer pair (i.e. multiplex PCR) may also be used (Persing et al., 1993, Diagnostic Molecular Microbiology: Principles and Applications, American Society for Microbiology, Washington, D.C.). For more details about the PCR protocols and amplicon detection methods, see Examples 9 to 14.

The person skilled in the art of DNA amplification knows the existence of other rapid amplification procedures such as ligase chain reaction (LCR), transcription-mediated amplification (TMA), self-sustained sequence replication (3SR), nucleic acid sequence-based amplification (NASBA), strand displacement amplification (SDA), branched DNA (bDNA) and cycling probe technology (CPT) (Lee et al., 1997, Nucleic Acid Amplification Technologies: Application to Disease Diagnosis, Eaton Publishing, Boston, MA ; Persing et al., 1993, Diagnostic Molecular Microbiology: Principles and Applications, American Society for Microbiology, Washington, D.C.). The scope of this invention is not limited to the use of amplification by PCR, hut rather includes the use of any rapid nucleic acid amplification method or any other procedure which may be used to increase rapidity and sensitivity of the tests. Any oligonucleotide suitable for the amplification of nucleic acids by approaches other than PCR and derived from the species-specific, genus-specific and universal DNA fragments as well as from selected antibiotic resistance gene sequences included in this document are also under the scope of this invention.

### Hybridization assays with oligonucleotide probes

In hybridization experiments, single-stranded oligonucleotides (size less than 100 nucleotides) have some advantages over DNA fragment probes for the detection of bacteria, such as ease of synthesis in large quantities, consistency in results from batch to batch and chemical stability. Briefly, for the hybridizations, oligonucleotides were 5' end-labeled with the radionucleotide γ-³²P(dATP) using T4 polynucleotide kinase (Pharmacia) (Sambrook et al., 1989, Molecular Cloning: A Laboratory Manual, 2nd ed., Cold Spring Harbor Laboratory Press, Cold Spring Harbor, NY). The unincorporated radionucleotide was removed by passing the labeled oligonucleotide through a Sephadex G-50™ column. Alternatively, oligonucleotides were labeled with biotin, either enzymatically at their 3' ends or incorporated directly during synthesis at their 5' ends, or with digoxigenin. It will be appreciated by the person skilled in the art that labeling means other than the three above labels may be used.

Each oligonucleotide probe was then tested for its specificity by hybridization to DNAs from a variety of bacterial and fungal species selected from Tables 4, 5 and 6. All of the bacterial or fungal species tested were likely to be pathogens associated with common infections or potential contaminants which can be isolated from clinical specimens. Each target DNA was released from bacterial cells using standard chemical treatments to lyse the cells (Sambrook et al., 1989, Molecular Cloning: A Laboratory Manual, 2nd ed., Cold Spring Harbor Laboratory Press, Cold Spring Harbor, NY). Subsequently, the DNA was denatured by conventional methods and then irreversibly fixed onto a solid support (e.g. nylon or nitrocellulose membranes) or free in solution. The fixed single-stranded target DNAs were then hybridized with the oligonucleotide probe cells (Sambrook et al., 1989, Molecular Cloning: A Laboratory Manual, 2nd ed., Cold Spring Harbor Laboratory Press, Cold Spring Harbor, NY). Pre-hybridization conditions were in 1 M NaCl + 10% dextran sulfate + 1% SDS + 100 µg/mL salmon sperm DNA at 65°C for 15 min. Hybridization was performed in fresh pre-hybridization solution containing the labeled probe at 65°C overnight. Post-hybridization washing conditions were as follows: twice in 3X SSC containing 1% SDS, twice in 2X SSC containing 1% SDS and twice in 1X SSC containing 1% SDS (all of these washes were at 65°C for 15 min), and a final wash in 0.1X SSC containing 1% SDS at 25°C for 15 min. Autoradiography of washed filters allowed the detection of selectively hybridized probes. Hybridization of the probe to a specific target DNA indicated a high degree of similarity between the nucleotide sequence of these two DNAs because of the high stringency of the washes.

An oligonucleotide probe was considered specific only when it hybridized solely to DNA from the species or genus from which it was isolated. Oligonucleotide probes found to be specific were subsequently tested for their ubiquity (i.e. ubiquitous probes recognized most or all isolates of the target species or genus) by hybridization to microbial DNAs from clinical isolates of the species or genus of interest including ATCC strains. The DNAs from strains of the target species or genus were denatured, fixed onto nylon membranes and hybridized as described above. Probes were considered ubiquitous when they hybridized specifically with the DNA from at least 80% of the isolates of the target species or genus.

### Specificity and ubiquity tests for oligonucleotide primers and probes

The specificity of oligonucleotide primers and probes, derived either from the DNA fragments sequenced by us or selected from databases, was tested by amplification of DNA or by hybridization with bacterial or fungal species selected from those listed in Tables 4, 5 and 6, as described in the two previous sections. Oligonucleotides found to be specific were subsequently tested for their ubiquity by amplification (for primers) or by hybridization (for probes) with bacterial DNAs from isolates of the target species or genus. Results for specificity and ubiquity tests with the oligonucleotide primers are summarized in Table 7. The specificity and ubiquity of the PCR assays using the selected amplification primer pairs were tested directly from cultures (see Examples 9 and 10) of bacterial or fungal species.

The various species-specific and genus-specific PCR assays which are described and/or claimed herein are all specific. For the PCR assays specific to bacterial species or genus, this means that DNA isolated from a wide variety of bacterial species, other than that from the target species or genus and selected from Tables 4 and 5, could not be amplified. For the PCR assay specific to *Candida albicans,* it means there was no amplification with genomic DNA from the fungal species listed in Table 6 as well as with a variety of bacterial species selected from Tables 4 and 5.

The various species-specific and genus-specific PCR assays which are described and/or claimed herein are also all ubiquitous (Table 7). (i) The species-specific PCR assays for *E. faecium, L. monocytogenes, S. saprophyticus, S. agalactiae* and *C*. *albicans* amplified genomic DNA from all or most strains of the target species tested, which were obtained from various sources and which are representative of the diversity within each target species (Table 7). The species identification of all of these strains was based on classical biochemical methods which are routinely used in clinical microbiology laboratories. (ii) The genus-specific PCR assays specific for *Enterococcus* spp., *Staphylococcus* spp., *Streptococcus* spp. and *Neisseria* spp. amplified genomic DNA from all or most strains of the target genus tested, which represent all clinically important bacterial species for each target genus. These strains were obtained from various sources and are representative of the diversity within each target genus. Again, the species identification of all of these strains was based on classical biochemical methods which are routinely used in clinical microbiology laboratories. More specifically, the four genus-specific PCR assays amplified the following species: (1) The *Enterococcus-specific* assay amplified efficiently DNA from all of the 11 enterococcal species tested including *E. avium, E. casseliflavus, E. dispar, E. durbans, E. faecalis, E. faecium, E. flavescens, E. gallinarum, E. hirae, E. mundtii* and *E*. *raffinosus.* (2) The *Neisseria*-specific assay amplified efficiently DNA from all of the 12 neisserial species tested including *N. canis, N. cinerea, N. elongata, N. flavescens, N. gonorrhoeae, N. lactamica, N. meningitidis, N. mucosa, N. polysaccharea, N. sicca, N. subflava* and *N. weaveri.* (3) The *Staphylococcus*-specific assay amplified efficiently DNA from 13 of the 14 staphylococcal species tested including *S*. *aureus, S. auriculatis, S. capitis, S. cohnii, S. epidennidis, S. haemolyticus, S. hominis, S. lugdunensis, S. saprophyticus, S. schleiferi, S. simulans, S. warneri* and *S*. *xylosus.* The staphylococcal species which could not be amplified is *S*. *sciuri.* (4) Finally, the *Streptococcus-specific* assay amplified efficiently DNA from all of the 22 streptococcal species tested including *S*. *agalactiae, S. anginosus, S. bovis, S. constellatus, S. crista, S. dysgalactiae, S. equi, S. gordonii, S. intermedius, S. mitis, S. mutans, S. oralis, S. parasafiguis, S. pneumoniae S. pyogenes, S. salivarius, S. sanguis, S. sabrinus, S. suis, S*. *uberis, S*. *vestibularis* and *S. viridans.* On the other hand, the *Streptococcus-specific* assay did not amplify 3 out of 9 strains of *S*. *mutans* and 1 out of 23 strains of *S*. *salivarius,* thereby showing a slight lack of ubiquity for these two streptococcal species.

All specific and ubiquitous amplification primers for each target microbial species or genus or antibiotic resistance gene investigated are listed in Annex VI. Divergence in the sequenced DNA fragments can occur, insofar as the divergence of these sequences or a part thereof does not affect the specificity of the probes or amplification primers. Variant bacterial DNA is under the scope of this invention.

The PCR amplification primers listed in Annex VI were all tested for their specificity and ubiquity using reference strains as well as clinical isolates from various geographical locations. The 351 reference strains used to test the amplification and hybridization assays (Tables 4, 5 and 6) were obtained from (i) the American Type Culture Collection (ATCC): 85%, (ii) the Laboratoire de santé publique du Québec (LSPQ): 10%, (iii) the Centers for Disease Control and Prevention (CDC): 3% , (iv) the National Culture Type Collection (NCTC): 1% and (v) several other reference laboratories throughout the world: 1%. These reference strains are representative of (i) 90 gram-negative bacterial species (169 strains; Table 4), (ii) 97 gram-positive bacterial species (154 strains; Table 5) and (iii) 12 fungal species (28 strains; Table 6).

### Antibiotic resistance genes

Antimicrobial resistance complicates treatment and often leads to therapeutic failures. Furthermore, overuse of antibiotics inevitably leads to the emergence of bacterial resistance. Our goal is to provide clinicians, in approximately one hour, the needed information to prescribe optimal treatments. Besides the rapid identification of negative clinical specimens with DNA-based tests for universal bacterial detection and the identification of the presence of a specific pathogen in the positive specimens with species- and/or genus-specific DNA-based tests, clinicians also need timely information about the ability of the bacterial pathogen to resist antibiotic treatments. We feel that the most efficient strategy to evaluate rapidly bacterial resistance to antimicrobials is to detect directly from the clinical specimens the most common and clinically important antibiotic resistance genes (i.e. DNA-based tests for the detection of antibiotic resistance genes). Since the sequence from the most important and common bacterial antibiotic resistance genes are available from databases, our strategy was to use the sequence from a portion or from the entire resistance gene to design specific oligonucleotide primers or probes which will be used as a basis for the development of rapid DNA-based tests. The sequence from each of the bacterial antibiotic resistance genes selected on the basis of their clinical relevance (i.e. high incidence and importance) is given in the Sequence Listing. Tables 9 and 10 summarize some characteristics of the selected antibiotic resistance genes. Our approach is unique because the antibiotic resistance genes detection and the bacterial detection and identification are performed simultaneously in multiplex assays under uniform PCR amplification conditions (Example 13).

Annex VI provides a list of all amplification primers selected from 26 clinically important antibiotic resistance genes which were tested in PCR assays. The various PCR assays for antibiotic resistance genes detection and identification were validated by testing several resistant bacterial isolates known to carry the targeted gene and obtained from various countries. The testing of a large number of strains which do not carry the targeted resistance gene was also performed to ensure that all assays were specific. So far, all PCR assays for antibiotic resistance genes are highly specific and have detected all control resistant bacterial strains known to carry the targeted gene. The results of some clinical studies to validate the array of PCR assays for the detection and identification of antibiotic resistance genes and correlate these DNA-based assays with standard antimicrobials susceptibility testing methods are presented in Tables 11 and 12.

### Universal bacterial detection

In the routine microbiology laboratory, a high percentage of clinical specimens sent for bacterial identification are negative by culture (Table 4). Testing clinical samples with universal amplification primers or universal probes to detect the presence of bacteria prior to specific identification and screen out the numerous negative specimens is thus useful as it saves costs and may rapidly orient the clinical management of the patients. Several amplification primers and probes were therefore synthesized from highly conserved portions of bacterial sequences from the *tuf* genes (Table 8). The universal primer selection was based on a multiple sequence alignment constructed with sequences determined by us or selected from available database sequences as described in Example 1 and Annex I.

For the identification of database sequences suitable for the universal detection of bacteria, we took advantage of the fact that the complete genome sequences for two distant microorganisms (i.e. *Mycoplasma genitalium* and *Haemophilus influenzae*) are available. A comparison of the amino acid sequence for all proteins encoded by the genome of these two distant microorganisms led to the identification of highly homologous proteins. An analysis of these homologous proteins allowed to select some promising candidates for the development of universal DNA-based assays for the detection of bacteria. Since the complete nucleotide sequence of several other microbial genomes are presently available in databases, a person skilled in the art could arrive to the same conclusions by comparing genomes sequences other than those of *Mycoplasma genitalium* and *Haemophilus influenzae.* The selected *tuf* gene encodes a protein (EF-Tu) involved in the translation process during protein synthesis. Subsequently, an extensive nucleotide sequence analysis was performed with the *tuf* gene sequences available in databases as well as with novel *tuf* sequences which we have determined as described previously. All computer analysis of amino acid and nucleotide sequences were performed by using the GCG programs. Subsequently, optimal PCR primers for the universal amplification of bacteria were selected with the help of the Oligo™ program. The selected primers are degenerated at several nucleotide positions and contain several inosines in order to allow the amplification of all clinically relevant bacterial species (Annex I). Inosine is a nucleotide analog able to specifically bind to any of the four nucleotides A, C, G or T. Degenerated oligonucleotides consist of an oligonucleotide mix having two or more of the four nucleotides A, C, G or T at the site of mismatches. The inclusion of inosine and/or of degenerescences in the amplification primers allow mismatch tolerance thereby permitting the amplification of a wider array of target nucleotide sequences (Dieffenbach and Dveksler, 1995 PCR Primer: A Laboratory Manual, Cold Spring Harbor Laboratory Press, Plainview, NY).

The amplification conditions with the universal primers were identical to those used for the species- and genus-specific amplification assays except that the annealing temperature was 50°C instead of 55°C. This universal PCR assay was specific and nearly ubiquitous for the detection of bacteria. The specificity for bacteria was verified by amplifying genomic DNA isolated from the 12 fungal species listed in Table 6 as well as genomic DNA from *Leishmania donovani, Saccharomyces cerevisiae* and human lymphocytes. None of the above eukaryotic DNA preparations could be amplified by the universal assay, thereby suggesting that this test is specific for bacteria. The ubiquity of the universal assay was verified by amplifying genomic DNAs from 116 reference strains which represent 95 of the most clinically relevant bacterial species. These species have been selected from the bacterial species listed in Tables 4 and 5. We found that 104 of these 116 strains could be amplified. The bacterial species which could not be amplified belong to the following genera: *Corynebacterium* (11 species) and *Stenotrophomonas* (1 species). Sequencing of the *tuf* genes from these bacterial species has been recently performed. This sequencing data has been used to select new universal primers which may be more ubiquitous. These primers are in the process of being tested. We also observed that for several species the annealing temperature had to be reduced to 45°C in order to get an efficient amplification. These bacterial species include *Gemella morbilbrum, Listeria* spp. (3 species) and *Gardnerella vaginalis.* It is important to note that the 95 bacterial species selected from Tables 4 and 5 to test the ubiquity of the universal assay include all of the most clinically relevant bacterial species associated with a variety of human infections acquired in the community or in hospitals (nosocomial infections). The most clinically important bacterial and fungal pathogens are listed in Tables 1 and 2.

### EXAMPLES AND ANNEXES

The following examples and annexes are intended to be illustrative of the various methods and compounds of the invention, rather than limiting the scope thereof.

The various annexes show the strategies used for the selection of amplification primers from *tuf* sequences or from the *recA* gene: (i) Annex I illustrates the strategy used for the selection of the universal amplification primers from *tuf* sequences. (ii) Annex II shows the strategy used for the selection of the amplification primers specific for the genus *Enterococcus* from *tuf* sequences. (iii) Annex III illustrates the strategy used for the selection of the amplification primers specific for the genus *Staphylococcus* from *tuf* sequences. (iv) Annex IV shows the strategy used for the selection of the amplification primers specific for the species *Candida albicans* from *tuf* sequences. (v) Annex V illustrates the strategy used for the selection of the amplification primers specific for the genus *Streptococcus* from *recA* sequences. (vi) Annex VI gives a list of all selected primer pairs. As shown in these annexes, the selected amplification primers may contain inosines and/or degenerescences. Inosine is a nucleotide analog able to specifically bind to any of the four nucleotides A, C, G or T. Alternatively, degenerated oligonucleotides which consist of an oligonucleotide mix having two or more of the four nucleotides A, C, G or T at the site of mismatches were used. The inclusion of inosine and/or of degenerescences in the amplification primers allow mismatch tolerance thereby permitting the amplification of a wider array of target nucleotide sequences (Dieffenbach and Dveksler, 1995 PCR Primer: A Laboratory Manual, Cold Spring Harbor Laboratory Press, Plainview, New York).

### EXAMPLES

### EXAMPLE 1 :

Selection of universal PCR primers from *tuf* sequences. As shown in Annex I, the comparison of *tuf* sequences from a variety of bacterial and eukaryotic species allowed the selection of PCR primers which are universal for the detection of bacteria. The strategy used to design the PCR primers was based on the analysis of a multiple sequence alignment of various *tuf* sequences. This multiple sequence alignment includes *tuf* sequences from 38 bacterial species and 3 eukaryotic species either determined by us or selected from databases (Table 13). A careful analysis of this multiple sequence alignment allowed the selection of primer sequences which are conserved within eubacteria but which discriminate sequences from eukaryotes, thereby permitting the universal detection of bacteria. As shown in Annex I, the selected primers contain several inosines and degenerescences. This was necessary because there is a relatively high polymorphism among bacterial *tuf* sequences despite the fact that this gene is highly conserved. In fact, among the *tuf* sequences that we determined, we found many nucleotide variations as well as some deletions and/or insertions of amino acids. The selected universal primers were specific and ubiquitous for bacteria (Table 7). Of the 95 most clinically important bacterial species tested, 12 were not amplified. These species belong to the genera *Corynebacterium* (11 species) and *Stenotrophomonas* (1 species). The universal primers did not amplify DNA of non-bacterial origin, including human and other types of eukaryotic DNA.

### EXAMPLE 2 :

Selection of genus-specific PCR primers from *tuf* sequences. As shown in Annexes 2 and 3, the comparison of *tuf* sequences from a variety of bacterial species allowed the selection of PCR primers specific for *Enterococcus* spp. or for *Staphylococcus* spp. The strategy used to design the PCR primers was based on the analysis of a multiple sequence alignment of various *tuf* sequences. These multiple sequence alignments include the *tuf* sequences of four representative bacterial species selected from each target genus as well as *tuf* sequences from species of other closely related bacterial genera. A careful analysis of those alignments allowed the selection of oligonucleotide sequences which are conserved within the target genus but which discriminate sequences from other closely related genera, thereby permitting the genus-specific and ubiquitous detection and identification of the target bacterial genus.

For the selection of primers specific for *Enterococcus* spp. (Annex II), we have sequenced a portion of approximately 890 bp of the *tuf* genes for *Enterococcus avium, E. faecalis, E. faecium* and *E*. *gallinarum.* All other *tuf* sequences used in the alignment were either sequenced by us or selected from databases. The analysis of this sequence alignment led to the selection of a primer pair specific and ubiquitous for *Enterococcus* spp. (Table 7). All of the 11 enterococcal species tested were efficiently amplified and there was no amplification with genomic DNA from bacterial species of other genera.

For the selection of primers specific for *Staphylococcus* spp. (Annex III), we have also sequenced a portion of approximately 890 bp of the *tuf* genes for *Staphylococcus aureus, S. epidermidis, S. saprophyticus* and *S*. *simulans.* All other *tuf* sequences used in the alignment were either sequenced by us or selected from databases. The analysis of this sequence alignment led to the selection of two primer pairs specific and ubiquitous for *Staphylococcus* spp. (Table 7). Annex III shows the strategy used to select one of these two PCR primer pairs. The same strategy was used to select the other primer pair. Of the 14 staphylococcal species tested, one (*S. sciuri*) could not be amplified by the *Staphylococcus-*specific PCR assays using either one of these two primer pairs. For PCR assays using either one of these two primer pairs, there was no amplification with DNA from species of other bacterial genera.

### EXAMPLE 3 :

Selection from *tuf* sequences of PCR primers specific for *Candida albicans.* As shown in Annex IV, the comparison of *tuf* sequences from a variety of bacterial and eukaryotic species allowed the selection of PCR primers specific for *Canada albicans.* The strategy used to design the PCR primers was based on the analysis of a multiple sequence alignment of various *tuf* sequences. This multiple sequence alignment includes *tuf* sequences of five representative fungal species selected from the genus *Candida* which were determined by our group (i.e. *C. albicans, C. glabrata, C. krusei, C. parapsilosis* and *C. tropicalis)* as well as *tuf* sequences from other closely related fungal species. *tuf* sequences from various bacterial species were also included. A careful analysis of this sequence alignment allowed the selection of primers from the *C. albicans tuf* sequence; these primers discriminate sequences from other closely related *Candida* species and other fungal species, thereby permitting the species-specific and ubiquitous detection and identification of *C*. *albicans* (Table 7). All of 88 *Candida albicans* strains tested were efficiently amplified and there was no amplification with genomic DNA from other fungal or bacterial species.

### EXAMPLE 4:

Selection of PCR primers specific for *Streptococcus* from *recA.* As shown in Annex V, the comparison of the various bacterial *recA* gene sequences available from databases (GenBank and EMBL) was used as a basis for the selection of PCR primers which are specific and ubiquitous for the bacterial genus *Streptococcus.* Since sequences of the *recA* gene are available for many bacterial species including five species of streptococci, it was possible to choose sequences well conserved within the genus *Streptococcus* but distinct from the *recA* sequences for other bacterial genera. When there were mismatches between the *recA* gene sequences from the five *Streptococcus* species, an inosine residue was incorporated into the primer (Annex V). The selected primers, each containing one inosine and no degenerescence, were specific and ubiquitous for *Streptococcus* species (Table 7). This PCR assay amplified all of the 22 streptococcal species tested. However, the *Streptococcus*-specific assay did not amplify DNA from 3 out of 9 strains of *S. mutans* and 1 out of 3 strains of *S*. *salivarius.* There was no amplification with genomic DNA from other bacterial genera (Table 7).

### EXAMPLE 5:

Nucleotide sequencing of DNA fragments. The nucleotide sequence of a portion of the *tuf* genes from a variety of bacterial or fungal species was determined by using the dideoxynucleotide chain termination sequencing method (Sanger et al., 1977, Proc. Natl. Acad. Sci. USA. 74:5463-5467). The sequencing was performed by using an Applied Biosystems automated DNA sequencer (model 373A) with their PRISM™ Sequenase® Terminator Double-stranded DNA Sequencing Kit (Perkin-Elmer Corp., Applied Biosystems Division, Foster City, CA). The sequencing strategy does not discriminate *tufA* and *tufB* genes because the sequencing primers hybridize efficiently to both bacterial *tuf* genes. These DNA sequences are shown in the sequence listing (SEQ ID Nos: 118 to 146). The presence of several degenerated nucleotides in the various *tuf* sequences determined by our group (Table 13) corresponds to sequence variations between *tufA* and *tufB.*

Oligonucleotide primers and probes selection. Oligonucleotide probes and amplification primers were selected from the given proprietary DNA fragments or database sequences using the Oligo™ program and were synthesized with an automated ABI DNA synthesizer (Model 391, Perkin-Elmer Corp., Applied Biosystems Division) using phosphoramidite chemistry.

### EXAMPLE 6 :

Labeling of oligonucleotides for hybridization assays. Each oligonucleotide was 5' end-labeled with γ-³²P (dATP) by the T4 polynucleotide kinase (Pharmacia) as described earlier. The label could also be non-radioactive.

Specificity test for Oligonucleotide probes. All labeled oligonucleotide probes were tested for their specificity by hybridization to DNAs from a variety of bacterial and fungal species selected from Tables 4, 5 and 6 as described earlier. Species-specific or genus-specific probes were those hybridizing only to DNA from the microbial species or genus from which it was isolated. Oligonucleotide probes found to be specific were submitted to ubiquity tests as follows.

Ubiquity test for oligonucleotide probes. Specific oligonucleotide probes were then used in ubiquity tests with strains of the target species or genus including reference strains and other strains obtained from various countries and which are representative of the diversity within each target species or genus. Chromosomal DNAs from the isolates were transferred onto nylon membranes and hybridized with labeled oligonucleotide probes as described for specificity tests. The batteries of isolates constructed for each target species or genus contain reference ATCC strains as well as a variety of clinical isolates obtained from various sources. Ubiquitous probes were those hybridizing to at least 80% of DNAs from the battery of clinical isolates of the target species or genus.

### EXAMPLE 7:

Same as example 6 except that a pool of specific oligonucleotide probes is used for microbial identification (i) to increase sensitivity and assure 100% ubiquity or (ii) to identify simultaneously more than one microbial species and/or genus. Microbial identification could be performed from microbial cultures or directly from any clinical specimen.

### EXAMPLE 8:

Same as example 6 except that bacteria or fungi were detected directly from clinical samples. Any biological sample was loaded directly onto a dot blot apparatus and cells were lysed *in situ* for bacterial or fungal detection and identification. Blood samples should be heparizined in order to avoid coagulation interfering with their convenient loading on a dot blot apparatus.

### EXAMPLE 9:

PCR amplification. The technique of PCR was used to increase the sensitivity and the rapidity of the assays. The sets of primers were tested in PCR assays performed directly from bacterial colonies or from a standardized bacterial suspension (see Example 10) to determine their specificity and ubiquity (Table 7). Examples of specific and ubiquitous PCR primer pairs are listed in Annex VI.

Specificity and ubiquity tests for amplification primers. The specificity of all selected PCR primer pairs was tested against DNAs from a variety of bacterial and fungal species selected from Tables 4, 5 and 6 as described earlier. Primer pairs found specific for each species or genus were then tested for their ubiquity to ensure that each set of primers could amplify at least 90% of DNAs from a battery of isolates of the target species or genus. The batteries of isolates constructed for each species contain reference ATCC strains and various clinical isolates from around the world which are representative of the diversity within each species or genus.

Standard precautions to avoid false positive PCR results should be taken (Kwok and Higuchi, 1989, Nature, 239:237-238). Methods to inactivate PCR amplification products such as the inactivation by uracil-N-glycosylase may be used to control PCR carryover.

### EXAMPLE 10:

Amplification directly from bacterial or yeast cultures. PCR assays were performed either directly from a bacterial colony or from a bacterial suspension, the latter being adjusted to a standard McFarland 0.5 (corresponds to approximately 1.5 x 10⁸ bacteria/mL). In the case of direct amplification from a colony, a portion of a colony was transferred using a plastic rod directly into a 20 µL PCR reaction mixture containing 50 mM KCl, 10 mM Tris-HCl (pH 9.0), 0.1% Triton X-100, 2.5 mM MgCl₂, 0.4 µM of each primer, 200 µM of each of the four dNTPs and 0.5 unit of *Taq* DNA polymerase (Promega) combined with the TaqStart™ antibody (Clontech Laboratories Inc.). For the bacterial suspension, 1 µL of the cell suspension was added to 19 µL of the same PCR reaction mixture. For the identification from yeast cultures, 1 µL of a standard McFarland 1.0 (corresponds to approximately 3.0 x 10⁸ bacteria/mL) concentrated 100 times by centrifugation was added directly to the PCR reaction. This concentration step for yeast cells was performed because a McFarland 0.5 for yeast cells has approximately 200 times fewer cells than a McFarland 0.5 for bacterial cells.

PCR reactions were then subjected to thermal cycling (3 min at 95°C followed by 30 cycles of 1 second at 95°C for the denaturation step and 30 seconds at 55°C for the annealing-extension step) using a PTC-200 thermal cycler. PCR amplification products were then analyzed by standard agarose gel (2%) electrophoresis. Amplification products were visualized in agarose gels containing 0.25 µg/mL of ethidium bromide under UV at 254 nm. The entire PCR assay can be completed in approximately one hour.

Primer sequences derived from highly conserved regions of the bacterial 16S ribosomal RNA gene were used to provide an internal control for all PCR reactions. Alternatively, the internal control was derived from sequences not found in microorganisms or in the human genome. The internal control was integrated into all amplification reactions to verify the efficiency of the PCR assays and to ensure that significant PCR inhibition was absent. The internal control derived from rRNA was also useful to monitor the efficiency of the bacterial lysis protocols. The internal control and the species-specific or genus-specific amplifications were performed simultaneously in multiplex PCR assays.

### EXAMPLE 11:

Amplification directly from urine specimens. For PCR amplification performed directly from urine specimens, 1 µL of urine was mixed with 4 µL of a lysis solution containing 500 mM KCl, 100 mM tris-HCl (pH 9.0), 1% triton X-100. After incubation for at least 15 minutes at room temperature, 1 µL of the treated urine specimen was added directly to 19 µL of the PCR reaction mixture. The final concentration of the PCR reagents was 50 mM KCl, 10 mM Tris (pH 9.0), 0.1% Triton X-100, 2.5 mM MgCl₂, 0.4 µM of each primer, 200 µM of each of the four dNTPs. In addition, each 20 µL reaction contained 0.5 unit of *Taq* DNA polymerase (Promega) combined with the TaqStart™ antibody (Clontech Laboratories Inc.).

Strategies for the internal control, PCR amplification and agarose gel detection of the amplicons are as previously described in example 10.

### EXAMPLE 12:

Detection of antibiotic resistance genes. The presence of specific antibiotic resistance genes which are frequently encountered and clinically relevant is identified using the PCR amplification or hybridization protocols described previously. Specific oligonucleotides used as a basis for the DNA-based tests are selected from the antibiotic resistance gene sequences. These tests, which allow the rapid evaluation of bacterial resistance to antimicrobial agents, can be performed either directly from clinical specimens, from a standardized bacterial suspension or from a bacterial colony and should complement diagnostic tests for the universal detection of bacteria as well as for the species-specific and genus-specific microbial detection and identification.

### EXAMPLE 13:

Same as examples 10 and 11 except that assays were performed by multiplex PCR (i.e. using several pairs of primers in a single PCR reaction) to reach an ubiquity of 100% for the specific targeted pathogen(s). For more heterogeneous microbial species or genus, a combination of PCR primer pairs may be required to detect and identify all representatives of the target species or genus.

Multiplex PCR assays could also be used to (i) detect simultaneously several microbial species and/or genera or, alternatively, (ii) to simultaneously detect and identify bacterial and/or fungal pathogens and detect specific antibiotic resistance genes either directly from a clinical specimen or from bacterial cultures.

For these applications, amplicon detection methods should be adapted to differentiate the various amplicons produced. Standard agarose gel electrophoresis could be used because it discriminates the amplicons based on their sizes. Another useful strategy for this purpose would be detection using a variety of fluorescent dyes emitting at different wavelengths. The fluorescent dyes can be each coupled with a specific oligonucleotide linked to a fluorescence quencher which is degraded during amplification to release the fluorescent dyes (e.g. TaqMan™, Perkin Elmer).

### EXAMPLE 14:

Detection of amplification products. The person skilled in the art will appreciate that alternatives other than standard agarose gel electrophoresis (Example 10) may be used for the revelation of amplification products. Such methods may be based on fluorescence polarization or on the detection of fluorescence after amplification (e.g. Amplisensor™, Biotronics; TaqMan™, Perkin-Elmer Corp.) or other labels such as biotin (SHARP Signal™ system, Digene Diagnostics). These methods are quantitative and may be automated. One of the amplification primers or an internal oligonucleotide probe specific to the amplicon(s) derived from the species-specific, genus-specific or universal DNA fragments is coupled with the fluorescent dyes or with any other label. Methods based on the detection of fluorescence are particularly suitable for diagnostic tests since they are rapid and flexible as fluorescent dyes emitting at different wavelengths are available.

### EXAMPLE 15:

Species-specific, genus-specific, universal and antibiotic resistance gene amplification primers can be used in other rapid amplification procedures such as the ligase chain reaction (LCR), transcription-mediated amplification (TMA), self-sustained sequence replication (3SR), nucleic acid sequence-based amplification (NASBA), strand displacement amplification (SDA), cycling probe technology (CPT) and branched DNA (bDNA) or any other methods to increase the sensitivity of the test. Amplifications can be performed from isolated bacterial cultures or directly from any clinical specimen. The scope of this invention is therefore not limited to the use of the DNA sequences from the enclosed Sequence Listing for PCR only but rather includes the use of any procedures to specifically detect bacterial DNA and which may be used to increase rapidity and sensitivity of the tests.

### EXAMPLE 16:

A test kit would contain sets of probes specific for each microbial species or genus as well as a set of universal probes. The kit is provided in the form of test components, consisting of the set of universal probes labeled with non-radioactive labels as well as labeled species- or genus-specific probes for the detection of each pathogen of interest in specific types of clinical samples. The kit will also include test reagents necessary to perform the pre-hybridization, hybridization, washing steps and hybrid detection. Finally, test components for the detection of known antibiotic resistance genes (or derivatives therefrom) will be included. Of course, the kit will include standard samples to be used as negative and positive controls for each hybridization test.

Components to be included in the kits will be adapted to each specimen type and to detect pathogens commonly encountered in that type of specimen. Reagents for the universal detection of bacteria will also be included. Based on the sites of infection, the following kits for the specific detection of pathogens may be developed:
- A kit for the universal detection of bacterial or fungal pathogens from all clinical specimens which contains sets of probes specific for highly conserved regions of the microbial genomes.
- A kit for the detection of microbial pathogens retrieved from urine samples, which contains 5 specific test components (sets of probes for the detection of *Enterococcus faecium, Enteroccus* species, *Staphylococcus saprophyticus, Staphylococcus* species and *Candida albicans).*
- A kit for the detection of respiratory pathogens which contains 3 specific test components (sets of probes for the detection of *Staphylococcus* species, *Enterococcus* species and *Candida albicans*).
- A kit for the detection of pathogens retrieved from blood samples, which contains 10 specific test components (sets of probes for the detection of *Streptococcus* species, *Streptococcus agalactiae, Staphylococcus* species, *Staphylococcus saprophyticus, Enterococcus* species, *Enterococcus faecium, Neisseria* species, *Neisseria meningitidis, Listeria monocytogenes* and *Candida albicans*). This kit can also be applied for direct detection and identification from blood cultures.

- A kit for the detection of pathogens causing meningitis, which contains 5 specific test components (sets of probes for the detection of *Streptococcus* species, *Listeria monocytogenes, Neisseria meningitidis, Neisseria* species and *Staphylococcus* species).
- A kit for the detection of clinically important antibiotic resistance genes which contains sets of probes for the specific detection of at least one of the 26 following genes associated with antibiotic resistance: *blaₜₑₘ, bla_{rob}, bla_{shw}, blaₒₓₐ, blaZ, aadB, aacC1, aacC2, aacC3, aacA4, aac6'-IIa, ermA, ermB, ermC, mecA, vanA, vanB, vanC, satA, aac(6')-aph(2"), aad(6'), vat, vga, msrA, sul* and *int.*
- Other kits adapted for the detection of pathogens from skin, abdominal wound or any other clinically relevant infections may also be developed.

### EXAMPLE 17:

Same as example 16 except that the test kits contain all reagents and controls to perform DNA amplification assays. Diagnostic kits will be adapted for amplification by PCR (or other amplification methods) performed directly either from clinical specimens or from microbial cultures. Components required for (i) universal bacterial detection, (ii) species-specific and genus-specific bacterial and/or fungal detection and identification and (iii) detection of antibiotic resistance genes will be included.

Amplification assays could be performed either in tubes or in microtitration plates having multiple wells. For assays in plates, the wells will contain the specific amplification primers and control DNAs and the detection of amplification products will be automated. Reagents and amplification primers for universal bacterial detection will be included in kits for tests performed directly from clinical specimens. Components required for species-specific and genus-specific bacterial and/or fungal detection and identification as well as for the simultaneous antibiotic resistance genes detection will be included in kits for testing directly from bacterial or fungal cultures as well as in kits for testing directly from any type of clinical specimen.

The kits will be adapted for use with each type of specimen as described in example 16 for hybridization-based diagnostic kits.

### EXAMPLE 18:

It is understood that the use of the probes and amplification primers described or claimed herein for bacterial and/or fungal detection and identification is not limited to clinical microbiology applications. In fact, we feel that other sectors could also benefit from these new technologies. For example, these tests could be used by industries for quality control of food, water, air, pharmaceutical products or other products requiring microbiological control. These tests could also be applied to detect and identify bacteria or fungi in biological samples from organisms other than humans (e.g. other primates, birds, plants, mammals, farm animals, livestock and others). These diagnostic tools could also be very useful for research purposes including clinical trials and epidemiological studies.

**Table 1. Distribution (%) of nosocomial pathogens for various human infections in USA (1990-1992)¹.**

| Pathogen | UTI² | SSI³ | BSI⁴ | Pneumonia | CSF⁵ |
|---|---|---|---|---|---|
| *Escherichia coli* | 27 | 9 | 5 | 4 | 2 |
| *Staphylococcus aureus* | 2 | 21 | 17 | 21 | 2 |
| *Staphylococcus epidermidis* | 2 | 6 | 20 | 0 | 1 |
| *Enterococcus faecalis* | 16 | 12 | 9 | 2 | 0 |
| *Enterococcus faecium* | 1 | 1 | 0 | 0 | 0 |
| *Pseudomonas aeruginosa* | 12 | 9 | 3 | 18 | 0 |
| *Klebsiella pneumoniae* | 7 | 3 | 4 | 9 | 0 |
| *Proteus mirabilis* | 5 | 3 | 1 | 2 | 0 |
| *Streptococcus pneumoniae* | 0 | 0 | 3 | 1 | 18 |
| Group B *Streptococci* | 1 | 1 | 2 | 1 | 6 |
| Other *Streptococci* | 3 | 5 | 2 | 1 | 3 |
| *Haemophilus influenzae* | 0 | 0 | 0 | 6 | 45 |
| *Neisseria meningitidis* | 0 | 0 | 0 | 0 | 14 |
| *Listeria monocytogenes* | 0 | 0 | 0 | 0 | 3 |
| Other *Enterococci* | 1 | 1 | 0 | 0 | 0 |
| Other *Staphylococci* | 2 | | 8 | 13 | 20 |
| *Candida albicans* | 9 | 3 | 5 | 5 | 0 |
| Other *Candida* | 2 | | 1 | 3 | 10 |
| *Enterobacter* spp. | 5 | 7 | 4 | 12 | 2 |
| *Acinetobacter* spp. | 1 | 1 | 2 | 4 | 2 |
| *Citrobacter* spp. | 2 | 1 | 1 | 1 | 0 |
| *Serratia marcescens* | 1 | 1 | 1 | 3 | 1 |
| Other *Klebsiella* | 1 | 1 | 1 | 2 | 1 |
| Others | 0 | 6 | 4 | 5 | 0 |

| | | | | | |
|---|---|---|---|---|---|
| ¹ Data recorded by the National Nosocomial Infections Surveillance (NNIS) from 80 hospitals (Emori and Gaynes, 1993, Clin. Microbiol. Rev., 6:428-442). ² Urinary tract infection. ³ Surgical site infection. ⁴ Bloodstream infection. ⁵ Cerebrospinal fluid. | | | | | |

**Table 2. Distribution (%) of bloodstream infection pathogens in Quebec (1995), Canada (1992), UK (1969-1988) and USA (1990-1992).**

| Organism | Quebec¹ | Canada² | UK³ | | USA⁴ |
|---|---|---|---|---|---|
| | | | Community-acquired | Hospital-acquired | Hospital-acquired |
| *E. coli* | 15.6 | 53.8 | 24.8 | 20.3 | 5.0 |
| *S. epidermidis* and other CoNS⁵ | 25.8 | NI⁶ | 0.5 | 7.2 | 31.0 |
| *S. aureus* | 9.6 | NI | 9.7 | 19.4 | 16.0 |
| *S. pneumoniae* | 6.3 | NI | 22.5 | 2.2 | NR⁷ |
| *E. faecalis* | 3.0 | NI | 1.0 | 4.2 | NR |
| *E. faecium* | 2.6 | NI | 0.2 | 0.5 | NR |
| *Enterococcus* spp. | NR | NI | NR | NR | 9.0 |
| *H. influenza* | 1.5 | NR | 3.4 | 0.4 | NR |
| *P. aeruginosa* | 1.5 | 8.2 | 1.0 | 8.2 | 3.0 |
| *K. pneumoniae* | 3.0 | 11.2 | 3.0 | 9.2 | 4.0 |
| *P. mirabilis* | NR | 3.9 | 2.8 | 5.3 | 1.0 |
| *S. pyogenes* | NR | NI | 1.9 | 0.9 | NR |
| *Enterobacter* spp. | 4.1 | 5.5 | 0.5 | 2.3 | 4.0 |
| *Candida* spp. | 8.5 | NI | NR | 1.0 | 8.0 |
| Others | 18.5 | 17.4⁸ | 28.7 | 18.9 | 19.0 |

| | | | | | |
|---|---|---|---|---|---|
| ¹ Data obtained for 270 isolates collected at the Centre Hospitalier de l'Université Laval (CHUL) during a 5 month period (May to October 1995). ² Data from 10 hospitals throughout Canada representing 941 gram-negative bacterial isolates. (Chamberland *et al.,* 1992, Clin. Infect. Dis., **15**:615-628). ³ Data from a 20-year study (1969-1988) for nearly 4000 isolates (Eykyn *et al.,* 1990, J. Antimicrob. Chemother., Suppl. C, **25**:41-58). ⁴ Data recorded by the National Nosocomial Infections Surveillance (NNIS) from 80 hospitals (Emori and Gaynes, 1993, Clin. Microbiol. Rev., **6**:428-442). ⁵ Coagulase-negative staphylococci. ⁶ NI, not included. This survey included only gram-negative species. ⁷ NR, incidence not reported for these species or genera. ⁸ In this case, 17.4 stands for other gram-negative bacterial species. | | | | | |

**Table 3. Distribution of positive and negative clinical specimens tested at the microbiology laboratory of the CHUL (February 1994 - January 1995).**

| Clinical specimens and/or sites | No. of samples tested (%) | % of positive specimens | % of negative specimens |
|---|---|---|---|
| Urine | 17,981 (54.5) | 19.4 | 80.6 |
| Blood culture/marrow | 10,010 (30.4) | 6.9 | 93.1 |
| Sputum | 1,266 (3.8) | 68.4 | 31.6 |
| Superficial pus | 1,136 (3.5) | 72.3 | 27.7 |
| Cerebrospinal fluid | 553 (1.7) | 1.0 | 99.0 |
| Synovial fluid | 523 (1.6) | 2.7 | 97.3 |
| Respiratory tract | 502 (1.5) | 56.6 | 43.4 |
| Deep pus | 473 (1.4) | 56.8 | 43.2 |
| Ears | 289 (0.9) | 47.1 | 52.9 |
| Pleural and pericardial fluid | 132 (0.4) | 1.0 | 99.0 |
| Peritoneal fluid | 101(0.3) | 28.6 | 71.4 |
| **Total:** | **32,966 (100.0)** | **20.0** | **80.0** |

**Table 4. Gram-negative bacterial species (90) used to test the specificity of PCR primers and DNA probes (continues on next page).**

| Bacterial species | Number of reference strains tested^{a} | Bacterial species | Number of reference strains tested^{a} |
|---|---|---|---|
| *Acinetobacter baumannii* | 1 | *Moraxella phenylpyruvica* | 1 |
| *Acinetobacter lwoffii* | 3 | *Morganella morganii* | 1 |
| *Actinobacillus lignieresii* | 1 | *Neisseria animalis* | 1 |
| *Alcaligenes faecalis* | 1 | *Neisseria canis* | 1 |
| *Alcaligenes odorans* | 1 | *Neisseria caviae* | 1 |
| *Alcaligenes xylosoxydans* | | *Neisseria cinerea* | 1 |
| subsp. *denitrificans* | 1 | *Neisseria cuniculi* | 1 |
| *Bacteroides distasonis* | 1 | *Neisseria elongata* | 1 |
| | | subsp. *elongata* | |
| *Bacteroides fragilis* | 1 | *Neisseria elongata* | 1 |
| | | subsp. *glycoytica* | |
| *Bacteroides ovatus* | 1 | *Neisseria flavescens* | 1 |
| *Bacteroides thetaiotaomicron* | 1 | *Neisseria flavescens Branham* | 1 |
| *Bacteroides vulgatus* | 1 | *Neisseria gonorrhoeae* | 18 |
| *Bordetella bronchiseptica* | 1 | *Neisseria lactamica* | 1 |
| *Bordetella parapertussis* | 1 | *Neisseria meningitidis* | 4 |
| *Bordetella pertussis* | 2 | *Neisseria mucosa* | 2 |
| *Burkholderia cepacia* | 1 | *Neisseria polysaccharea* | 1 |
| *Citrobacter amalonaticus* | 1 | *Neisseria sicca* | 3 |
| *Citrobacter diversus* subsp. *koseri* | 2 | *Neisseria subflava* | 3 |
| *Citrobacter freundii* | 1 | *Neisseria weaveri* | 1 |
| *Comamonas acidovorans* | 1 | *Ochrobactrum antropi* | 1 |
| *Enterobacter aerogenes* | 1 | *Pasteurella aerogenes* | 1 |
| *Enterobacter agglomerans* | 1 | *Pasteurella multocida* | 1 |
| *Enterobacter cloacae* | 1 | *Prevotella melaninogenica* | 1 |
| *Escherichia coli* | 9 | *Proteus mirabilis* | 3 |
| *Escherichia fergusonii* | 1 | *Proteus vulgaris* | 1 |
| *Escherichia hermannii* | 1 | *Providencia alcalifaciens* | 1 |
| *Escherichia vulneris* | 1 | *Providencia rettgeri* | 1 |
| *Flavobacterium meningosepticum* | 1 | *Providencia rustigianii* | 1 |
| *Flavobacterium indologenes* | 1 | *Providencia stuartii* | 1 |
| *Flavobacterium odoratum* | 1 | *Pseudomonas aeruginosa* | 14 |
| *Fusobacterium necrophorum* | 2 | *Pseudomonas fluorescens* | 2 |
| *Gardnerella vaginalis* | 1 | *Pseudomonas stutzeri* | 1 |
| *Haemophilus haemolyticus* | 1 | *Salmonella arizonae* | 1 |
| *Haemophilus influenzae* | 12 | *Salmonella choleraesuis* | 1 |
| *Haemophilus parahaemolyticus* | 1 | *Salmonella gallinarum* | 1 |
| *Haemophilus parainfluenzae* | 2 | *Salmonella typhimurium* | 3 |
| *Hafnia alvei* | 1 | *Serratia liquefaciens* | 1 |
| *Kingella indologenes* subsp. *suttonella* | 1 | *Serratia marcescens* | 1 |
| *Kingella kingae* | 1 | *Shewanella putida* | 1 |
| *Klebsiella ornithinolytica* | 1 | *Shigella boydii* | 1 |
| *Klebsiella oxytoca* | 1 | *Shigella dysenteriae* | 1 |
| *Klebsiella pneumoniae* | 8 | *Shigella flexneri* | 1 |
| *Moraxella atlantae* | 1 | *Shigella sonnei* | 1 |
| *Moraxella catarrhalis* | 5 | *Stenotrophomonas maltophilia* | 1 |
| *Moraxella lacunata* | 1 | *Yersinia enterocolitica* | 1 |
| *Moraxella osloensis* | 1 | | |

| | | | |
|---|---|---|---|
| ^{a} Most reference strains were obtained from the American Type Culture Collection (ATCC). The other reference strains were obtained from (i) the Laboratoire de Santé Publique du Québec (LSPQ), (ii) the Center for Disease Control and Prevention (CDC) and (iii) the National Culture Type Collection (NCTC). | | | |

**Table 5. Gram-positive bacterial species (97) used to test the specificity of PCR primers and DNA probes (continues on next page).**

| Bacterial species | Number of reference strains tested^{a} | Bacterial species | Number of reference strains tested^{a} |
|---|---|---|---|
| *Abiotrophia adiacens* | 1 | *Micrococcus kristinae* | 1 |
| *Abiotrophia defectiva* | 1 | *Micrococcus luteus* | 1 |
| *Actinomyces israelii* | 1 | *Micrococcus lylae* | 1 |
| *Clostridium perfringens* | 1 | *Micrococcus roseus* | 1 |
| *Corynebacterium accolens* | 1 | *Micrococcus varians* | 1 |
| *Corynebacterium aquaticum* | 1 | *Peptococcus niger* | 1 |
| *Corynebacterium bovis* | 1 | *Peptostreptococcus anaerobius* | 1 |
| *Corynebacterium cervicis* | 1 | *Peptostreptococcus asaccharolyticus* | 1 |
| *Corynebacterium diphteriae* | 6 | *Staphylococcus aureus* | 10 |
| *Corynebacterium flavescens* | 1 | *Staphylococcus auricularis* | 1 |
| *Corynebacterium genitalium* | 6 | *Staphylococcus capitis* subsp. *urealyticus* | 1 |
| *Corynebacterium jeikeium* | 1 | *Staphylococcus cohnii* | 1 |
| *Corynebacterium kutcheri* | 1 | *Staphylococcus epidermidis* | 2 |
| *Corynebacterium matruchotii* | 1 | *haemolyticus* | 2 |
| *Corynebacterium minutissimum* | 1 | *Staphylococcus hominis* | 2 |
| *Corynebacterium mycetoides* | 1 | *Staphylococcus lugdunensis* | 1 |
| *Corynebacterium pseudodiphtheriticum* | 1 | *Staphylococcus saprophyticus* | 3 |
| *Corynebacterium pseudogenitalium* | 6 | *Staphylococcus schleiferi* | 1 |
| *Corynebacterium renale* | 1 | *Staphylococcus sciuri* | 1 |
| *Corynebacterium striatum* | 1 | *Staphylococcus imulans* | 1 |
| *Corynebacterium ulcerans* | 1 | *Staphylococcus warneri* | 1 |
| *Corynebacterium urealyticum* | 1 | *Staphylococcus xylosus* | 1 |
| *Corynebacterium xerosis* | 1 | *Streptococcus agalactiae* | 6 |
| *Enterococcus avium* | 1 | *Streptococcus anginosus* | 2 |
| *Enterococcus casseliflavus* | 1 | *Streptococcus bovis* | 2 |
| *Enterococcus cecorum* | 1 | *Streptococcus constellatus* | 1 |
| *Enterococcus dispar* | 1 | *Streptococcus crista* | 1 |
| *Enterococcus durans* | 1 | *Streptococcus dysgalactiae* | 1 |
| *Enterococcus faecalis* | 6 | *Streptococcus equi* | 1 |
| *Enterococcus faecium* | 3 | *Streptococcus gordonii* | 1 |
| *Enterococcus flavescens* | 1 | *Group C Streptococci* | 1 |
| *Enterococcus gallinarum* | 3 | *Group D Streptococci* | 1 |
| *Enterococcus hirae* | 1 | *Group E Streptococci* | 1 |
| *Enterococcus mundtii* | 1 | *Group F Streptococci* | 1 |
| *Enterococcus pseudoavium* | 1 | *Group G Streptococci* | 1 |
| *Enterococcus raffinosus* | 1 | *Streptococcus intermedius* | 1 |
| *Enterococcus saccharolyticus* | 1 | *Streptococcus mitis* | 2 |
| *Enterococcus solitarius* | 1 | *Streptococcus mutans* | 1 |
| *Eubacterium lentum* | 1 | *Streptococcus oralis* | 1 |
| *Gemella haemolysans* | 1 | *Streptococcus parasanguis* | 1 |
| *Gemella morbillorum* | 1 | *Streptococcus pneumoniae* | 6 |
| *Lactobacillus acidophilus* | 1 | *Streptococcus pyogenes* | 3 |
| *Listeria innocua* | 1 | *Streptococcus salivarius* | 2 |
| *Listeria ivanovii* | 1 | *Streptococcus sanguis* | 2 |
| *Listeria grayi* | 1 | *Streptococcus sobrinus* | 1 |
| *Listeria monocytogenes* | 3 | *Streptococcus suis* | 1 |
| *Listeria murrayi* | 1 | *Streptococcus uberis* | 1 |
| *Listeria seeligeri* | 1 | *Streptococcus vestibularis* | 1 |
| *Listeria welshimeri* | 1 | | |

| | | | |
|---|---|---|---|
| ^{a} Most reference strains were obtained from the American Type Culture Collection (ATCC). The other reference strains were obtained from (i) the Laboratoire de Santé Publique du Québec (LSPQ), (ii) the Center for Disease Control and Prevention (CDC) and (iii) the National Culture Type Collection (NCTC). | | | |

**Table 6. Fungal species (12) used to test the specificity of PCR primers and DNA probes.**

| Fungal species | Number of reference strains tested^{a} |
|---|---|
| *Candida albicans* | 12 |
| *Candida glabrata* | 1 |
| *Candida guilliermondii* | 1 |
| *Candida kefyr* | 3 |
| *Candida krusei* | 2 |
| *Candida lusitaniae* | 1 |
| *Candida parapsilosis* | 2 |
| *Candida tropicalis* | 3 |
| *Rhodotorula glutinis* | 1 |
| *Rhodotorula minuta* | 1 |
| *Rhodotorula rubra* | 1 |
| *Saccharomyces cerevisiae* | 1 |

| | |
|---|---|
| ^{a} Most reference strains were obtained from (i) the American Type Culture Collection (ATCC) and (ii) the Laboratoire de Santé Publique du Québec (LSPQ). | |

**Table 7. PCR assays developed for several clinically important bacterial and fungal pathogens (continues on next page).**

| Organism | Primer Pair^{a} SEQ ID NO | Amplicon size (bp) | Ubiquity^{b} | DNA amplification from | |
|---|---|---|---|---|---|
| | | | | culture^{c} | specimens^{d} |
| *Enterococcus faecium* | 1-2 | 216 | 79/80 | + | + |
| *Listeria monocytogenes* | 3-4 | 130 | 164/168^{e} | + | + |
| *Neisseria meningitidis* | 5-6 | 177 | 258/258 | + | + |
| *Staphylococcus saprophyticus* | 7-8 | 149 | 245/260 | + | NT |
| Streptococcus agalactiae | 9-10 | 154 | 29/29 | + | + |
| *Candida albicans* | 11-12 | 149 | 88/88 | + | NT |
| *Enterococcus* spp. (11 species)^{f} | 13-14 | 112 | 87/87 | + | NT |
| *Neisseria* spp. (12 species)^{f} | 15-16 | 103 | 321/321 | + | + |
| *Staphylococcus* spp. (14 species) | 17-18 | 192 | 13/14 | + | NT |
| | 19-20 | 221 | 13/14 | + | NT |
| *Streptococcus* spp. (22 species)^{f} | 21-22 | 153 | 210/214^{g} | + | + |
| Universal detection^{h} (95 species)¹ | 23-24 | 309 | 104/ 116ⁱ | + | + |

| | | | | | |
|---|---|---|---|---|---|
| ^{a} All primer pairs are specific in PCR assays since no amplification was observed with DNA from the bacterial and fungal species other than the species of interest listed in Tables 4, 5 and 6. ^{b} Ubiquity was tested by using reference strains as well as strains from throughout the world, which are representatite of the diversity within each target species or genus. ^{c} For all primer pairs, PCR amplifications performed directly from a standardized ^{d} microbial suspension (MacFarland) or from a colony were all specific and ubiquitous. ^{d} PCR assays performed directly from blood cultures, urine specimens or cerebrospinal fluid. NT, not tested. ^{e} The four L. *monocytogenes* strains undetected are not clinical isolates. These strains were isolated from food and are not associated with a human infection. ^{f} The bacterial species tested include all those clinically relevant for each genus (Tables 4 and 5). All of these species were efficiently amplified by their respective genus-specific PCR assay, except for the *Siaphylococcus-specific* assay, which does not amplify S. *sciuri.* ^{g} The *Streptococcus-specific* PCR assay did not amplify 3 out of 9 strains of *S. mutans* and 1 out of 3 strains of *S. salivarius.* ^{h} The primers selected for universal bacterial detection do not amplify DNA of non-bacterial origin, including human and other types of eukaryotic genomic DNA. ⁱ For the universal amplification, the 95 bacterial species tested represent the most clinically important bacterial species listed in Tables 4 and 5. The 12 strains not amplified are representatives of genera *Corynebacterium* (11 species) and *Stenotrophomonas* (1 species). | | | | | |

**Table 8. Target genes for the various genus-specific, species-specific and universal amplification assays.**

| Microorganisms | Gene | Protein encoded |
|---|---|---|
| *Candida albicans* | *tuf* | translation elongation factor EF-Tu |
| *Enterococcus faecium* | *ddl* | D-alanine:D-alanine ligase |
| *Listeria monocytogenes* | *actA* | actin-assembly inducing protein |
| *Neisseria meningitidis* | *omp* | outer membrane protein |
| *Streptococcus agalactiae* | *cAMP* | cAMP factor |
| *Staphylococcus saprophyticus* | unknown | unknown |
| *Enterococcus* spp. | *tuf* | translation elongation factor EF-Tu |
| *Neisseria* spp. | *asd* | ASA-dehydrogenase |
| *Staphylococcus* spp. | *tuf* | translation elongation factor EF-Tu |
| *Streptococcus* spp. | *recA* | RecA protein |
| Universal detection | *tuf* | translation elongation factor EF- Tu |

**Table 9. Antibiotic resistance genes selected for diagnostic purposes.**

| Genes | SEQ ID NOs | | Antibiotics | Bacteria^{a} |
|---|---|---|---|---|
| | selected primers | originating fragment | | |
| | | | | |
| *blaₒₓₐ* | 49-50 | 110 | β-lactams | *Enterobacteriaceae,Ps eudomonadaceae* |
| | | | | |
| *blaZ* | 51-52 | 111 | β-lactams | *Enterococcus* spp. |
| *aac6'-IIa* | 61-64 | 112 | Aminoglycosides | *Pseudomonadaceae* |
| *ermA* | 91-92 | 113 | Macrolides | *Staphylococcus* spp. |
| *ermB* | 93-94 | 114 | Macrolides | *Staphylococcus* spp. |
| *ermC* | 95-96 | 115 | Macrolides | *Staphylococcus* spp. |
| *vanB* | 71-74 | 116 | Vancomvcin | *Enterococcus* spp. |
| *vanC* | 75-76 | 117 | Vancomycin | *Enterococcus* spp. |
| *aad(6')* | 173-174 | - | Streptomycin | *Enterococcus* spp. |

| | | | | |
|---|---|---|---|---|
| ^{a} Bacteria having high incidence for the specified antibiotic resistance genes. The presence of these antibiotic resistance genes in other bacteria is not excluded. | | | | |

**Table 10. Antibiotic resistance genes from our co-pending US (N.S. 08/526840) and PCT (PCT/CA/95/00528) patent applications for which we have selected PCR primer pairs.**

| Genes | SEQ ID NOs of selected primers | Antibiotics | Bacteria^{a} |
|---|---|---|---|
| | | | |
| *blaₜₑₘ* | 37-40 | β-lactams | *Enterobacteriaceae,Ps eudomonadaceae,Hae mophilus* spp., *Neisseria* spp. |
| *bla_{rob}* | 45-48 | β-lactams | *Haemophilus* spp., *Pasteurella* spp. |
| *blaₛₕᵥ* | 41-44 | β-lactams | *Klebsiella* spp. and other *Enterobacteriaceae* |
| | | | |
| *aadB* | 53-54 | Aminoglycosides | *Enterobacteriaceae,* |
| *aac*C1 | 55-56 | | *Pseudomonadaceae* |
| *aac*C2 | 57-58 | | |
| *aac*C3 | 59-60 | | |
| *aac*A4 | 65-66 | | |
| *mec*A | 97-98 | β-lactams | *Staphylococcus* spp. |
| *van*A | 67-70 | Vancomycin | *Enterococcus* spp. |
| *sat*A | 81-82 | Macrolides | *Enterococcus* spp. |
| *aac*(6')*-aph*(2") | 83-86 | Aminoglycosides | *Enterococcus* spp., *Staphylococcus* spp. |
| *vat* | 87-88 | Macrolides | *Staphylococcus* spp. |
| *vga* | 89-90 | Macrolides | *Staphylococcus* spp. |
| *msrA* | 77-80 | Erythromycin | *Staphylococcus* spp. |
| *int* | 99-102 | β-lactams, trimethoprim, | *Enterobacteriaceae,* |
| *sul* | 103-106 | aminoglycosides, antiseptic, chloramphenicol | *Pseudomonadaceae* |

| | | | |
|---|---|---|---|
| ^{a} Bacteria having high incidence for the specified antibiotic resistance genes. The presence of these antibiotic resistance genes in other bacteria is not excluded. | | | |

**Table 11. Correlation between disk diffusion and PCR amplification of antibiotic resistance genes in Staphylococcus species^{a}.**

| | | | Disk diffusion (Kirby-Bauer)^{b} | | |
|---|---|---|---|---|---|
| Antibiotic | Phenotype | PCR | Resistant | Intermediate | Sensitive |
| Penicillin | *bla*Z | + | 165 | 0 | 0 |
| | | - | 0 | 0 | 31 |
| Oxacillin | *mecA* | + | 51 | 11 | 4 |
| | | - | 2 | 0 | 128 |
| Gentamycin | *aac*(6')*aph*(2") | + | 24 | 18 | 6 |
| | | - | 0 | 0 | 148 |
| Erythromycin | *ermA* | + | 15 | 0 | 0 |
| | *ermB* | + | 0 | 0 | 0 |
| | *ermC* | + | 43 | 0 | 0 |
| | *msrA* | + | 4 | 0 | 0 |
| | | - | 0 | 1 | 136 |

| | | | | | |
|---|---|---|---|---|---|
| ^{a} The *Staphylococcus* strains studied include *S. aureus* (82 strains), *S. epidermidis* (83 strains), *S. hominis* (2 strains), *S. capitis* (3 strains), *S. haemolyticus* (9 strains), S. *simulans* (12 strains) and *S. warneri* (5 strains), for a total of 196 strains. ^{b} Susceptibility testing was performed by the method of Kirby-Bauer according to the protocol reccommended by the National Committee of Clinical Laboratory Standards (NCCLS). | | | | | |

**Table 12. Correlation between disk diffusion profiles and PCR amplification of antibiotic resistance genes in Enterococcus species^{a}.**

| | | | Disk diffusion (Kirby-Bauer)^{b} | |
|---|---|---|---|---|
| Antibiotic | Phenotype | PCR | Resistant | Sensitive |
| Ampicillin | *blaZ* | + | 0 | 2 |
| | | - | 1 | 30 |
| Gentamycin | *aac*(6')*aph*(2") | + | 51 | 1 |
| | | - | 3 | 38 |
| Streptomycin | *aad*(6') | + | 26 | 15 |
| | | - | 6 | 27 |
| Vancomycin | *vanA* | + | 36 | 0 |
| | *vanB* | + | 26 | 0 |
| | | - | 0 | 40 |

| | | | | |
|---|---|---|---|---|
| ^{a} The *Enterococcus* strains studied include *E. faecalis* (33 strains) and *E. faecium* (69 strains), for a total of 102 strains. ^{b} Susceptibility testing was performed by the method of Kirby-Bauer according to the protocol reccommended by the National Committee of Clinical Laboratory Standards (NCCLS). | | | | |

**Table 13. Origin of tuf sequences in the Sequence Listing (continues on next page).**

| SEQ ID NO | Bacterial or fungal species | Source |
|---|---|---|
| 118 | *Abiotrophia adiacens* | This patent |
| 119 | *Abiotrophia defectiva* | This patent |
| 120 | *Candida albicans* | This patent |
| 121 | *Candida glabrata* | This patent |
| 122 | *Candida krusei* | This patent |
| 123 | *Candida parapsilosis* | This patent |
| 124 | *Candida tropicalis* | This patent |
| 125 | *Corynebacterium accolens* | This patent |
| 126 | *Corynebacterium diphteriae* | This patent. |
| 127 | *Corynebacterium genitalium* | This patent |
| 128 | *Corynebacterium jeikeium* | This patent |
| 129 | *Corynebacterium pseudotuberculosis* | This patent |
| 130 | *Corynebacterium striatum* | This patent |
| 131 | *Enterococcus avium* | This patent |
| 132 | *Enterococcus faecalis* | This patent |
| 133 | *Enterococcus faecium* | This patent |
| 134 | *Enterococcus gallinarum* | This patent |
| 135 | *Gardnerella vaginalis* | This patent |
| 136 | *Listeria innocua* | This patent |
| 137 | *Listeria ivanovii* | This patent |
| 138 | *Listeria monocytogenes* | This patent |
| 139 | *Listeria seeligeri* | This patent |
| 140 | *Staphylococcus aureus* | This patent |
| 141 | *Staphylococcus epidermidis* | This patent |
| 142 | *Staphylococcus saprophyticus* | This patent |
| 143 | *Staphylococcus simulans* | This patent |
| 144 | *Streptococcus agalactiae* | This patent |
| 145 | *Streptococcus pneumoniae* | This patent |
| 146 | *Streptococcus salivarius* | This patent |
| 147 | *Agrobacterium tumefaciens* | Database |
| 148 | *Bacillus subtilis* | Database |
| 149 | *Bacteroides fragilis* | Database |
| 150 | *Borrelia burgdorferi* | Database |
| 151 | *Brevibacterium linens* | Database |
| 152 | *Burkholderia cepacia* | Database |
| 153 | *Chlamydia trachomatis* | Database |
| 154 | *Escherichia coli* | Database |
| 155 | *Fibrobacter succinogenes* | Database |
| 156 | *Flavobacterium ferrugineum* | Database |
| 157 | *Haemophilus influenzae* | Database |
| 158 | *Helicobacter pylori* | Database |
| 159 | *Micrococcus luteus* | Database |
| 160 | *Mycobacterium tuberculosis* | Database |
| 161 | *Mycoplasma genitalium* | Database |
| 162 | *Neisseria gonorrhoeae* | Database |
| 163 | *Rickettsia prowazekii* | Database |
| 164 | *Salmonella typhimurium* | Database |
| 165 | *Shewanella putida* | Database |
| 166 | *Stigmatella aurantiaca* | Database |
| 167 | *Streptococcus pyogenes* | Database |
| 168 | *Thiobacillus cuprinus* | Database |
| 169 | *Treponema pallidum* | Database |
| 170 | *Ureaplasma urealyticum* | Database |
| 171 | *Wolinella succinogenes* | Database |

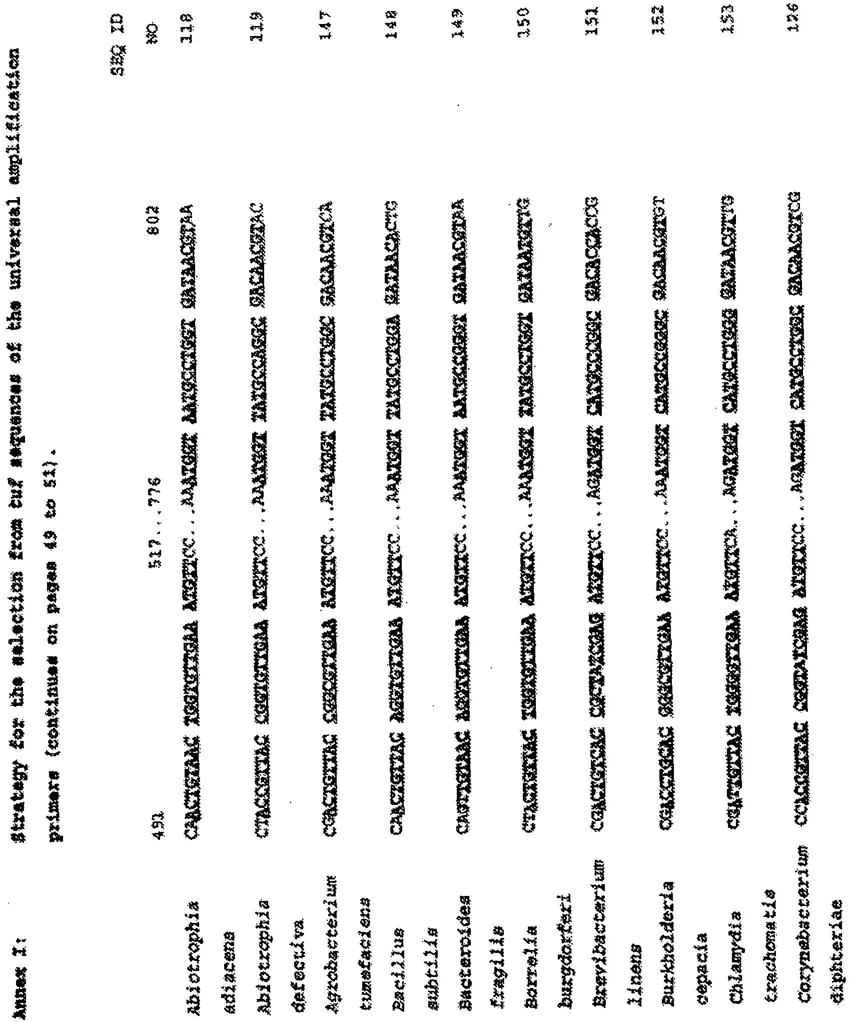

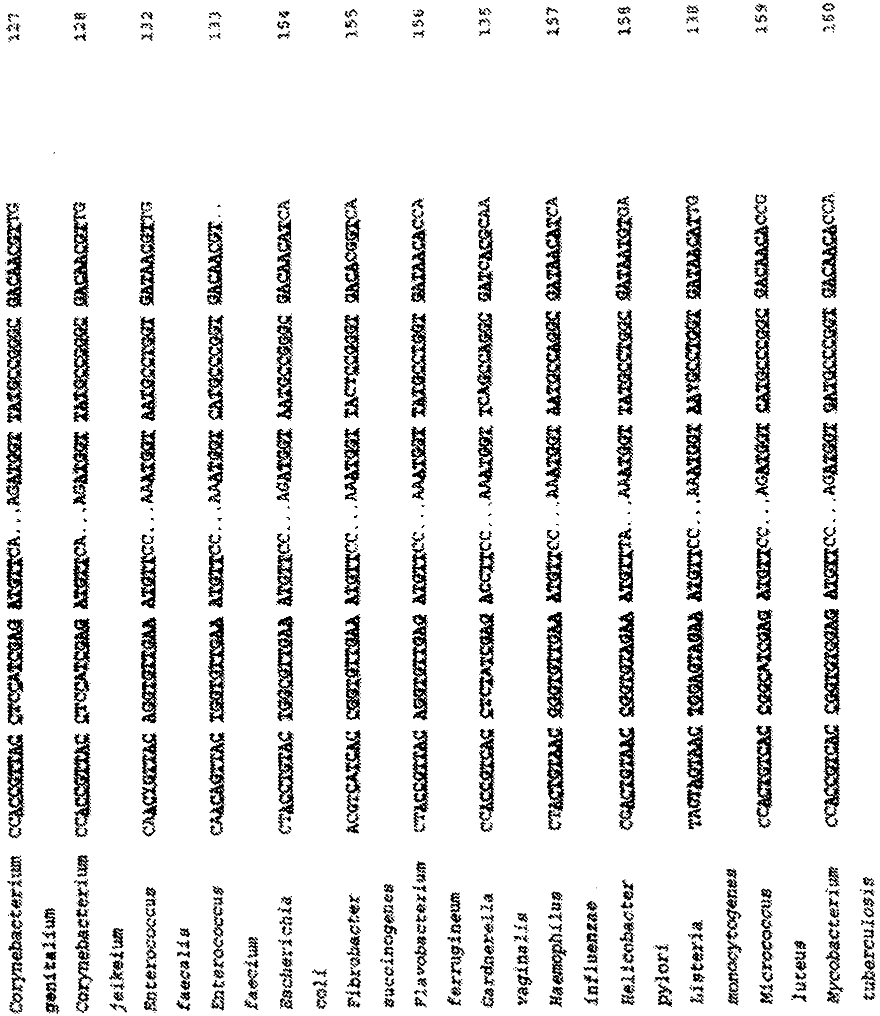

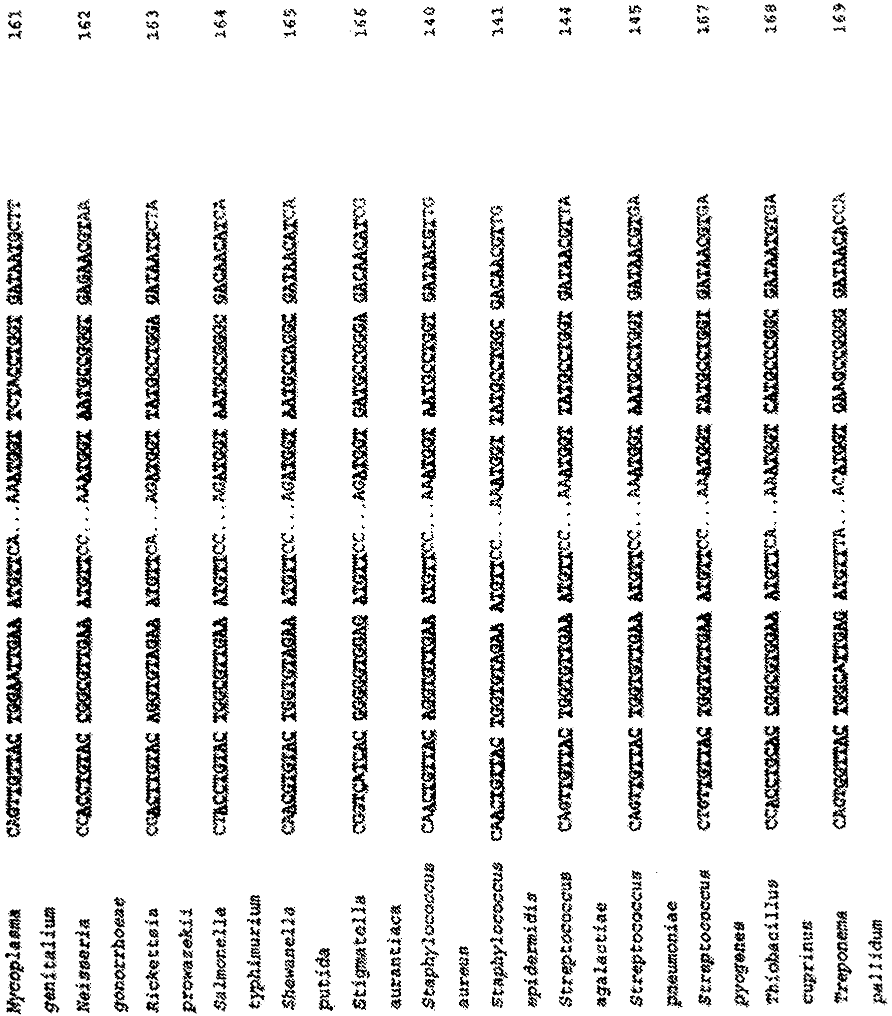

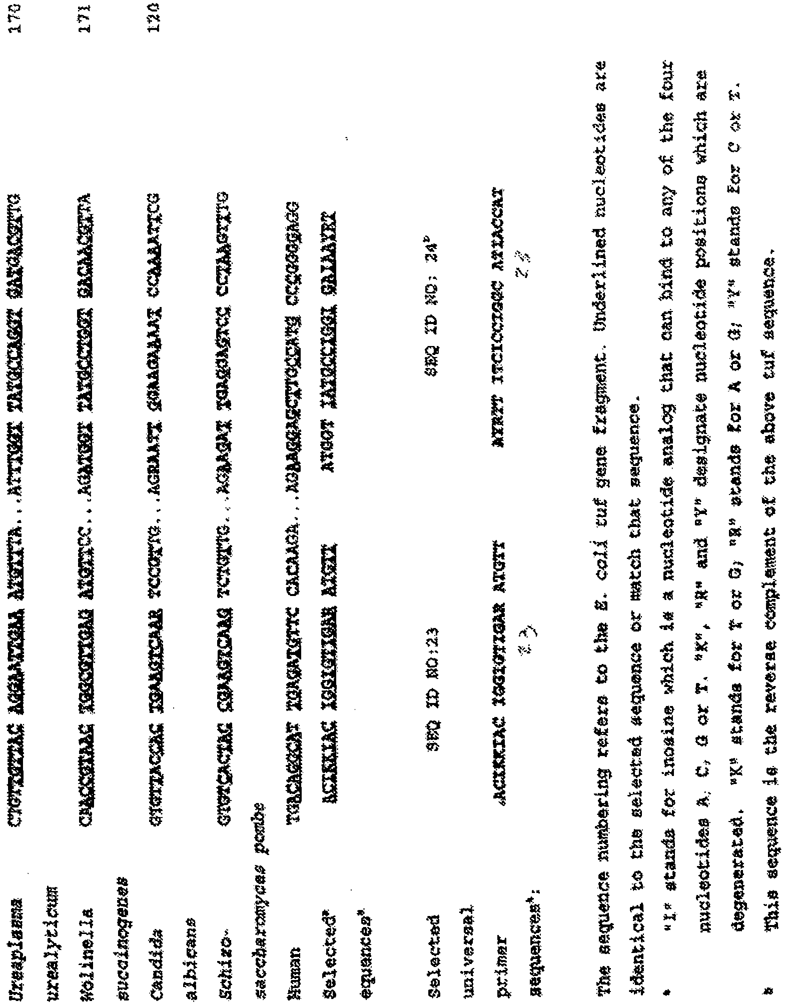

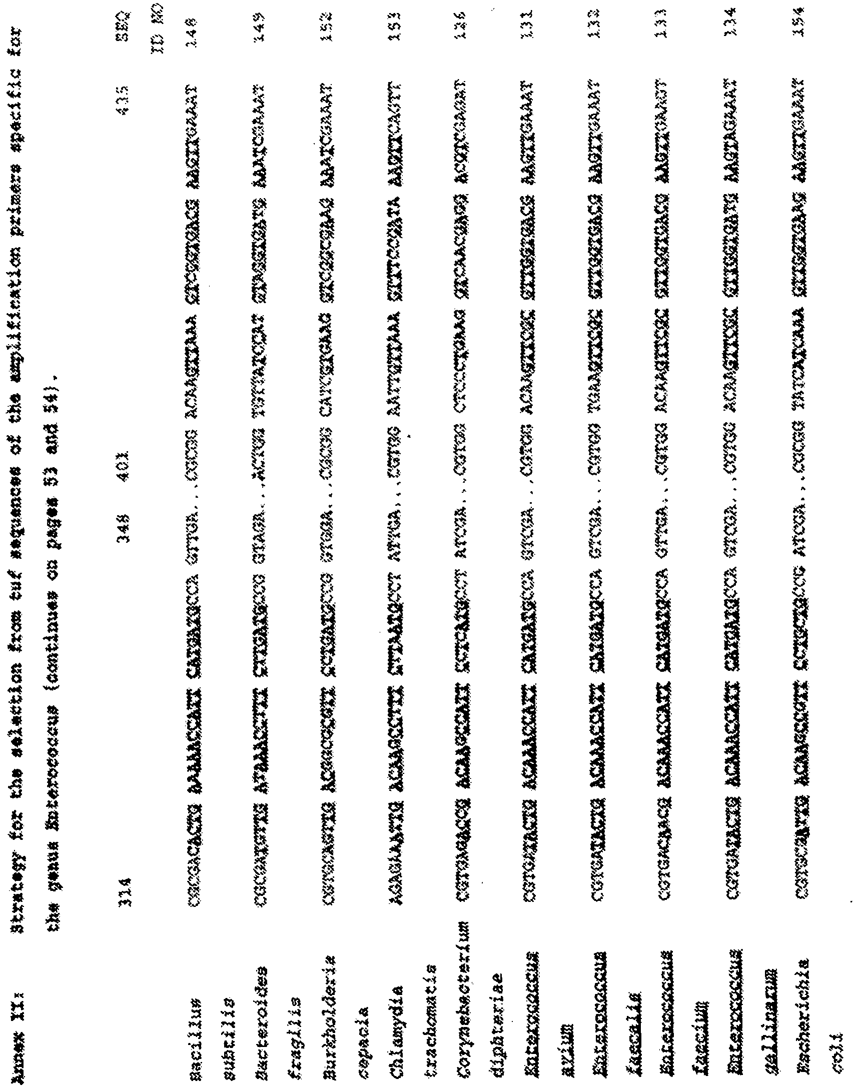

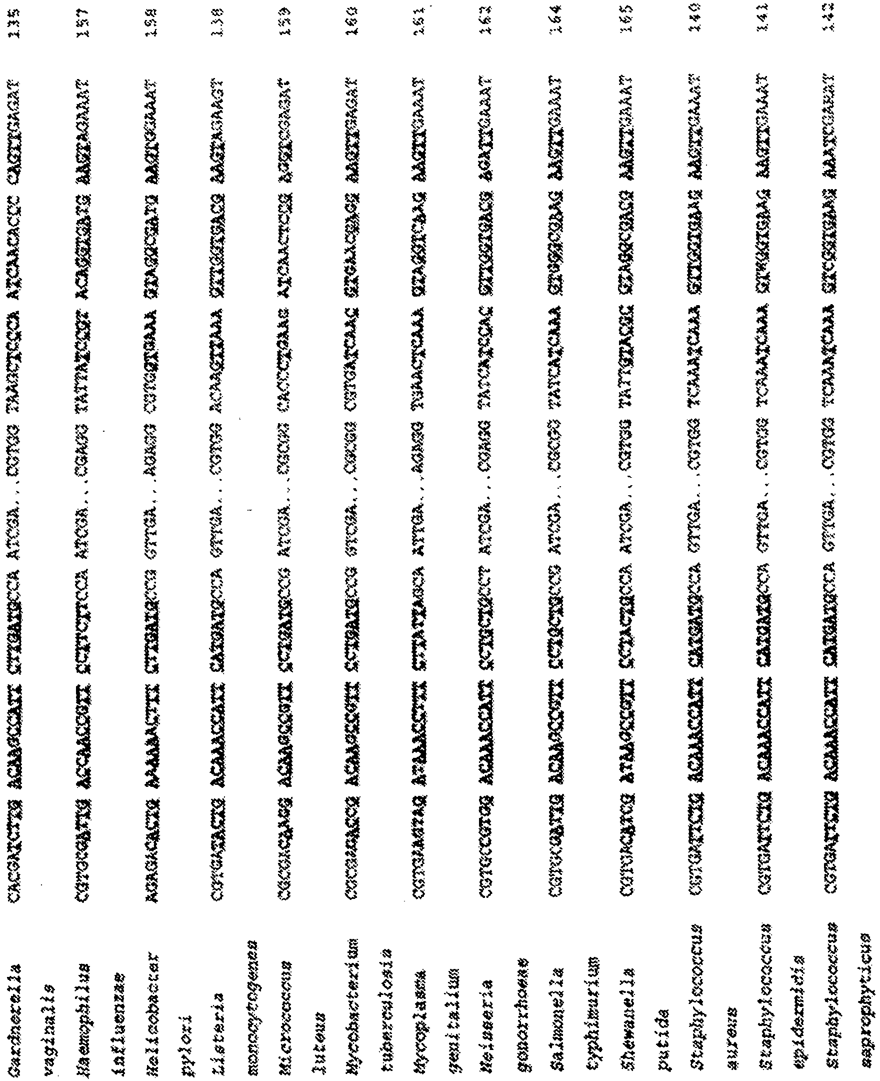

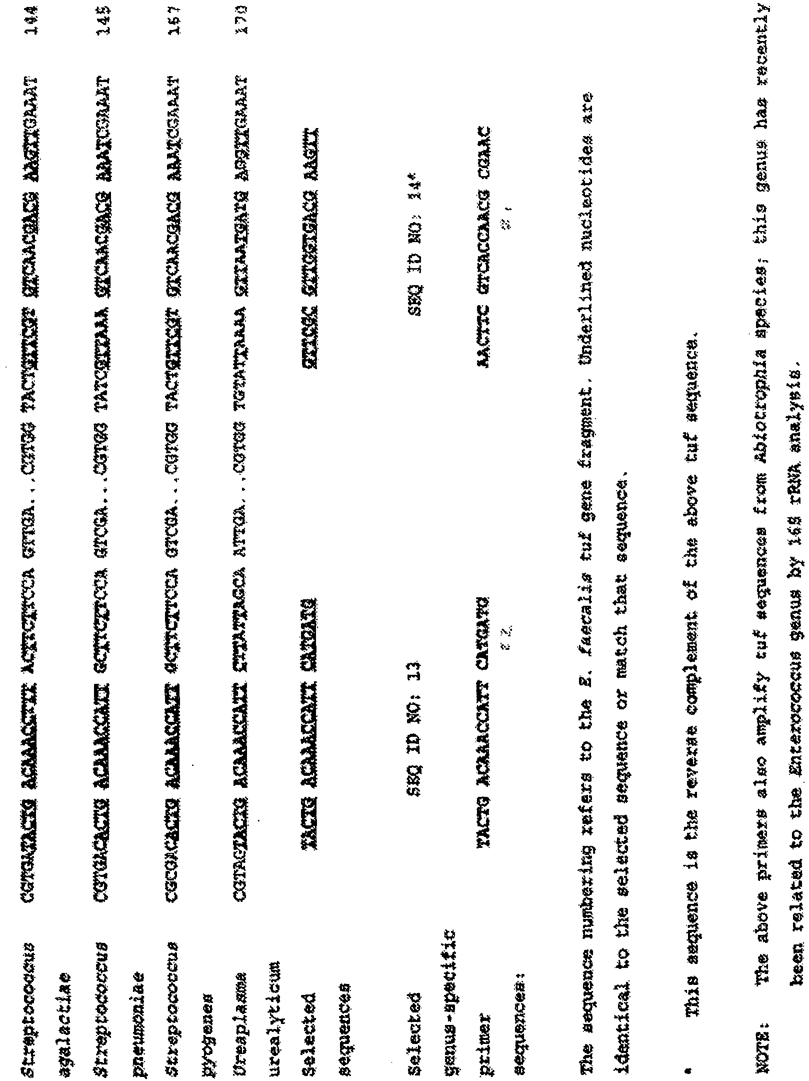

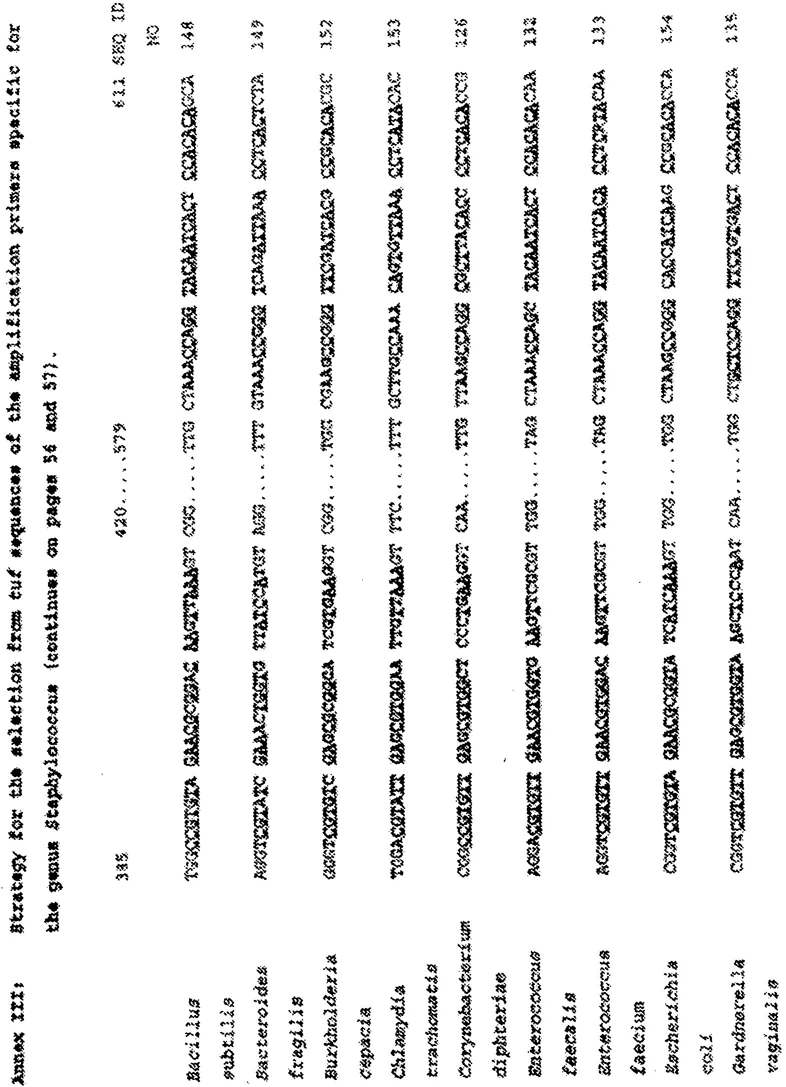

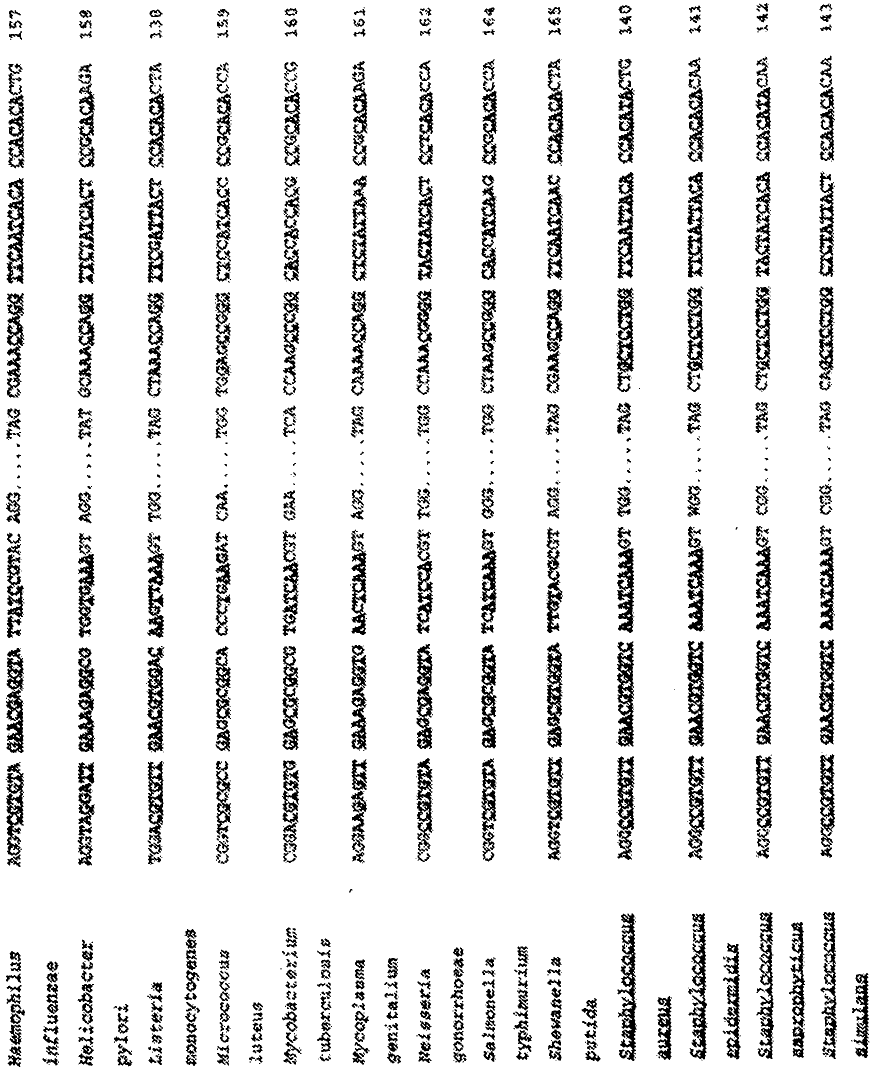

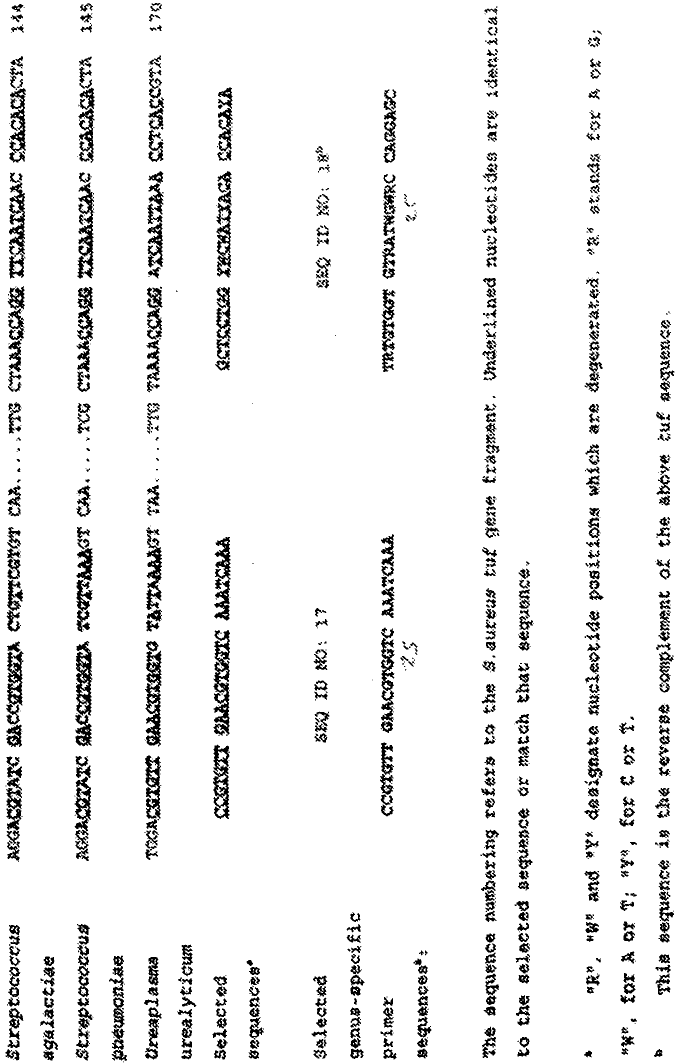

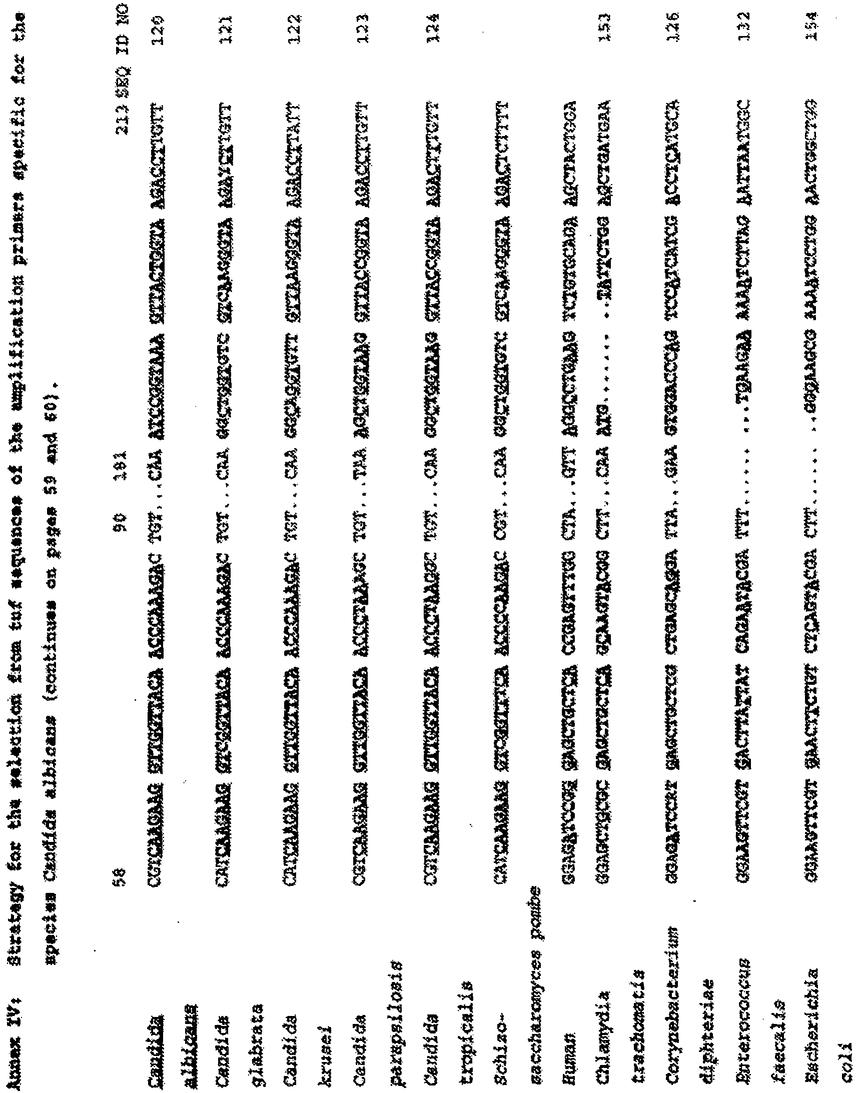

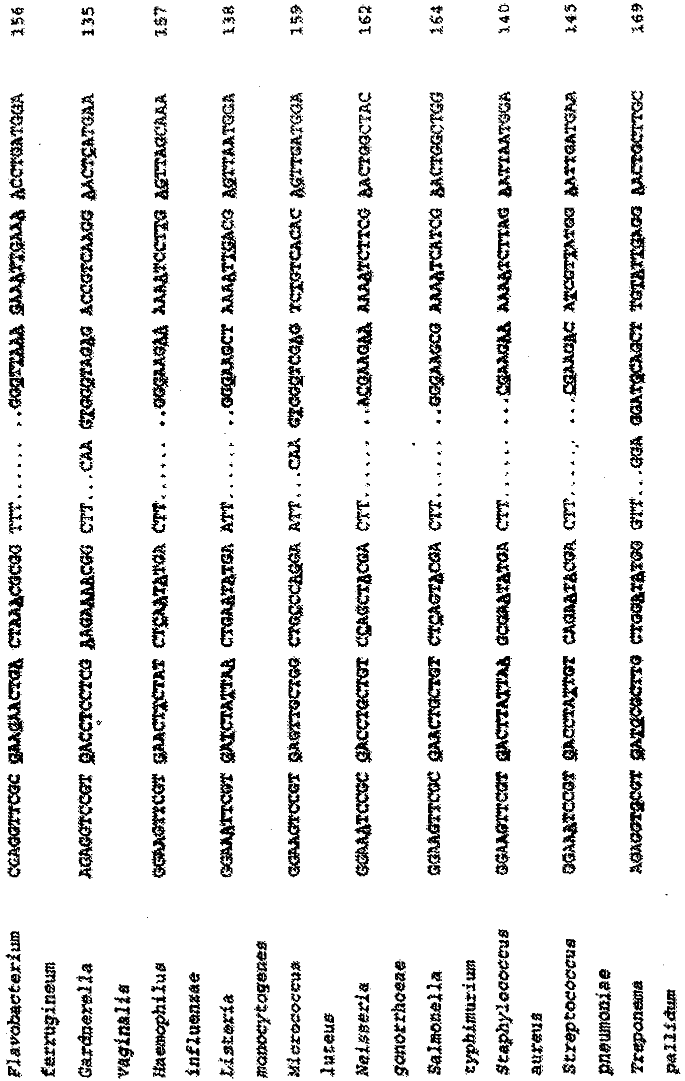

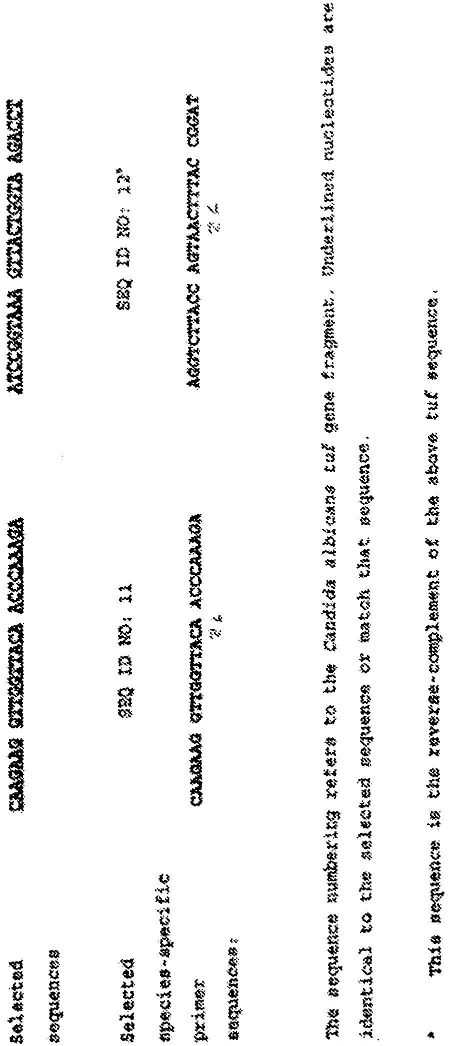

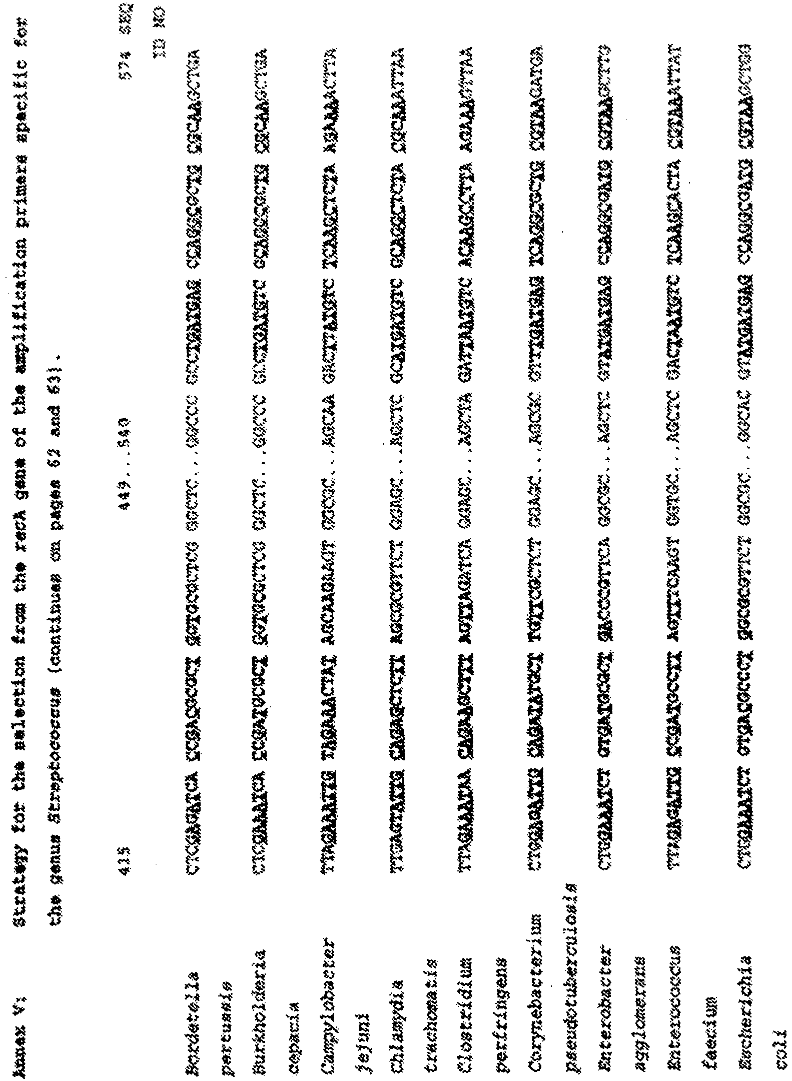

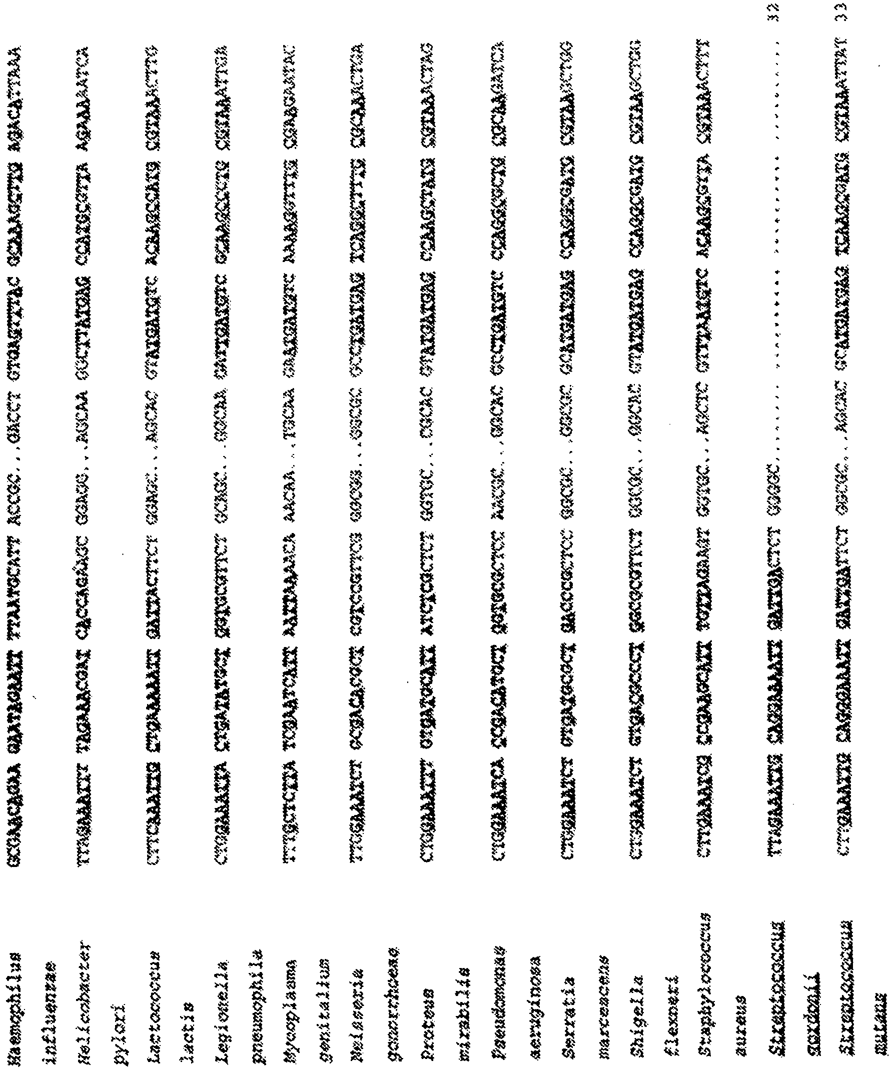

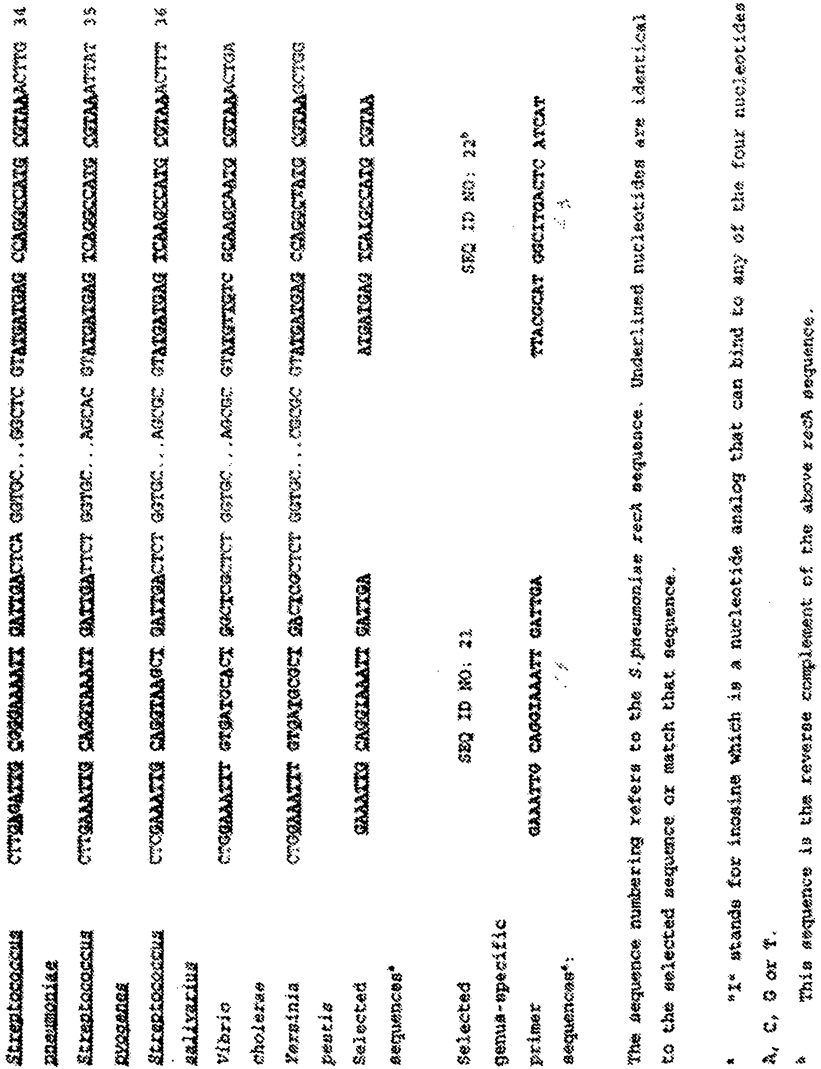

**Annex VI: Specific and ubiquitous primers for DNA amplification**

| SEQ ID NO | Nucleotide sequence | Originating DNA fragment | |
|---|---|---|---|
| | | SEQ ID NO | Nucleotide position |
| **Bacterial species: *Enterococcus faecium*** | | | |
| | | | |
| 1 | 5'-TGC TTT AGC AAC AGC CTA TCA G | 26^{a} | 273-294 |
| 2^{b} | 5'-TAA ACT TCT TCC GGC ACT TCG | 26^{a} | 468-488 |
| | | | |

| **Bacterial species: *Listeria monocytogenes*** | | | |
|---|---|---|---|
| | | | |
| 3 | 5'-TGC GGC TAT AAA TGA AGA GGC | 27^{a} | 339-359 |
| 4^{b} | 5'-ATC CGA TGA TGC TAT GGC TTT | 27^{a} | 448468 |
| | | | |

| Bacterial species: ***Neisseria meningitidis*** | | | |
|---|---|---|---|
| | | | |
| 5 | 5'-CCA GCG GTA TTG TTT GGT GGT | 28^{a} | 56-76 |
| 6^{b} | 5'-CAG GCG GCC TTT AAT AAT TTC | 28^{a} | 212-232 |
| | | | |

| **Bacterial species: *Staphylococcus saprophyticus*** | | | |
|---|---|---|---|
| | | | |
| 7 | 5'- AGA TCG AAT TCC ACA TGA AGG TTA TTA TGA | 29^{c} | 290-319 |
| 8^{b} | 5'- TCG CTT CTC CCT CAA CAA TCA AAC TAT CCT | 29^{c} | 409-438 |
| | | | |

| **Bacterial species: *Streptococcus agalactiae*** | | | |
|---|---|---|---|
| | | | |
| 9 | 5'-TTT CAC CAG CTG TAT TAG AAG TA | 30^{a} | 59-81 |
| 10^{b} | 5'-GTT CCC TGA ACA TTA TCT TTG AT | 30^{a} | 190-212 |
| | | | |

| **Fungal species: *Candida albicans*** | | | |
|---|---|---|---|
| | | | |
| 11 | 5'-CAA GAA GGT TGG TTA CAA CCC AAA GA | 120^{c} | 61-86 |
| 12^{b} | 5'-AGG TCT TAC CAG TAA CTT TAC CGG AT | 120^{c} | 184-209 |

| | | | |
|---|---|---|---|
| ^{a} Sequences from databases. ^{b} These sequences are from the opposite DNA strand of the sequence of the originating fragment given in the Sequence Listing. ^{c} Sequences determined by our group. | | | |

**Annex VI: Specific and ubiquitous primers for DNA amplification (continues on next page)**

| SEQ ID NO | Nucleotide sequence | Originating DNA fragment | |
|---|---|---|---|
| | | SEQ ID NO | Nucleotide position |
| **Bacterial genus: *Enterococcus*** | | | |
| 13 | 5'-TAC TGA CAA ACC ATT CAT GAT G | 131-134^{a,b} | 319-340^{c} |
| 14^{d} | 5'-AAC TTC GTC ACC AAC GCG AAC | 131-134^{a,b} | 410-430^{c} |
| | | | |

| **Bacterial genus: *Neisseria*** | | | |
|---|---|---|---|
| | | | |
| 15 | 5'-CTG GCG CGG TAT GGT CGG TT | 31^{e} | 21-40^{f} |
| 16^{d} | 5'-GCC GAC GTT GGA AGT GGT AAA G | 31^{e} | 102-123^{f} |

| **Bacterial genus: *Staphylococcus*** | | | |
|---|---|---|---|
| | | | |
| 17 | 5'-CCG TGT TGA ACG TGG TCA AAT CAA A | 140-143^{a,b} | 391-415^{g} |
| 18^{d} | 5'-TRT GTG GTG TRA TWG WRC CAG GAG C | 140-143^{a,b} | 584-608^{g} |
| 19 | 5'-ACA ACG TGG WCA AGT WTT AGC WGC T | 140-143^{a,b} | 562-583⁹ |
| 20^{d} | 5'-ACC ATT TCW GTA CCT TCT GGT AAG T | 140-143^{a,b} | 729-753^{g} |
| | | | |

| **Bacterial genus: *Streptococcus*** | | | |
|---|---|---|---|
| | | | |
| 21 | 5'-GAA ATT GCA GGI AAA TTG ATT GA | 32-36^{e} | 418-440^{h} |
| 22^{d} | 5'-TTA CGC ATG GCI TGA CTC ATC AT | 32-36^{e} | 547-569^{h} |
| | | | |

| | **Universal primers** | | |
|---|---|---|---|
| | | | |
| 23 | 5'-ACI KKI ACI GGI GTI GAR ARG TT | 118-146^{a,b} | 493-515ⁱ |
| | | 147-171^{a,e} | |
| 24^{d} | 5' -AYR TTI TCI CCI GGC ATI ACC AT | 118-146^{a.b} | 778-800¹ |
| | | 147-171^{a,e} | |

| | | | |
|---|---|---|---|
| ^{a} These sequences were aligned to derive the corresponding primer. ^{b} *tuf* sequences determined by our group. ^{c} The nucleotide positions refer to the *E. faecalis tuf* gene fragment (SEQ ID NO: 132). ^{d} These sequences are from the opposite DNA strand of the sequence of the originating fragment given in the Sequence Listing. ^{e} Sequences from databases. ^{f} The nucleotide positions refer to the *N. meningitidis asd* gene fragment (SEQ ID NO: 31). ^{g} The nucleotide positions refer to the *S*. *aureus tuf* gene fragment (SEQ ID NO: 140). ^{h} The nucleotide positions refer to the *S*. *pneumoniae recA* gene (SEQ ID NO: 34). ⁱ The nucleotide positions refer to the *E. coli tuf* gene fragment (SEQ ID NO: 154). | | | |

**Annex VI: Specific and ubiquitous primers for DNA amplification**

| SEQ ID NO | Nucleotide sequence | Originating DNA fragment | |
|---|---|---|---|
| | | SEQ ID NO | Nucleotide position |
| **Antibiotic resistance gene: *blaₜₑₘ*** | | | |
| 37 | 5'-CTA TGT GGC GCG GTA TTA TC | - | - |
| 38 | 5'-CGC AGT GTT ATC ACT CAT GG | - | - |

| | | | |
|---|---|---|---|
| 39 | 5'-CTG AAT GAA GCC ATA CCA AA | - | - |
| 40 | 5'-ATC AGC AAT AAA CCA GCC AG | - | - |
| | | | |

| **Antibiotic resistance gene: *blaₛₕᵥ*** | | | |
|---|---|---|---|
| | | | |
| 41 | 5'-TTA CCA TGA GCG ATA ACA GC | - | - |
| 42 | 5'-CTC ATT CAG TTC CGT TTC CC | - | - |
| | | | |
| 43 | 5'-CAG CTG CTG CAG TGG ATG GT | - | - |
| 44 | 5'-CGC TCT GCT TTG TTA TTC GG | - | - |
| | | | |

| **Antibiotic resistance gene: *bla_{rob}*** | | | |
|---|---|---|---|
| | | | |
| 45 | 5'-TAC GCC AAC ATC GTG GAA AG | - | - |
| 46 | 5'-TTG AAT TTG GCT TCT TCG GT | - | - |
| | | | |
| 47 | 5'-GGG ATA CAG AAA CGG GAC AT | - | - |
| 48 | 5'-TAA ATC TTT TTC AGG CAG CG | - | - |
| | | | |

| **Antibiotic resistance gene: *blatₒₓₐ*** | | | |
|---|---|---|---|
| | | | |
| 49 | 5'-GAT GGT TTG AAG GGT TTA TTA TAA G | 110^{a} | 686-710 |
| 50^{b} | 5'-AAT TTA GTG TGT TTA GAA TGG TGA T | 110^{a} | 802-826 |
| | | | |

| **Antibiotic resistance gene: *blaZ*** | | | |
|---|---|---|---|
| | | | |
| 51 | 5'-ACT TCA ACA CCT GCT GCT TTC | 111^{a} | 511-531 |
| 52^{b} | 5'-TGA CCA CTT TTA TCA GCA ACC | 111^{a} | 663-683 |
| | | | |

| **Antibiotic resistance gene *aadB*** | | | |
|---|---|---|---|
| | | | |
| 53 | 5'-GGC AAT AGT TGA AAT GCT CG | - | - |
| 54 | 5'-CAG CTG TTA CAA CGG ACT GG | - | - |
| | | | |

| **Antibiotic resistance gene: *aacC1*** | | | |
|---|---|---|---|
| | | | |
| 55 | 5'-TCT ATG ATC TCG CAG TCT CC | - | - |
| 56 | 5'-ATC GTC ACC GTA ATC TGC TT | - | - |

| | | | |
|---|---|---|---|
| ^{a} Sequences from databases. ^{b} These sequences are from the opposite DNA strand of the sequence of the originating fragment given in the Sequence Listing. | | | |

**Annex VI: Specific and ubiquitous primers for DNA amplification**

| SEQ ID NO | Nucleotide sequence | Originating DNA fragment | |
|---|---|---|---|
| | | SEQ ID NO | Nucleotide position |
| **Antibiotic resistance gene: *aacC2*** | | | |
| | | | |
| 57 | 5'-CAT TCT CGA TTG CTT TGC TA | - | - |
| 58 | 5'-CCG AAA TGC TTC TCA AGA TA | - | - |
| | | | |

| **Antibiotic resistance gene: *aacC3*** | | | |
|---|---|---|---|
| | | | |
| 59 | 5'-CTG GAT TAT GGC TAC GGA GT | - | - |
| 60 | 5'-AGC AGT GTG ATG GTA TCC AG | - | - |
| | | | |

| **Antibiotic resistance gene: *aac6'-IIa*** | | | |
|---|---|---|---|
| | | | |
| 61 | 5'-GAC TCT TGA TGA AGT GCT GG | 112^{a} | 123-142 |
| 62" | 5'-CTG GTC TAT TCC TCG CAC TC | 112^{a} | 284-303 |
| | | | |
| 63 | 5'-TAT GAG AAG GCA GGA TTC GT | 112^{a} | 445-464 |
| 64^{b} | 5'-GCT TTC TCT CGA AGG CTT GT | 112^{a} | 522-541 |
| | | | |

| **Antibiotic resistance gene: *aacA4*** | | | |
|---|---|---|---|
| | | | |
| 65 | 5'-GAG TTG CTG TTC AAT GAT CC | - | - |
| 66 | 5'-GTG TTT GAA CCA TGT ACA CG | - | - |
| | | | |

| **Antibiotic resistance gene: *aad(6')*** | | | |
|---|---|---|---|
| | | | |
| 173 | 5'-TCT TTA GCA GAA CAG GAT GAA | - | - |
| 174 | 5'-GAA TAA TTC ATA TCC TCC G | - | - |
| | | | |

| **Antibiotic resistance gene: *vanA*** | | | |
|---|---|---|---|
| 67 | 5'-TGT AGA GGT CTA GCC CGT GT | - | - |
| 68 | 5'-ACG GGG ATA ACG ACT GTA TG | - | - |
| | | | |
| 69 | 5'-ATA AAG ATG ATA GGC CGG TG | - | - |
| 70 | 5'-TGC TGT CAT ATT GTC TTG CC | - | - |
| | | | |

| **Antibiotic resistance gene: *vanB*** | | | |
|---|---|---|---|
| | | | |
| 71 | 5'-ATT ATC TTC GGC GGT TGC TC | 116^{a} | 22-41 |
| 72^{b} | 5'-GAC TAT CGG CTT CCC ATT CC | 116^{a} | 171-190 |
| | | | |
| 73 | 5'-CGA TAG AAG CAG CAG GAC AA | 116^{a} | 575-594 |
| 74^{b} | 5'-CTG ATG GAT GCG GAA GAT AC | 116^{a} | 713-732 |

| | | | |
|---|---|---|---|
| ^{a} Sequences from databases. ^{b} These sequences are from the opposite DNA strand of the sequence of the originating fragment given in the Sequence Listing. | | | |

**Annex VI: Specific and ubiquitous primers for DNA amplification**

| SEQ ID NO | Nucteotide sequence | Originating DNA fragment | |
|---|---|---|---|
| | | SEQ ID NO | Nucleotide position |
| **Antibiotic resistance gene: *vanC*** | | | |
| | | | |
| 75 | 5'-GCC TTA TGT ATG AAC AAA TGG | 117^{a} | 373-393 |
| -76^{b} | 5'-GTG ACT TTW GTG ATC CCT TTT GA | 117^{a} | 541-563 |
| | | | |

| **Antibiotic resistance gene: *msrA*** | | | |
|---|---|---|---|
| | | | |
| 77 | 5'-TCC AAT CAT TGC ACA AAA TC | - | - |
| 78 | 5'-AAT TCC CTC TAT TTG GTG GT | - | - |
| | | | |
| 79 | 5'-TCC CAA GCC AGT AAA GCT AA | - | - |
| 80 | 5'-TGG TTT TTC AAC TTC TTC CA | - | - |
| | | | |

| **Antibiotic resistance gene: *satA*** | | | |
|---|---|---|---|
| | | | |
| 81 | 5'-TCA TAG AAT GGA TGG CTC AA | - | - |
| 82 | 5'-AGC TAC TAT TGC ACC ATC CC | - | - |
| | | | |

| **Antibiotic resistance gene: *aac(6')-aph(2")*** | | | |
|---|---|---|---|
| | | | |
| 83 | 5'-CAA TAA GGG CAT ACC AAA AAT C | - | - |
| 84 | 5'-CCT TAA CAT TTG TGG CAT TAT C | - | - |
| | | | |
| 85 | 5'-TTG GGA AGA TGA AGT TTT TAG A | - | - |
| 86 | 5'-CCT TTA CTC CAA TAA TTT GGC T | - | - |
| | | | |

| **Antibiotic resistance gene: *vat*** | | | |
|---|---|---|---|
| | | | |
| 87 | 5'-TTT CAT CTA TTC AGG ATG GG | - | - |
| 88 | 5'-GGA GCA ACA TTC TTT GTG AC | - | - |
| | | | |

| **Antibiotic resistance gene: *vga*** | | | |
|---|---|---|---|
| | | | |
| 89 | 5'-TGT GCC TGA AGA AGG TAT TG | - | - |
| 90 | 5'-CGT GTT ACT TCA CCA CCA CT | - | - |
| | | | |

| **Antibiotic resistance gene: *ermA*** | | | |
|---|---|---|---|
| | | | |
| 91 | 5'-TAT CTT ATC GTT GAG AAG GGA TT | 113^{a} | 370-392 |
| 92^{b} | 5'-CTA CAC TTG GCT TAG GAT GAA A | 113^{a} | 487-508 |

| | | | |
|---|---|---|---|
| ^{a} Sequences from databases. ^{b} These sequences are from the opposite DNA strand of the sequence of the originating fragment given in the Sequence Listing. | | | |

**Annex VI: Specific and ubiquitous primers for DNA amplification**

| **SEQ ID NO** | **Nucleotide sequence** | **Originating DNA fragment** | |
|---|---|---|---|
| | | **SEQ ID NO** | **Nucteotide position** |
| **Antibiotic resistance gene: *ermB*** | | | |
| | | | |
| 93 | 5'-CTA TCT GAT TGT TGA AGA AGG ATT | 114^{a} | 366-389 |
| 94^{b} | 5'-GTT TAC TCT TGG TTT AGG ATG AAA | 114^{a} | 484-507 |
| | | | |

| **Antibiotic resistance gene: *ermC*** | | | |
|---|---|---|---|
| | | | |
| 95 | 5'-CTT GTT GAT CAC GAT AAT TTC C | 115^{a} | 214-235 |
| 96^{b} | 5'-ATC TTT TAG CAA ACC CGT ATT C | 115^{a} | 382-403 |
| | | | |

| **Antibiotic resistance gene: *mecA*** | | | |
|---|---|---|---|
| | | | |
| 97 | 5'-AAC AGG TGA ATT ATT AGC ACT TGT AAG | - | - |
| 98 | 5'-ATT GCT GTT AAT ATT TTT TGA GTT GAA | - | - |
| | | | |

| **Antibiotic resistance gene: *int*** | | | |
|---|---|---|---|
| | | | |
| 99 | 5'-GTG ATC GAA ATC CAG ATC C | - | - |
| 100 | 5'-ATC CTC GGT TTT CTG GAA G | - | - |
| | | | |
| 101 | 5'-CTG GTC ATA CAT GTG ATG G | - | - |
| 102 | 5'-GAT GTT ACC CGA GAG CTT G | - | - |
| | | | |

| **Antibiotic resistance gene: *sul*** | | | |
|---|---|---|---|
| | | | |
| 103 | 5'-TTA AGC GTG CAT AAT AAG CC | - | - |
| 104 | 5'-TTG CGA TTA CTT CGC CAA CT | - | - |
| | | | |
| 105 | 5'-TTT ACT AAG CTT GCC CCT TC | - | - |
| 106 | 5'-AAA AGG CAG CAA TTA TGA GC | - | - |

| | | | |
|---|---|---|---|
| ^{a} Sequences from databases. ^{b} These sequences are from the opposite DNA strand of the sequence of the originating fragment given in the Sequence Listing. | | | |

## Claims

1. A method for determining or detecting the presence and/or amount of one or more *tuf* nucleic acids from the *Listeria monocytogenes* bacterial species in a sample, said method comprising:
a) i) contacting said sample with at least one oligonucleotide having at least 12 nucleotides in length derived from and which hybridizes specifically to SEQ ID NO: 138 or a complementary sequence thereof, so as to perform an hybridization assay; or
ii) contacting said sample with at least two oligonucleotides, wherein at least one oligonucleotide has at least 12 nucleotides in length and is derived from and hybridizes specifically to SEQ ID NO: 138 or a complementary sequence thereof, so as to perform an amplification reaction; and
b) detecting the presence, amount or both of hybridized oligonucleotides or amplified products in a) as an indication of the presence, amount or both of said one or more *tuf* nucleic acids from *Listeria monocytogenes.*

2. The method of claim 1, further comprising determining the presence and/or amount of one or more nucleic acids from a bacterial and/or fungal species and/or genuses selected from the group consisting of *Enterococcus faecium, Neisseria meningitidis, Staphylococcus saprophyticus, Streptococcus agalactiae, Candida albicans,* one or more members of the *Enterococcus* genus, one or more members of the *Neisseria* genus, one or more members of the *Staphylococcus* genus, one or more members of the *Streptococcus* genus and one or more members of the *Candida* genus,
wherein said method further comprises at least one oligonucleotide having at least 12 nucleotides in length for determining the presence and/or amount of one or more nucleic acids from said bacterial and/or fungal species which is derived from and specifically hybridizes to:
SEQ ID NO: 26 or a complementary sequence thereof, for determining the presence or amount of one or more nucleic acids from *Enterococcus faecium;*
SEQ ID NO: 28 or a complementary sequence thereof, for determining the presence or amount of one or more nucleic acids from *Neisseria meningitidis;*
SEQ ID NO: 29 or a complementary sequence thereof, for determining the presence or amount of one or more nucleic acids from *Staphylococcus saprophyticus;*
SEQ ID NO: 30 or a complementary sequence thereof, for determining the presence or amount of one or more nucleic acids from *Streptococcus agalactiae;* or SEQ ID NO: 120 or a complementary sequence thereof, for determining the presence or amount of one or more nucleic acids from *Candida albicans;* or
wherein said method further comprises at least one oligonucleotide for determining the presence and/or amount of one or more nucleic acids from said bacterial and/or fungal genus which is derived from and hybridizes specifically to:
SEQ ID NOs: 131-134 or complementary sequences thereof, for determining the presence or amount of one or more nucleic acids from one or more members of the *Enterococcus* genus;
SEQ ID NO: 31 or a complementary sequence thereof, for determining the presence or amount of one or more nucleic acids from one or more members of the *Neisseria* genus;
SEQ ID NO: 140-143 or complementary sequences thereof, for determining the presence or amount of one or more nucleic acids from one or more members of the *Staphylococcus* genus;
SEQ ID NOs: 33-36 or complementary sequences thereof, for determining the presence or amount of one or more nucleic acids from one or more members of the *Streptococcus* genus; or
SEQ ID NOs: 120-124 or complementary sequences thereof, for determining the presence or amount of one or more nucleic acids from one or more members of the *Candida* genus.

3. The method of claim 2, wherein the presence and/or amount of said one or more nucleic acids from bacterial and/or fungal species and/or genuses is determined, wherein said at least one oligonucleotide comprises a nucleotide sequence selected from the group consisting of:
SEQ ID NOs: 1 and 2, parts thereof or complementary sequences thereof, for determining the presence or amount of one or more nucleic acids from *Enterococcus faecium;*
SEQ ID NOs: 5 and 6, parts thereof or complementary sequences thereof, for determining the presence or amount of one or more nucleic acids from *Neisseria meningitidis;*
SEQ ID NOs: 7 and 8, parts thereof or complementary sequences thereof, for determining the presence or amount of one or more nucleic acids from *Staphylococcus saprophyticus;*
SEQ ID NOs: 9 and 10, parts thereof or complementary sequences thereof, for determining the presence or amount of one or more nucleic acids from *Streptococcus agalactiae;*
SEQ ID NOs: 11 and 12, parts thereof or complementary sequences thereof, for determining the presence or amount of one or more nucleic acids from *Candida albicans;*
SEQ ID NOs: 13 and 14, parts thereof or complementary sequences thereof, for determining the presence or amount of one or more nucleic acids from one or more members of the *Enterococcus* genus;
SEQ ID NOs: 15 and 16, parts thereof or complementary sequences thereof, for determining the presence or amount of one or more nucleic acids from one or more members of the *Neisseria* genus;
SEQ ID NOs: 17-20, parts thereof or complementary sequences thereof, for determining the presence or amount of one or more nucleic acids from one or more members of the *Staphylococcus* genus; and
SEQ ID NOs: 21 and 22, parts thereof or complementary sequences thereof, for determining the presence or amount of one or more nucleic acids from one or more members of the *Streptococcus* genus.

4. The method of any one of claims 1-3, further comprising determining the presence and/or amount of one or more nucleic acids from one or more bacterial antibiotic resistance genes selected from the group consisting of *bla_{tem,} blaₛₕᵥ, bla_{rob}, blaₒₓₐ, blaZ, aadB, aacc1, aacC2, aacC3, aac6'-lla, aacA4, aad(6'), vanA, vanB, vanC, msrA, satA, aac(6')-aph(2"), vat, vga, ermA, ermB, ermC, mecA, int* and *sul.*

5. The method of claim 4, wherein said one or more bacterial antibiotic resistance genes are selected from the group consisting of *blaₒₓₐ, blaZ, aac6'-Ila, vanB, vanC, ermA, ermB and ermC,* and wherein said method comprises at least one oligonucleotide having at least 12 nucleotides in length for determining the presence of one or more nucleic acids from one or more bacterial antibiotic resistance genes that is derived from and hybridizes specifically to one or more of the nucleotide sequences selected from the group consisting of:
SEQ ID NO: 110 or a complementary sequence thereof for the detection of *blaₒₓₐ*;
SEQ ID NO: 111 or a complementary sequence thereof for the detection of *blaZ*;
SEQ ID NO: 112 or a complementary sequence thereof for the detection of *aac6'-Ila;*
SEQ ID NO: 113 or a complementary sequence thereof for the detection of *ermA;*
SEQ ID NO: 114 or a complementary sequence thereof for the detection of *ermB*;
SEQ ID NO: 115 or a complementary sequence thereof for the detection of *ermC*;
SEQ ID NO: 116 or a complementary sequence thereof for the detection of *vanB*; and
SEQ ID NO: 117 or a complementary sequence thereof for the detection of *vanC.*

6. The method of claim 4, comprising at least one oligonucleotide for determining the presence and/or amount of one or more nucleic acids from one or more bacterial antibiotic resistance genes, wherein said at least one oligonucleotide comprises a nucleotide sequence selected from the group consisting of:
SEQ ID NOs: 37-40, parts thereof having at least 12 nucleotides in length length or complementary sequences thereof for the detection of *bla*_{*tem*;}
SEQ ID NOs: 41-44, parts thereof having at least 12 nucleotides in length or complementary sequences thereof for the detection of *blaₛₕᵥ*;
SEQ ID NOs: 45-48, parts thereof having at least 12 nucleotides in length or complementary sequences thereof for the detection of *bla_{rob}*;
SEQ ID NOs: 49-50, parts thereof having at least 12 nucleotides in length or complementary sequence thereof for the detection of *blaₒₓₐ*;
SEQ ID NOs: 51-52, parts thereof having at least 12 nucleotides in length or complementary sequences thereof for the detection of *b*/*aZ*;
SEQ ID NOs: 53-54, parts thereof having at least 12 nucleotides in length or complementary sequences thereof for the detection of *aad*B;
SEQ ID NOs: 55-56, parts thereof having at least 12 nucleotides in length or complementary sequences thereof for the detection of *aac*C1;
SEQ ID NOs: 57-58, parts thereof having at least 12 nucleotides in length or complementary sequences thereof for the detection of *aac*C2;
SEQ ID NOs: 59-60, parts thereof having at least 12 nucleotides in length or complementary sequences thereof for the detection of *aac*C3;
SEQ ID NOs: 61-64, parts thereof having at least 12 nucleotides in length or complementary sequences thereof for the detection of *aac6'-Ila.;*
SEQ ID NOs: 65-66, parts thereof having at least 12 nucleotides in length or complementary sequences thereof for the detection of *aac*C4;
SEQ ID NOs: 67-70, parts thereof or complementary sequences thereof for the detection of *vanA*;
SEQ ID NOs: 71-74, parts thereof having at least 12 nucleotides in length or complementary sequences thereof for the detection of *vanB*;
SEQ ID NOs: 75-76, parts thereof having at least 12 nucleotides in length or complementary sequences thereof for the detection of *vanC*.
SEQ ID NOs: 77-80, parts thereof having at least 12 nucleotides in length or complementary sequences thereof for the detection of *msrA*;
SEQ ID NOs: 81-82, parts thereof having at least 12 nucleotides in length or complementary sequences thereof for the detection of *satA*;
SEQ ID NOs: 83-86, parts thereof having at least 12 nucleotides in length or complementary sequences thereof for the detection of *aac*(6')-*aph*(2");
SEQ ID NOs: 87-88, parts thereof having at least 12 nucleotides in length or complementary sequences thereof for the detection of *vat*;
SEQ ID NOs: 89-90, parts thereof having at least 12 nucleotides in length or complementary sequences thereof for the detection of *vga*;
SEQ ID NOs: 91-92, parts thereof having at least 12 nucleotides in length or complementary sequences thereof for the detection of *ermA*;
SEQ ID NOs: 93-94, parts thereof having at least 12 nucleotides in length or complementary sequences thereof for the detection of *ermB*;
SEQ ID NOs: 95-96, parts thereof having at least 12 nucleotides in length or complementary sequences thereof for the detection of *ermC*;
SEQ ID NOs: 97-98, parts thereof having at least 12 nucleotides in length or complementary sequences thereof for the detection of *mec*A;
SEQ ID NOs: 99-102, parts thereof having at least 12 nucleotides in length or complementary sequences thereof for the detection of *int*; and
SEQ ID NOs: 103-106, parts thereof having at least 12 nucleotides in length or complementary sequences thereof for the detection of *sul*.

7. The method of any one of claims 1-6, wherein said determining is performed simultaneously.

8. The method of any one of claims 1-7, which is performed directly from a test sample.

9. The method of any one of claims 1-8, which is performed directly from a test sample consisting of a bacterial and/or fungal culture or suspension.

10. The method of any one of claims 1-9, wherein said nucleic acids are amplified by a method selected from the group consisting of:
a) polymerase chain reaction (PCR),
b) ligase chain reaction (LCR),
c) nucleic acid sequence-based amplification (NASBA),
d) self-sustained sequence replication (3SR),
e) strand displacement amplification (SDA),
f) branched DNA signal amplification (bDNA),
g) transcription-mediated amplification (TMA),
h) cycling probe technology (CPT),
i) nested PCR, and
j) multiplex PCR.

11. The method of claim 10, wherein said nucleic acids are amplified by PCR.

12. The method of claim 11, wherein the amplification conditions are uniform.

13. The method of any one of claims 1-9, further comprising:
a) i) depositing and fixing on an inert support or leaving in solution said one or more nucleic acids from bacteria of said sample, or
ii) inoculating said sample on an inert support, and lysing *in situ* said inoculated sample to release said one or more nucleic acids in said sample, said one or more nucleic acids being in a substantially single-stranded form;
b) contacting said substantially single-stranded one or more nucleic acids with an oligonucleotide probe having at least 12 nucleotides in length derived from and which hybridizes specifically to a *tuf* nucleotide sequence defined in SEQ ID NO: 138 or a complementary sequence thereof;
c) detecting the presence of said hybridization complex on said inert support or in said solution as an indication of the presence and/or amount of said one or more *tuf* nucleic acids from bacteria in said sample.

14. The method of any one of claims 1-12, further comprising the use of a primer pair for the detection of bacterial or fungal nucleic acids, wherein said primer pair is selected from the group consisting of SEQ ID NOs: 23 and 24, 107 and 108, and 109 and 172.

15. An isolated oligonucleotide having at least 12 nucleotides in length derived from and which hybridizes specifically to: SEQ ID NO:138, or a sequence complementary thereof, for determining the presence and/or amount of one or more *tuf* nucleic acids from the *Listeria monocytogenes* species.

16. An isolated *tuf* nucleic acid from *Listeria monocytogenes* having the nucleotide sequence defined in SEQ ID NO: 138, a part thereof or a complementary sequence thereof.

17. A recombinant plasmid comprising the isolated oligonucleotide of claim 15 or the nucleic acid of claim 16.

18. A recombinant host which has been transformed by the recombinant plasmid of claim 17.

19. The recombinant host of claim 18, wherein said host is *Escherichia coli.*

20. A diagnostic kit for determining or detecting the presence and/or amount of one or more nucleic acids from the *Listeria monocytogenes* bacterial species in a sample, comprising any suitable combination of species-specific probes and/or primers having at least 12 nucleotides in length derived from and which hybridize specifically to SEQ ID NO:138 or a complementary sequence thereof for determining or detecting the presence and/or amount of one or more *tuf* nucleic acids from the *Listeria monocytogenes* species.

21. The diagnostic kit of claim 20, further comprising any suitable combination of probes and/or primers having at least 12 nucleotides in length derived from and which hybridize specifically to one or more of the nucleotide sequences selected from the group consisting of SEQ ID NOs: 110-117 and complementary sequences thereof, for the detection and/or quantification of the nucleic acids of any combination of the bacterial resistance genes selected from the group consisting of *blaₒₓₐ*, *blaZ, aac6'-Ila, ermA, ermB, ermC,* and *vanC.*

22. The diagnostic kit of claim 20, further comprising any suitable combination of primers comprising a nucleotide sequence selected from the group consisting of SEQ ID NOs: 37-106, 173 and 174, parts thereof having at least 12 nucleotides in length and complementary sequences thereof, for the simultaneous detection and/or quantification of nucleic acids of any bacterial antibiotic resistance genes selected from the group consisting of *blaₜₑₘ, bla_{rob}, blaₛₕᵥ, blaₒₓₐ, blaZ, aadB, aacC1, aacC2, aacC3, aacA4, aac6'-lla, and(6'), ermA, ermB, ermC, mecA, vanA, vanB, vanC, satA, arc(6')-aph(2"), vat, vga, msrA, sul* and *int.*

23. The diagnostic kit of any one of claims 20-22, further comprising any suitable combination of primers comprising a nucleotide sequence selected from the group consisting of SEQ ID NOs: 1, 2 and 5-23, parts thereof having at least 12 nucleotides in length and complementary sequences thereof, for the detection and/or quantification of nucleic acids of any bacterial and/or fungal species and/or genus selected from the group consisting of *Enterococcus faecium, Staphylococcus saprophyticus, Neisseria meningitidis, Streptococcus agalactiae, Candida albicans, Enterococcus* genus, *Staphylococcus* genus, *Neisseria* genus and *Streptococcus* genus.

24. The diagnostic kit of any one of claims 20-23, further comprising any suitable combination of primers having at least 12 nucleotides in length, said combination of primers comprising a pair of primers consisting of a nucleotide sequence selected from the group consisting of SEQ ID NOs: 23, 24, 107, 108, 109 and 172 and complementary sequences thereof, for the simultaneous universal detection and/or quantification of nucleic acids of bacterium or fungus.

25. The diagnostic kit of claim 24, wherein said pair of primers consists of nucleic acid sequences defined in SEQ ID NOs: 23 and 24; 107 and 108 or 109 and 172.

26. The isolated oligonucleotide of claim 15, wherein said oligonucleotide consists of 12-30 nucleotides in length.

27. The diagnostic kit of any one of claims 20-25, wherein said probes and/or primers are from 12-30 nucleotides in length.

28. The method of any one of claims 1-12 and 14, wherein multiplex amplification is used.

29. The method of any one of claims 1-12, 14and 28, wherein said oligonucleotide is capable of specifically amplifying its target nucleic acid using PCR conditions comprising for each amplification cycle a denaturation step of 1 s at 95°C and an annealing-extension step of 30 s at 45-55°C.

30. The method of any one of claims 1-12, 14and 28, wherein said oligonucleotide is capable of specifically amplifying its target nucleic acid using PCR conditions comprising for each amplification cycle a denaturation step of 1 s at 95°C and an annealing-extension step of 30 s at 55°C.

31. The oligonucleotide of claim 15 or 26 or the diagnostic kit of any one of claims 20-25 and 27, wherein said oligonucleotide or said probes and/or primers are capable of specifically amplifying their target nucleic acid(s) using PCR conditions comprising for each amplification cycle a denaturation step of 1 s at 95°C and an annealing-extension step of 30 s at 45-55°C.

32. The oligonucleotide of claim 15 or 26 or the diagnostic kit of any one of claims 20-25 and 27, wherein said oligonucleotide or said probes and/or primers are capable of specifically amplifying their target nucleic acid(s) using PCR conditions comprising for each amplification cycle a denaturation step of 1 s at 95°C and an annealing-extension step of 30 s at 55°C.

33. The method of any one of claims 1-14 and 28, wherein said oligonucleotide is capable of hybridizing specifically to its target nucleic acid under the following conditions:
- hybridization in pre-hybridization solution at 65°C overnight followed by post-hybridization washings:
twice in 3X SSC containing 1% SDS at 65°C for 15 min;
twice in 2X SSC containing 1% SDS at 65°C for 15 min;
twice in 1X SSC containing 1% SDS at 65°C for 15 min; and
a final wash in 0.1X SSC containing 1% SDS at 25°C for 15 min.

34. The oligonucleotide of claim 15 or 26 or the diagnostic kit of any one of claims 20-25 and 27, wherein said oligonucleotide or said probes and/or primers are capable of hybridizing specifically to their target nucleic acid(s) under the following conditions:
- hybridization in pre-hybridization solution at 65°C overnight followed by post-hybridization washings:
twice in 3X SSC containing 1% SDS at 65°C for 15 min;
twice in 2X SSC containing 1% SDS at 65°C for 15 min;
twice in 1X SSC containing 1% SDS at 65°C for 15 min; and
a final wash in 0.1X SSC containing 1% SDS at 25°C for 15 min.

35. The method of any one of claims 1-14, 28-30 and 33, wherein said oligonucleotide consists of 12-30 nucleotides in length.

## Patentansprüche

1. Verfahren zum Bestimmen oder Nachweisen der Gegenwart und/oder Menge einer oder mehrerer *tuf* Nukleinsäuren aus der Bakterienart *Listeria monocytogenes* in einer Probe, wobei das Verfahren Folgendes umfasst:
a) i) in Kontakt bringen der Probe mit mindestens einem Oligonukleotid, das eine Länge von mindestens 12 Nukleotiden aufweist, das abgeleitet ist von und spezifisch hybridisiert mit SEQ ID NO:138 oder einer dazu komplementären Sequenz, um einen Hybridisierungsassay durchzuführen; oder
ii) in Kontakt bringen der Probe mit mindestens zwei Oligonukleotiden, wobei mindestens ein Oligonukleotid eine Länge von mindestens 12 Nukleotiden aufweist und abgeleitet ist von und spezifisch hybridisiert mit SEQ ID NO:138 oder einer dazu komplementären Sequenz, um eine Amplifikationsreaktion durchzuführen; und
b) Nachweisen der Gegenwart, Menge oder beides hybridisierter Oligonukleotide oder amplifizierter Produkte in a) als Anhaltspunkt für die Gegenwart, Menge oder beides von einem oder mehreren *tuf* Nukleinsäuren aus *Listeria monocytogenes.*

2. Verfahren nach Anspruch 1, das ferner das Bestimmen der Gegenwart und/oder Menge einer oder mehrerer Nukleinsäuren umfasst aus einer Bakterien- und/oder Pilzart und/oder Gattungen ausgewählt aus der Gruppe bestehend aus *Enterococcus faecium, Neisseria meningitidis, Staphylococcus saprophyticus, Streptococcus agalactiae, Candida albicans,* einem oder mehreren Mitgliedern der Gattung *Enterococcus,* einem oder mehreren Mitgliedern der Gattung *Neisseria,* einem oder mehreren Mitgliedern der Gattung, einem oder mehreren Mitgliedern der Gattung *Staphylococcus,* einem oder mehreren Mitgliedern der Gattung Streptococcus und einem oder mehreren Mitgliedern der Gattung *Candida,*
wobei das Verfahren ferner mindestens ein Oligonukleotid umfasst, das eine Länge von mindestens 12 Nukleotiden aufweist, zum Bestimmen der Gegenwart und/oder Menge einer oder mehrerer Nukleinsäuren der Bakterien- und/oder Pilzarten, das abgeleitet ist und spezifisch hybridisiert mit:
SEQ ID NO:26 oder einer dazu komplementären Sequenz zum Bestimmen der Gegenwart oder Menge einer oder mehrerer Nukleinsäuren aus *Enterococcus faecium;*
SEQ ID NO:28 oder einer dazu komplementären Sequenz zum Bestimmen der Gegenwart oder Menge einer oder mehrerer Nukleinsäuren aus *Neisseria meningitidis;*
SEQ ID NO:29 oder einer dazu komplementären Sequenz zum Bestimmen der Gegenwart oder Menge eines oder mehrerer Nukleinsäuren aus *Staphylococcus saprophyticus;*
SEQ ID NO:30 oder einer dazu komplementären Sequenz zum Bestimmen der Gegenwart oder Menge einer oder mehrerer Nukleinsäuren aus *Streptococcus agalactiae;* oder
SEQ ID NO:120 oder einer dazu komplementären Sequenz zum Bestimmen der Gegenwart oder Menge einer oder mehrerer Nukleinsäuren aus *Candida albicans;* oder
wobei das Verfahren ferner mindestens ein Oligonukleotid umfasst zum Bestimmen der Gegenwart und/oder Menge einer oder mehrerer Nukleinsäuren der Bakterien- und/oder Pilzgattung, das abgeleitet ist von und spezifisch hybridisiert mit:
SEQ ID NO:131-134 oder dazu komplementären Sequenzen zum Bestimmen der Gegenwart oder Menge einer oder mehrerer Nukleinsäuren aus einem oder mehreren Mitgliedern der Gattung *Enterococcus;*
SEQ ID NO:31 oder einer dazu komplementären Sequenz zum Bestimmen der Gegenwart oder Menge einer oder mehrerer Nukleinsäuren aus einem oder mehreren Mitgliedern der Gattung *Neisseria;*
SEQ ID NO:140-143 oder dazu komplementären Sequenzen zum Bestimmen der Gegenwart oder Menge einer oder mehrerer Nukleinsäuren aus einem oder mehreren Mitgliedern der Gattung *Staphylococcus;*
SEQ ID NO:33-36 oder dazu komplementären Sequenzen zum Bestimmen der Gegenwart oder Menge einer oder mehrerer Nukleinsäuren aus einem oder mehreren Mitgliedern der Gattung *Streptococcus;*
SEQ ID NO:120-124 oder dazu komplementären Sequenzen zum Bestimmen der Gegenwart oder Menge einer oder mehrerer Nukleinsäuren aus einem oder mehreren Mitgliedern der Gattung *Candida.*

3. Verfahren nach Anspruch 2, wobei die Gegenwart und/oder Menge der einen oder mehreren Nukleinsäuren aus Bakterien- und/oder Pilzarten und/oder -gattungen bestimmt wird, wobei das mindestens eine Oligonukleotid eine Nukleotidsequenz umfasst, die ausgewählt ist aus der Gruppe bestehend aus:
SEQ ID NOs: 1 und 2, Teilen davon oder dazu komplementären Sequenzen zum Bestimmen der Gegenwart oder Menge einer oder mehrerer Nukleinsäuren aus *Enterococcus faecium;*
SEQ ID NOs: 5 und 6, Teilen davon oder dazu komplementären Sequenzen zum Bestimmen der Gegenwart oder Menge einer oder mehrerer Nukleinsäuren aus *Neisseria meningitidis;*
SEQ ID NOs: 7 und 8, Teilen davon oder dazu komplementären Sequenzen zum Bestimmen der Gegenwart oder Menge einer oder mehrerer Nukleinsäuren aus *Staphylococcus saprophyticus;*
SEQ ID NOs: 9 und 10, Teilen davon oder dazu komplementären Sequenzen zum Bestimmen der Gegenwart oder Menge einer oder mehrerer Nukleinsäuren aus *Streptococcus agalactiae;*
SEQ ID NOs: 11 und 12, Teilen davon oder dazu komplementären Sequenzen zum Bestimmen der Gegenwart oder Menge einer oder mehrerer Nukleinsäuren aus *Candida albicans;*
SEQ ID NOs: 13 und 14, Teilen davon oder dazu komplementären Sequenzen zum Bestimmen der Gegenwart oder Menge einer oder mehrerer Nukleinsäuren aus einem oder mehreren Mitgliedern der Gattung *Enterococcus;*
SEQ ID NOs: 15 und 16, Teilen davon oder dazu komplementären Sequenzen zum Bestimmen der Gegenwart oder Menge einer oder mehrerer Nukleinsäuren aus einem oder mehreren Mitgliedern der Gattung *Neisseria;*
SEQ ID NOs: 17-20, Teilen davon oder dazu komplementären Sequenzen zum Bestimmen der Gegenwart oder Menge einer oder mehrerer Nukleinsäuren aus einem oder mehreren Mitgliedern der Gattung *Staphylococcus;*
SEQ ID NOs: 21 und 22, Teilen davon oder dazu komplementären Sequenzen zum Bestimmen der Gegenwart oder Menge einer oder mehrerer Nukleinsäuren aus einem oder mehreren Mitgliedern der Gattung *Streptococcus.*

4. Verfahren nach einem der Ansprüche 1-3, das ferner das Bestimmen der Gegenwart und/oder Menge einer oder mehrerer Nukleinsäuren eines oder mehrerer bakterieller Antibiotikaresistenzgene umfasst, ausgewählt aus der Gruppe bestehend aus *blaₜₑₘ, blaₛₕᵥ, bla_{rob}, blaₒₓₐ, blaZ, aadB*, *aacC1, aacC2, aacC3, aac6'-Ila, aacA4, aad(6), vanA, vanB, vanC, msrA, satA, aac(6')-aph(2'), vat, vga, ermA, ermB, ermC, mecA, int* und *sul.*

5. Verfahren nach Anspruch 4, wobei die einen oder mehreren bakteriellen Antibiotikaresistenzgene ausgewählt werden aus der Gruppe bestehend aus *blaₒₓₐ, blaZ, aac6'-Ila, vanB, vanC, ermA, ermB* und *ermC* und wobei das Verfahren mindestens ein Oligonukleotid umfasst, das eine Länge von mindestens 12 Nukleotiden aufweist, zum Bestimmen der Gegenwart einer oder mehrerer Nukleinsäuren eines oder mehrerer bakterieller Antibiotikaresistenzgene, das abgeleitet ist von und spezifisch an eine oder mehrere Nukleotidsequenzen hybridisiert ausgewählt aus der Gruppe bestehend aus:
SEQ ID NO:110 oder einer dazu komplementären Sequenz zum Nachweisen von *blaₒₓₐ*;
SEQ ID NO:111 oder einer dazu komplementären Sequenz zum Nachweisen von *blaZ*;
SEQ ID NO:112 oder einer dazu komplementären Sequenz zum Nachweisen von *aac6'-Ila;*
SEQ ID NO:113 oder einer dazu komplementären Sequenz zum Nachweisen von *ermA*;
SEQ ID NO:114 oder einer dazu komplementären Sequenz zum Nachweisen von *ermB*;
SEQ ID NO:115 oder einer dazu komplementären Sequenz zum Nachweisen von *ermC*;
SEQ ID NO:116 oder einer dazu komplementären Sequenz zum Nachweisen von *vanB*;
SEQ ID NO:117 oder einer dazu komplementären Sequenz zum Nachweisen von *vanC.*

6. Verfahren nach Anspruch 4, das mindestens ein Oligonukleotid umfasst zum Bestimmen der Gegenwart und/oder Menge einer oder mehrerer Nukleinsäuren eines oder mehrerer bakterieller Antibiotikaresistenzgene, wobei das mindestens eine Oligonukleotid eine Nukleotidsequenz umfasst, die ausgewählt ist aus der Gruppe bestehend aus:
SEQ ID NO:37-40, Teilen davon, die eine Länge von mindestens 12 Nukleotiden aufweisen oder dazu komplementären Sequenzen zum Nachweisen von *bla*_{*tem*;}
SEQ ID NO:41-44, Teilen davon, die eine Länge von mindestens 12 Nukleotiden aufweisen oder dazu komplementären Sequenzen zum Nachweisen von *blaₛₕᵥ*;
SEQ ID NO:45-48, Teilen davon, die eine Länge von mindestens 12 Nukleotiden aufweisen oder dazu komplementären Sequenzen zum Nachweisen von *bla_{rob}*;
SEQ ID NO:49-50, Teilen davon, die eine Länge von mindestens 12 Nukleotiden aufweisen oder dazu komplementären Sequenzen zum Nachweisen von *blaₒₓₐ*;
SEQ ID NO:51-52, Teilen davon, die eine Länge von mindestens 12 Nukleotiden aufweisen oder dazu komplementären Sequenzen zum Nachweisen von *blaZ*;
SEQ ID NO:53-54, Teilen davon, die eine Länge von mindestens 12 Nukleotiden aufweisen oder dazu komplementären Sequenzen zum Nachweisen von *aadB*;
SEQ ID NO:55-56, Teilen davon, die eine Länge von mindestens 12 Nukleotiden aufweisen oder dazu komplementären Sequenzen zum Nachweisen von *aacC1*;
SEQ ID NO:57-58, Teilen davon, die eine Länge von mindestens 12 Nukleotiden aufweisen oder dazu komplementären Sequenzen zum Nachweisen von *aacC2*;
SEQ ID NO:59-60, Teilen davon, die eine Länge von mindestens 12 Nukleotiden aufweisen oder dazu komplementäre Sequenzen zum Nachweisen von *aacC3*;
SEQ ID NO:61-64, Teilen davon, die eine Länge von mindestens 12 Nukleotiden aufweisen oder dazu komplementären Sequenzen zum Nachweisen von *aac6'-Ila;*
SEQ ID NO:65-66, Teilen davon, die eine Länge von mindestens 12 Nukleotiden aufweisen oder dazu komplementären Sequenzen zum Nachweisen von *aacC4*;
SEQ ID NO:67-70, Teilen davon, die eine Länge von mindestens 12 Nukleotiden aufweisen oder dazu komplementären Sequenzen zum Nachweisen von *vanA*;
SEQ ID NO:71-74, Teilen davon, die eine Länge von mindestens 12 Nukleotiden aufweisen oder dazu komplementären Sequenzen zum Nachweisen von *vanB*;
SEQ ID NO:75-76, Teilen davon, die eine Länge von mindestens 12 Nukleotiden aufweisen oder dazu komplementären Sequenzen zum Nachweisen von *vanC*;
SEQ ID NO:77-80, Teilen davon, die eine Länge von mindestens 12 Nukleotiden aufweisen oder dazu komplementären Sequenzen zum Nachweisen von *msrA*;
SEQ ID NO:81-82, Teilen davon, die eine Länge von mindestens 12 Nukleotiden aufweisen oder dazu komplementären Sequenzen zum Nachweisen von *satA*;
SEQ ID NO:83-86, Teilen davon, die eine Länge von mindestens 12 Nukleotiden aufweisen oder dazu komplementären Sequenzen zum Nachweisen von *aac(6')-aph(2")*;
SEQ ID NO:87-88, Teilen davon, die eine Länge von mindestens 12 Nukleotiden aufweisen oder dazu komplementären Sequenzen zum Nachweisen von *vat*;
SEQ ID NO:89-90, Teilen davon, die eine Länge von mindestens 12 Nukleotiden aufweisen oder dazu komplementären Sequenzen zum Nachweisen von *vga;*
SEQ ID NO:91-92, Teilen davon, die eine Länge von mindestens 12 Nukleotiden aufweisen oder dazu komplementären Sequenzen zum Nachweisen von *ermA*;
SEQ ID NO:93-94, Teilen davon, die eine Länge von mindestens 12 Nukleotiden aufweisen oder dazu komplementären Sequenzen zum Nachweisen von *ermB*;
SEQ ID NO:95-96, Teilen davon, die eine Länge von mindestens 12 Nukleotiden aufweisen oder dazu komplementären Sequenzen zum Nachweisen von *ermC*;
SEQ ID NO:97-98, Teilen davon, die eine Länge von mindestens 12 Nukleotiden aufweisen oder dazu komplementären Sequenzen zum Nachweisen von *mecA*;
SEQ ID NO:99-102, Teilen davon, die eine Länge von mindestens 12 Nukleotiden aufweisen oder dazu komplementären Sequenzen zum Nachweisen von *int;* und
SEQ ID NO:103-106 Teilen davon, die eine Länge von mindestens 12 Nukleotiden aufweisen oder dazu komplementären Sequenzen zum Nachweisen von *sul.*

7. Verfahren nach einem der Ansprüche 1-6, wobei das Bestimmen gleichzeitig durchgeführt wird.

8. Verfahren nach einem der Ansprüche 1-7, das direkt von einer Testprobe durchgeführt wird.

9. Verfahren nach einem der Ansprüche 1-8, das direkt von einer Testprobe durchgeführt wird, die aus einer Bakterien- und/oder Pilzkultur oder -suspension besteht.

10. Verfahren nach einem der Ansprüche 1-9, wobei die Nukleinsäuren durch ein Verfahren amplifiziert werden, das ausgewählt ist aus der Gruppe bestehend aus:
a) Polymerasekettenreaktion (PCR);
b) Ligasekettenreaktion (LCR);
c) Nukleinsäuresequenz-basierender Amplifikation (NASBA);
d) Selbsttragender Sequenzreplikation (3SR);
e) Strangverdrängungsamplifikation (SDA);
f) Verzweigter DNA-Signalamplifikation (bDNA);
g) Transkriptions-vermittelter Amplifikation (TMA);
h) Zyklischer Sondentechnologie (CPT);
i) Geschachtelter PCR und
j) Multiplex-PCR.

11. Verfahren nach Anspruch 10, wobei die Nukleinsäuren durch PCR amplifiziert werden.

12. Verfahren nach Anspruch 11, wobei die Amplifikationsbedingungen einheitlich sind.

13. Verfahren nach einem der Ansprüche 1-9, das ferner Folgendes umfasst:
a) i) Ablagern und Befestigen der einen oder mehreren Nukleinsäuren der Bakterien der Probe auf einem inerten Träger oder Belassen dieser in Lösung; oder
ii) Inokulieren der Probe auf einem inerten Träger und *in situ* Lysieren der inokulierten Probe, um die eine oder mehreren Nukleinsäuren in der Probe freizusetzen, wobei die eine oder mehreren Nukleinsäuren in im Wesentlichen einzelsträngiger Form vorliegen;
b) in Kontakt bringen der im Wesentlichen einzelsträngigen einen oder mehreren Nukleinsäuren mit einer Oligonukleotidsonde, die eine Läge von mindestens 12 Nukleotiden aufweist, die abgeleitet ist von und spezifisch hybridisiert mit einer in SEQ ID NO:138 definierten *tuf* Nukleotidsequenz oder einer dazu komplementären Sequenz;
c) Nachweisen der Gegenwart des Hybridisierungskomplexes auf dem inerten Träger oder in der Lösung als Anhaltspunkt für die Gegenwart und/oder die Menge der einen oder mehreren *tuf* Nukleinsäuren aus Bakterien in der Probe.

14. Verfahren nach einem der Ansprüche 1-12, das ferner die Verwendung eines Primerpaars umfasst zum Nachweis von bakteriellen oder pilzlichen Nukleinsäuren, wobei das Primerpaar ausgewählt wird aus der Gruppe bestehend aus SEQ ID NO:23 und 24, 107 und 108 und 109 und 172.

15. Isoliertes Oligonukleotid, das eine Länge von mindestens 12 Nukleotiden aufweist, das abgeleitet ist von und spezifisch hybridisiert mit SEQ ID NO:138 oder einer dazu komplementären Sequenz, zum Bestimmen der Gegenwart und/oder Menge eines oder mehrerer *tuf* Nukleinsäuren der Art *Listeria monocytogenes.*

16. Isolierte *tuf* Nukleinsäure von *Listeria monocytogenes,* die eine in SEQ ID NO:138 definierte Nukleotidsequenz aufweist, ein Teil davon oder eine dazu komplementäre Sequenz.

17. Rekombinantes Plasmid, welches das isolierte Oligonukleotid nach Anspruch 15 oder die Nukleinsäure nach Anspruch 16 umfasst.

18. Rekombinanter Wirt, der mit dem rekombinanten Plasmid nach Anspruch 17 transformiert wurde.

19. Rekombinanter Wirt nach Anspruch 18, wobei der Wirt *Escherichia coli* ist.

20. Diagnostischer Kit zum Bestimmen oder Nachweisen der Gegenwart und/oder Menge einer oder mehrerer Nukleinsäuren aus der Bakterienart *Listeria monocytogenes* in einer Probe, der eine beliebige geeignete Kombination artspezifischer Sonden und/oder Primer umfasst, die eine Länge von mindestens 12 Nukleotiden aufweisen, die abgeleitet sind von und spezifisch hybridisieren mit SEQ ID NO:138 oder einer dazu komplementären Sequenz, zum Bestimmen oder Nachweisen der Gegenwart und/oder Menge eines oder mehrerer *tuf* Nukleinsäuren der Art *Listeria monocytogenes.*

21. Diagnostischer Kit nach Anspruch 20, der ferner eine beliebige geeignete Kombination von Sonden und/oder Primern umfasst, die eine Länge von mindestens 12 Nukleotiden aufweisen, die abgeleitet sind von und spezifisch hybridisieren mit einer oder mehreren der Nukleotidsequenzen ausgewählt aus der Gruppe bestehend aus SEQ ID NO:110-117 und dazu komplementären Sequenzen, zum Bestimmen und/oder Quantifizieren der Nukleinsäuren einer beliebigen Kombination der Bakterienresistenzgene ausgewählt aus der Gruppe bestehend aus *blaₒₓₐ, blaZ, aac6'-Ila, ermA, ermB, ermC und vanC.*

22. Diagnostischer Kit nach Anspruch 20, der ferner eine beliebige geeignete Kombination von Primern umfasst, die eine Nukleotidsequenz umfassen, die ausgewählt ist aus der Gruppe bestehend aus SEQ ID NO:37-106, 173 und 174, Teilen davon, die eine Länge von mindestens 12 Nukleotiden aufweisen und dazu komplementären Sequenzen, zum gleichzeitigen Bestimmen und/oder Quantifizieren von Nukleinsäuren beliebiger Bakterienresistenzgene ausgewählt aus der Gruppe bestehend aus *blaₜₑₘ, bla_{rob}, blaₛₕᵥ, blaₒₓₐ, blaZ, arcB, aacC1, aacC2, aacC3, aacA4, aac6'-Ila, aad(6), ermA, ermB, ermC, mecA, vanA, vanB, vanC, satA, aac(6)-aph(2'), vat, vga, msrA, sul* und *int.*

23. Diagnostischer Kit nach einem der Ansprüche 20-22, der ferner eine beliebige geeignete Kombination von Primern umfasst, die eine Nukleotidsequenz umfassen, die ausgewählt ist aus der Gruppe bestehend aus SEQ ID NO:1, 2 und 5-23, Teilen davon, die eine Länge von mindestens 12 Nukleotiden aufweisen und dazu komplementären Sequenzen, zum Bestimmen und/oder Quantifizieren von Nukleinsäuren einer beliebigen Bakterien- und/oder Pilzart und/oder -gattung ausgewählt aus der Gruppe bestehend aus *Enterococcus faecium, Staphylococcus saprophyticus, Neisseria meningitidis, Streptococcus agalactiae, Candida albicans,* der Gattung *Enterococcus,* der Gattung *Staphylococcus,* der Gattung *Neisseria* und der Gattung *Streptococcus.*

24. Diagnostischer Kit nach einem der Ansprüche 20-23, der ferner eine beliebige geeignete Kombination von Primern umfasst, die eine Länge von mindestens 12 Nukleotiden aufweisen, wobei die Kombination von Primern ein Primerpaar umfasst, das aus einer Nukleotidsequenz besteht, die ausgewählt ist aus der Gruppe bestehend aus SEQ ID NO:23, 24, 107, 108, 109 und 172 und dazu komplementären Sequenzen, zum gleichzeitigen universellen Bestimmen und/oder Quantifizieren von Nukleinsäuren eines Bakteriums oder Pilzes.

25. Diagnostischer Kit nach Anspruch 24, wobei das Primerpaar aus den in SEQ ID NOs: 23 und 24, 107 und 108 oder 109 und 172 definierten Nukleinsäuresequenzen besteht.

26. Isoliertes Oligonukleotid nach Anspruch 15, wobei das Oligonukleotid aus einer Länge von 12-30 Nukleotiden besteht.

27. Diagnostischer Kit nach einem der Ansprüche 20-25, wobei die Sonden und/oder Primer eine Länge von 12-30 Nukleotiden aufweisen.

28. Verfahren nach einem der Ansprüche 1-12 und 14, wobei Multiplex-Amplifikation verwendet wird.

29. Verfahren nach einem der Ansprüche 1-12, 14 und 28, wobei das Oligonukleotid in der Lage ist, dessen Zielnukleinsäure spezifisch zu amplifizieren unter Verwendung von PCR-Bedingungen, die für jeden Amplifikationszyklus einen Denaturierungsschritt von 1 s bei 95 °C und einen Anlagerungs-Verlängerungsschritt von 30 s bei 45-55 °C umfassen.

30. Verfahren nach einem der Ansprüche 1-12, 14 und 28, wobei das Oligonukleotid in der Lage ist, dessen Zielnukleinsäure spezifisch zu amplifizieren unter Verwendung von PCR-Bedingungen, die für jeden Amplifikationszyklus einen Denaturierungsschritt von 1 s bei 95 °C und einen Anlagerungs-Verlängerungsschritt von 30 s bei 55 °C umfassen.

31. Oligonukleotid nach Anspruch 15 oder 26 oder diagnostischer Kit nach einem der Ansprüche 20-25 und 27, wobei das Oligonukleotid oder die Sonden und/oder Primer in der Lage sind, deren Zielnukleinsäure(n) spezifisch zu amplifizieren unter Verwendung von PCR-Bedingungen, die für jeden Amplifikationszyklus einen Denaturierungsschritt von 1 s bei 95 °C und einen Anlagerungs-Verlängerungsschritt von 30 s bei 45-55 °C umfassen.

32. Oligonukleotid nach Anspruch 15 oder 26 oder diagnotischer Kit nach einem der Ansprüche 20-25 und 27, wobei das Oligonukleotid oder die Sonden und/oder Primer in der Lage sind, deren Zielnukleinsäure(n) spezifisch zu amplifizieren unter Verwendung von PCR-Bedingungen, die für jeden Amplifikationszyklus einen Denaturierungsschritt von 1 s bei 95 °C und einen Anlagerungs-Verlängerungsschritt von 30 s bei 55 °C umfassen.

33. Verfahren nach einem der Ansprüche 1-14 und 28, wobei das Oligonukleotid in der Lage ist, spezifisch mit dessen Zielnukleinsäure zu hybridisieren unter den folgenden Bedingungen:
- Hybridisierung in Vorhybridisierungslösung bei 65 °C über Nacht gefolgt von Nachhybridisierungs-Waschgängen:
zweimal in 3X SSC enthaltend 1% SDS 15 min lang bei 65 °C;
zweimal in 2X SSC enthaltend 1% SDS 15 min lang bei 65 °C;
zweimal in 1X SSC enthaltend 1% SDS 15 min lang bei 65 °C; und
einem finalen Waschgang in 0,1X SSC enthaltend 1% SDS 15 min lang bei 25 °C.

34. Oligonukleotid nach Anspruch 15 oder 26 oder diagnostischer Kit nach einem der Ansprüche 20-25 und 27, wobei das Oligonukleotid oder die Sonden und/oder Primer in der Lage sind, spezifisch mit deren Zielnukleinsäure(n) zu hybridisieren unter den folgenden Bedingungen:
- Hybridisierung in Vorhybridisierungslösung bei 65 °C über Nacht gefolgt von Nachhybridisierungs-Waschgängen:
zweimal in 3X SSC enthaltend 1 % SDS 15 min lang bei 65 °C;
zweimal in 2X SSC enthaltend 1 % SDS 15 min lang bei 65 °C;
zweimal in 1X SSC enthaltend 1% SDS 15 min lang bei 65 °C; und
einem finalen Waschgang in 0,1X SSC enthaltend 1% SDS 15 min lang bei 25 °C.

35. Verfahren nach einem der Ansprüche 1-14, 28-30 und 33, wobei das Oligonukleotid aus einer Länge von 12-30 Nukleotiden besteht.

## Revendications

1. Procédé de détermination ou de détection de la présence et/ou de la quantité d'un ou de plusieurs acides nucléiques *tuf* issus de l'espèce bactérienne *Listeria monocytogenes* dans un échantillon, ledit procédé comprenant :
a) i) la mise en contact dudit échantillon avec au moins un oligonucléotide d'une longueur d'au moins 12 nucléotides issu de et qui s'hybride spécifiquement à SEQ ID NO : 138 ou une séquence complémentaire de celle-ci, pour réaliser un essai d'hybridation ; ou
ii) la mise en contact dudit échantillon avec au moins deux oligonucléotides, au moins un nucléotide ayant une longueur d'au moins 12 nucléotides et étant issu de et s'hybridant spécifiquement à SEQ ID NO : 138 ou une séquence complémentaire de celle-ci, pour réaliser une réaction d'amplification ; et
b) la détection de la présence, de la quantité, ou des deux, d'oligonucléotides hybridés ou de produits amplifiés en a) en tant qu'indication de la présence, de la quantité, ou des deux, dudit ou desdits acides nucléiques *tuf* issus de *Listeria monocytogenes.*

2. Procédé selon la revendication 1, comprenant en outre la détermination de la présence et/ou de la quantité d'un ou de plusieurs acides nucléiques issus d'une espèce et/ou d'un genre bactériens et/ou fongiques sélectionnés dans le groupe constitué de *Enterococcus faecium, Neisseria meningitidis, Staphylococcus saprophyticus, Streptococcus agalactiae, Candida albicans,* un ou plusieurs membres du genre *Enterococcus,* un ou plusieurs membres du genre *Neisseria,* un ou plusieurs membres du genre *Staphylococcus,* un ou plusieurs membres du genre *Streptococcus* et un ou plusieurs membres du genre *Candida,*
dans lequel ledit procédé comprend au moins un oligonucléotide ayant une longueur d'au moins 12 nucléotides pour déterminer la présence et/ou la quantité d'un ou plusieurs acides nucléiques issus de ladite espèce bactérienne et/ou fongique qui est issu de et s'hybride spécifiquement à :
SEQ ID NO : 26 ou une séquence complémentaire de celle-ci pour déterminer la présence ou la quantité d'un ou de plusieurs acides nucléiques de *Enterococcus faecium ;*
SEQ ID NO : 28 ou une séquence complémentaire de celle-ci pour déterminer la présence ou la quantité d'un ou de plusieurs acides nucléiques de *Neisseria meningitidis ;*
SEQ ID NO : 29 ou une séquence complémentaire de celle-ci pour déterminer la présence ou la quantité d'un ou de plusieurs acides nucléiques de *Staphylococcus saprophyticus ;*
SEQ ID NO : 30 ou une séquence complémentaire de celle-ci pour déterminer la présence ou la quantité d'un ou de plusieurs acides nucléiques de *Streptococcus agalactiae ;* ou
SEQ ID NO : 120 ou une séquence complémentaire de celle-ci pour déterminer la présence ou la quantité d'un ou de plusieurs acides nucléiques de *Candida albicans* ; ou
dans lequel ledit procédé comprend en outre au moins un oligonucléotide pour déterminer la présence et/ou la quantité d'un ou de plusieurs acides nucléiques issus dudit genre bactérien et/ou fongique qui est issu de et s'hybride spécifiquement à :
SEQ ID NO : 131 à 134 ou des séquences complémentaires de celles-ci pour déterminer la présence ou la quantité d'un ou de plusieurs acides nucléiques issus d'un ou plusieurs membres du genre *Enterococcus ;*
SEQ ID NO : 31 ou une séquence complémentaire de celle-ci pour déterminer la présence ou la quantité d'un ou de plusieurs acides nucléiques issus d'un ou plusieurs membres du genre *Neisseria ;*
SEQ ID NO : 140 à 143 ou des séquences complémentaires de celles-ci pour déterminer la présence ou la quantité d'un ou de plusieurs acides nucléiques issus d'un ou plusieurs membres du genre *Staphylococcus ;*
SEQ ID NO : 33 à 36 ou des séquences complémentaires de celles-ci pour déterminer la présence ou la quantité d'un ou de plusieurs acides nucléiques issus d'un ou plusieurs membres du genre *Streptococcus ;* ou
SEQ ID NO : 120 à 124 ou des séquences complémentaires de celles-ci pour déterminer la présence ou la quantité d'un ou de plusieurs acides nucléiques issus d'un ou plusieurs membres du genre *Candida.*

3. Procédé selon la revendication 2, dans lequel la présence et/ou la quantité dudit ou desdits acides nucléiques issus d'espèces et/ou de genres bactériens et/ou fongiques sont déterminées, dans lequel au moins un oligonucléotide comprend une séquence de nucléotides sélectionnée dans le groupe constitué de :
SEQ ID NO: 1 et 2, des parties de celles-ci ou des séquences complémentaires de celles-ci, pour déterminer la présence ou la quantité d'un ou de plusieurs acides nucléiques de *Enterococcus faecium* ;
SEQ ID NO: 5 et 6, des parties de celles-ci ou des séquences complémentaires de celles-ci, pour déterminer la présence ou la quantité d'un ou de plusieurs acides nucléiques de *Neisseria meningitidis* ;
SEQ ID NO: 7 et 8, des parties de celles-ci ou des séquences complémentaires de celles-ci, pour déterminer la présence ou la quantité d'un ou de plusieurs acides nucléiques de *Staphylococcus saprophyticus* ;
SEQ ID NO: 9 et 10, des parties de celles-ci ou des séquences complémentaires de celles-ci, pour déterminer la présence ou la quantité d'un ou de plusieurs acides nucléiques de *Streptococcus agalactiae* ;
SEQ ID NO: 11 et 12, des parties de celles-ci ou des séquences complémentaires de celles-ci, pour déterminer la présence ou la quantité d'un ou de plusieurs acides nucléiques de *Candida albicans* ;
SEQ ID NO: 13 et 14, des parties de celles-ci ou des séquences complémentaires de celles-ci, pour déterminer la présence ou la quantité d'un ou de plusieurs acides nucléiques issus d'un ou de plusieurs membres du genre *Enterococcus ;*
SEQ ID NO: 15 et 16, des parties de celles-ci ou des séquences complémentaires de celles-ci, pour déterminer la présence ou la quantité d'un ou de plusieurs acides nucléiques issus d'un ou de plusieurs membres du genre *Neisseria* ;
SEQ ID NO: 17 à 20, des parties de celles-ci ou des séquences complémentaires de celles-ci, pour déterminer la présence ou la quantité d'un ou de plusieurs acides nucléiques issus d'un ou de plusieurs membres du genre *Staphylococcus ;* et
SEQ ID NO: 21 et 22, des parties de celles-ci ou des séquences complémentaires de celles-ci, pour déterminer la présence ou la quantité d'un ou de plusieurs acides nucléiques issus d'un ou de plusieurs membres du genre *Streptococcus.*

4. Procédé selon l'une quelconque des revendications 1 à 3, comprenant en outre la détermination de la présence et/ou de la quantité d'un ou de plusieurs acides nucléiques issus d'un ou de plusieurs gènes bactériens de résistance aux antibiotiques sélectionnés dans le groupe constitué de *blaₜₑₘ, blaₛₕᵥ, bla_{rob}, blaₒₓₐ, blaZ, aadB, aacC1,* aacC2, aacC3, *aac6'-IIa, aacA4, aad(6'), vanA, vanB, vanC, msrA, satA, aac(6')-aph(2"), vat, vga, ermA, ermB, ermC, mecA, int* et *sul.*

5. Procédé selon la revendication 4, dans lequel ledit ou lesdits gènes bactériens de résistance aux antibiotiques sont sélectionnés dans le groupe constitué de *blaₒₓₐ, blaZ, aac6'-IIa, vanB, vanC, ermA, ermB and ermC,* et dans lequel ledit procédé comprend au moins un oligonucléotide d'une longueur d'au moins 12 nucléotides pour déterminer la présence d'un ou plusieurs acides nucléiques issus d'un ou de plusieurs gènes bactériens de résistance aux antibiotiques qui est issu de et s'hybride spécifiquement à au moins une des séquences de nucléotides sélectionnées dans le groupe constitué de :
SEQ ID NO : 110 ou une séquence complémentaire de celle-ci pour la détection de blaₒₓₐ ;
SEQ ID NO : 111 ou une séquence complémentaire de celle-ci pour la détection de *blaZ* ;
SEQ ID NO : 112 ou une séquence complémentaire de celle-ci pour la détection de *aac6'-IIa* ;
SEQ ID NO : 113 ou une séquence complémentaire de celle-ci pour la détection de *ermA* ;
SEQ ID NO : 114 ou une séquence complémentaire de celle-ci pour la détection de *ermB* ;
SEQ ID NO : 115 ou une séquence complémentaire de celle-ci pour la détection de *ermC* ;
SEQ ID NO : 116 ou une séquence complémentaire de celle-ci pour la détection de *vanB* ; et
SEQ ID NO : 117 ou une séquence complémentaire de celle-ci pour la détection de *vanC.*

6. Procédé selon la revendication 4, comprenant au moins un oligonucléotide pour déterminer la présence et/ou la quantité d'un ou de plusieurs acides nucléiques issus d'un ou de plusieurs gènes bactériens de résistance aux antibiotiques, dans lequel ledit ou lesdits oligonucléotides comprennent une séquence de nucléotides sélectionnée dans le groupe constitué de :
SEQ ID NO : 37 à 40, des parties de celles-ci ayant une longueur d'au moins 12 nucléotides ou des séquences complémentaires de celles-ci pour la détection de blaₜₑₘ ;
SEQ ID NO : 41 à 44, des parties de celles-ci ayant une longueur d'au moins 12 nucléotides ou des séquences complémentaires de celles-ci pour la détection de blaₛₕᵥ ;
SEQ ID NO : 45 à 48, des parties de celles-ci ayant une longueur d'au moins 12 nucléotides ou des séquences complémentaires de celles-ci pour la détection de bla_{rob} ;
SEQ ID NO : 49 à 50, des parties de celles-ci ayant une longueur d'au moins 12 nucléotides ou une séquence complémentaire de celles-ci pour la détection de blaₒₓₐ ;
SEQ ID NO : 51 à 52, des parties de celles-ci ayant une longueur d'au moins 12 nucléotides ou des séquences complémentaires de celles-ci pour la détection de *blaZ* ;
SEQ ID NO : 53 à 54, des parties de celles-ci ayant une longueur d'au moins 12 nucléotides ou des séquences complémentaires de celles-ci pour la détection de *aadB* ;
SEQ ID NO : 55 à 56, des parties de celles-ci ayant une longueur d'au moins 12 nucléotides ou des séquences complémentaires de celles-ci pour la détection de *aacC1* ;
SEQ ID NO : 57 à 58, des parties de celles-ci ayant une longueur d'au moins 12 nucléotides ou des séquences complémentaires de celles-ci pour la détection de aacC2 ;
SEQ ID NO : 59 à 60, des parties de celles-ci ayant une longueur d'au moins 12 nucléotides ou des séquences complémentaires de celles-ci pour la détection de aacC3 ;
SEQ ID NO : 61 à 64, des parties de celles-ci ayant une longueur d'au moins 12 nucléotides ou des séquences complémentaires de celles-ci pour la détection de *aac6'-IIa* ;
SEQ ID NO : 65 à 66, des parties de celles-ci ayant une longueur d'au moins 12 nucléotides ou des séquences complémentaires de celles-ci pour la détection de aacC4 ;
SEQ ID NO: 67 à 70, des parties de celles-ci ou des séquences complémentaires de celles-ci pour la détection de *vanA* ;
SEQ ID NO : 71 à 74, des parties de celles-ci ayant une longueur d'au moins 12 nucléotides ou des séquences complémentaires de celles-ci pour la détection de *vanB* ;
SEQ ID NO : 75 à 76, des parties de celles-ci ayant une longueur d'au moins 12 nucléotides ou des séquences complémentaires de celles-ci pour la détection de *vanC* ;
SEQ ID NO : 77 à 80, des parties de celles-ci ayant une longueur d'au moins 12 nucléotides ou des séquences complémentaires de celles-ci pour la détection de *msrA* ;
SEQ ID NO : 81 à 82, des parties de celles-ci ayant une longueur d'au moins 12 nucléotides ou des séquences complémentaires de celles-ci pour la détection de *satA* ;
SEQ ID NO : 83 à 86, des parties de celles-ci ayant une longueur d'au moins 12 nucléotides ou des séquences complémentaires de celles-ci pour la détection de *aac(6')-aph(2")* ;
SEQ ID NO : 87 à 88, des parties de celles-ci ayant une longueur d'au moins 12 nucléotides ou des séquences complémentaires de celles-ci pour la détection de *vat ;*
SEQ ID NO : 89 à 90, des parties de celles-ci ayant une longueur d'au moins 12 nucléotides ou des séquences complémentaires de celles-ci pour la détection de *vga* ;
SEQ ID NO : 91 à 92, des parties de celles-ci ayant une longueur d'au moins 12 nucléotides ou des séquences complémentaires de celles-ci pour la détection de *ermA* ;
SEQ ID NO : 93 à 94, des parties de celles-ci ayant une longueur d'au moins 12 nucléotides ou une séquence complémentaire de celles-ci pour la détection de *ermB* ;
SEQ ID NO : 95 à 96, des parties de celles-ci ayant une longueur d'au moins 12 nucléotides ou des séquences complémentaires de celles-ci pour la détection de *ermC* ;
SEQ ID NO : 97 à 98, des parties de celles-ci ayant une longueur d'au moins 12 nucléotides ou des séquences complémentaires de celles-ci pour la détection de *mecA* ;
SEQ ID NO : 99 à 102, des parties de celles-ci ayant une longueur d'au moins 12 nucléotides ou des séquences complémentaires de celles-ci pour la détection de *int* ; et
SEQ ID NO : 103 à 106, des parties de celles-ci ayant une longueur d'au moins 12 nucléotides ou des séquences complémentaires de celles-ci pour la détection de *sul.*

7. Procédé selon l'une quelconque des revendications 1 à 6, dans lequel ladite détermination est réalisée simultanément.

8. Procédé selon l'une quelconque des revendications 1 à 7, qui est réalisé directement à partir d'un échantillon de test.

9. Procédé selon l'une quelconque des revendications 1 à 8, qui est réalisé directement à partir d'un échantillon de test constitué d'une culture ou une suspension bactérienne et/ou fongique.

10. Procédé selon l'une quelconque des revendications 1 à 9, dans lequel lesdits acides nucléiques sont amplifiés par un procédé sélectionné dans le groupe constitué de :
a) une réaction de polymérisation en chaîne (PCR),
b) une réaction de ligature en chaîne (LCR),
c) une amplification basée sur une séquence d'acide nucléique (NASBA),
d) une réplication de séquence auto-entretenue (3SR),
e) une amplification par déplacement de brin (SDA),
f) une amplification de signal par ADN branché (bDNA),
g) une amplification médiée par la transcription (TMA),
h) une technologie d'amplification par dégradation cyclique de sonde (CPT),
i) une PCR nichée, et
j) une PCR multiplexe.

11. Procédé selon la revendication 10, dans lequel lesdits acides nucléiques sont amplifiés par PCR.

12. Procédé selon la revendication 11, dans lequel les conditions d'amplification sont uniformes.

13. Procédé selon l'une quelconque des revendications 1 à 9, comprenant en outre :
a) i) le dépôt et la fixation sur un support inerte dudit ou desdits acides nucléiques de bactéries dudit échantillon, ou le fait de laisser en solution ledit ou lesdits acides nucléiques de bactéries dudit échantillon, ou
ii) l'inoculation dudit échantillon sur un support inerte, et la lyse *in situ* dudit échantillon inoculé afin de libérer ledit ou lesdits acides nucléiques dans ledit échantillon, ledit ou lesdits acides nucléiques étant sous une forme essentiellement simple brin ;
b) la mise en contact dudit ou desdits acides nucléiques essentiellement simple brin avec une sonde oligonucléotidique d'une longueur d'au moins 12 nucléotides issue de et qui s'hybride spécifiquement à une séquence de nucléotides *tuf* définie dans SEQ ID NO : 138 ou une séquence complémentaire de celle-ci ;
c) la détection de la présence dudit complexe d'hybridation sur ledit support inerte ou dans ladite solution en tant qu'indication de la présence et/ou de la quantité dudit ou desdits acides nucléiques *tuf* issus de bactéries dans ledit échantillon.

14. Procédé selon l'une quelconque des revendications 1 à 12, comprenant en outre l'utilisation d'une paire d'amorces pour la détection d'acides nucléiques bactériens ou fongiques, dans lequel ladite paire d'amorces est sélectionnée dans le groupe constitué de SEQ ID NO : 23 et 24, 107 et 108, et 109 et 172.

15. Oligonucléotide isolé ayant une longueur d'au moins 12 nucléotides issu de et qui s'hybride spécifiquement à SEQ ID NO : 138, ou une séquence complémentaire de celle-ci, pour déterminer la présence et/ou la quantité d'un ou de plusieurs acides nucléiques *tuf* issus de l'espèce *Listeria monocytogenes.*

16. Acide nucléique *tuf* issu de *Listeria monocytogenes* ayant la séquence de nucléotides définie dans SEQ ID NO : 138, une partie de celle-ci ou une séquence complémentaire de celle-ci.

17. Plasmide recombinant comprenant l'oligonucléotide selon la revendication 15 ou l'acide nucléique selon la revendication 16.

18. Hôte recombinant qui a été transformé par le plasmide recombinant selon la revendication 17.

19. Hôte recombinant selon la revendication 18, dans lequel ledit hôte est *Escherichia coli.*

20. Kit de diagnostic pour déterminer ou détecter la présence et/ou la quantité d'un ou de plusieurs acides nucléiques de l'espèce bactérienne *Listeria monocytogenes* dans un échantillon, comprenant toute combinaison adaptée de sondes et/ou d'amorces spécifiques d'une espèce d'une longueur d'au moins 12 nucléotides issues de et qui s'hybrident spécifiquement à SEQ ID NO: 138 ou une séquence complémentaire de celle-ci, pour déterminer ou détecter la présence et/ou la quantité d'un ou de plusieurs acides nucléiques *tuf* issus de l'espèce *Listeria monocytogenes.*

21. Kit de diagnostic selon la revendication 20, comprenant en outre toute combinaison adaptée de sondes et/ou d'amorces d'une longueur d'au moins 12 nucléotides issues de et qui s'hybrident spécifiquement à une ou plusieurs des séquences de nucléotides sélectionnées dans le groupe constitué de SEQ ID NO : 110 à 117 et des séquences complémentaires de celles-ci, pour la détection et/ou la quantification des acides nucléiques de toute combinaison des gènes de résistance bactériens sélectionnés dans le groupe constitué de *blaₒₓₐ, blaZ, aac6'-IIa, ermA, ermB, ermC,* et *vanC.*

22. Kit de diagnostic selon la revendication 20, comprenant en outre toute combinaison adaptée d'amorces comprenant une séquence de nucléotides sélectionnée dans le groupe constitué de SEQ ID NO : 37 à 106, 173 et 174, des parties de celles-ci ayant une longueur d'au moins 12 nucléotides et des séquences complémentaires de celles-ci, pour la détection et/ou la quantification simultanées d'acides nucléiques de tout gène bactérien de résistance aux antibiotiques sélectionné dans le groupe constitué de *blaₜₑₘ, bla_{rob}, blaₛₕᵥ, blaₒₓₐ, blaZ, aadB, aacC1, aacC2*, aacC3, *aacA4, aac6'-IIa, aad(6'), ermA, ermB, ermC, mecA, vanA, vanB, vanC, satA, aac(6')-aph(2"), vat, vga, msrA, sul* et *int.*

23. Kit de diagnostic selon l'une quelconque des revendications 20 à 22, comprenant en outre toute combinaison adaptée d'amorces comprenant une séquence de nucléotides sélectionnée dans le groupe constitué de SEQ ID NO : 1, 2 et 5 à 23, des parties de celles-ci ayant une longueur d'au moins 12 nucléotides et des séquences complémentaires de celles-ci, pour la détection et/ou la quantification d'acides nucléiques de toute espèce et/ou de tout genre bactériens et/ou fongiques sélectionnés dans le groupe constitué de *Enterococcus faecium, Staphylococcus saprophyticus, Neisseria meningitidis, Streptococcus agalactiae, Candida albicans,* du genre *Enterococcus,* du genre *Staphylococcus,* du genre *Neisseria* et du genre *Streptococcus.*

24. Kit de diagnostic selon l'une quelconque des revendications 20 à 23, comprenant en outre toute combinaison adaptée d'amorces ayant une longueur d'au moins 12 nucléotides, ladite combinaison d'amorces comprenant une paire d'amorces constituée d'une séquence de nucléotides sélectionnée dans le groupe constitué de SEQ ID NO : 23, 24, 107, 108, 109 et 172 et des séquences complémentaires de celles-ci, pour la détection et/ou la quantification universelles simultanées d'acides nucléiques d'une bactérie ou d'un champignon.

25. Kit de diagnostic selon la revendication 24, dans lequel ladite paire d'amorces est constituée de séquences d'acides nucléiques définies dans SEQ ID NO : 23 et 24 ; 107 et 108 ou 109 et 172.

26. Oligonucléotide isolé selon la revendication 15, dans lequel ledit oligonucléotide est d'une longueur de 12 à 30 nucléotides.

27. Kit de diagnostic selon l'une quelconque des revendications 20 à 25, dans lequel lesdites sondes et/ou lesdites amorces sont d'une longueur de 12 à 30 nucléotides.

28. Procédé selon l'une quelconque des revendications 1 à 12 et 14, dans lequel une amplification multiplexe est utilisée.

29. Procédé selon l'une quelconque des revendications 1 à 12, 14 et 28, dans lequel ledit oligonucléotide est capable de spécifiquement amplifier son acide nucléique cible en utilisant des conditions de PCR comprenant pour chaque cycle d'amplification une étape de dénaturation de 1 s à 95°C et une étape de renaturation-extension de 30 s à 45 à 55°C.

30. Procédé selon l'une quelconque des revendications 1 à 12, 14 et 28, dans lequel ledit oligonucléotide est capable de spécifiquement amplifier son acide nucléique cible en utilisant des conditions de PCR comprenant pour chaque cycle d'amplification une étape de dénaturation de 1 s à 95°C et une étape de renaturation-extension de 30 s à 55°C.

31. Oligonucléotide selon la revendication 15 ou 26 ou le kit de diagnostic selon l'une quelconque des revendications 20 à 25 et 27, dans lequel ledit oligonucléotide ou lesdites sondes et/ou lesdites amorces sont capables de spécifiquement amplifier leur(s) acide(s) nucléique(s) cible(s) en utilisant des conditions de PCR comprenant pour chaque cycle d'amplification une étape de dénaturation de 1 s à 95°C et une étape de renaturation-extension de 30 s à 45 à 55°C.

32. Oligonucléotide selon la revendication 15 ou 26 ou le kit de diagnostic selon l'une quelconque des revendications 20 à 25 et 27, dans lequel ledit oligonucléotide ou lesdites sondes et/ou lesdites amorces sont capables de spécifiquement amplifier leur(s) acide(s) nucléique(s) cible(s) en utilisant des conditions de PCR comprenant pour chaque cycle d'amplification une étape de dénaturation de 1 s à 95°C et une étape de renaturation-extension de 30 s à 55°C.

33. Procédé selon l'une quelconque des revendications 1 à 14 et 28, dans lequel ledit oligonucléotide est capable de spécifiquement s'hybrider à son acide nucléique cible dans les conditions suivantes :
- hybridation dans une solution de pré-hybridation à 65°C pendant une nuit suivie par des lavages post-hybridation :
deux fois dans du SSC 3X contenant du SDS à 1 % à 65°C pendant 15 minutes ;
deux fois dans du SSC 2X contenant du SDS à 1 % à 65°C pendant 15 minutes ;
deux fois dans du SSC 1X contenant du SDS à 1 % à 65°C pendant 15 minutes ; et
un lavage final dans du SSC 0,1X contenant du SDS à 1 % à 25°C pendant 15 minutes.

34. Oligonucléotide selon la revendication 15 ou 26, ou kit de diagnostic selon l'une quelconque des revendications 20 à 25 et 27, dans lequel ledit oligonucléotide ou lesdites sondes et/ou lesdites amorces sont capables de spécifiquement s'hybrider à leur(s) acide(s) nucléique(s) cible(s) dans les conditions suivantes :
- hybridation dans une solution de pré-hybridation à 65°C pendant une nuit suivie par des lavages post-hybridation :
deux fois dans du SSC 3X contenant du SDS à 1 % à 65°C pendant 15 minutes ;
deux fois dans du SSC 2X contenant du SDS à 1 % à 65°C pendant 15 minutes ;
deux fois dans du SSC 1X contenant du SDS à 1 % à 65°C pendant 15 minutes ; et
un lavage final dans du SSC 0,1X contenant du SDS à 1 % à 25°C pendant 15 minutes.

35. Procédé selon l'une quelconque des revendications 1 à 14, 28 à 30 et 33, dans lequel ledit oligonucléotide est d'une longueur de 12 à 30 nucléotides.


### SEQUENCE LISTING

<110> GENEOHM SCIENCES CANADA INC.
<120> SPECIES-SPECIFIC, GENUS-SPECIFIC AND UNIVERSAL DNA PROBES AND AMPLIFICATION PRIMERS TO RAPIDLY DETECT AND IDENTIFY COMMON BACTERIAL AND FUNGAL PATHOGENS AND ASSOCIATED ANTIBIOTIC RESISTANCE GENES...
<130> GOH13863EPD2
<140> Divisional Application based on EP09163281.0
   <141> 2009-06-19
<150> US 08/743,637
   <151> 1996-11-04
<160> 174
<170> PatentIn version 3.3
<210> 1
   <211> 22
   <212> DNA
   <213> Artificial Sequence
<220>
   <223> Oligonucleotide that binds to Enterococcus faecium
<400> 1
   tgctttagca acagcctatc ag 22
<210> 2
   <211> 21
   <212> DNA
   <213> Artificial Sequence
<220>
   <223> Oligonucleotide that binds to Enterococcus faecium
<400> 2
   taaacttctt ccggcacttc g 21
<210> 3
   <211> 21
   <212> DNA
   <213> Artificial Sequence
<220>
   <223> Oligonucleotide that binds to Listeria monocytogenes
<400> 3
   tgcggctata aatgaagagg c 21
<210> 4
   <211> 21
   <212> DNA
   <213> Artificial Sequence
<220>
   <223> Oligonucleotide that binds to Listeria monocytogenes
<400> 4
   atccgatgat gctatggctt t 21
<210> 5
   <211> 21
   <212> DNA
   <213> Artificial Sequence
<220>
   <223> Oligonucleotide that binds to Neisseria meningitidis
<400> 5
   ccagcggtat tgtttggtgg t 21
<210> 6
   <211> 21
   <212> DNA
   <213> Artificial Sequence
<220>
   <223> Oligonucleotide that binds to Neisseria meningitidis
<400> 6
   caggcggcct ttaataattt c 21
<210> 7
   <211> 30
   <212> DNA
   <213> Artificial Sequence
<220>
   <223> Oligonucleotide that binds to Staphylococcus saprophyticus
<400> 7
   agatcgaatt ccacatgaag gttattatga 30
<210> 8
   <211> 30
   <212> DNA
   <213> Artificial Sequence
<220>
   <223> Oligonucleotide that binds to Staphylococcus saprophyticus
<400> 8
   tcgcttctcc ctcaacaatc aaactatcct 30
<210> 9
   <211> 23
   <212> DNA
   <213> Artificial Sequence
<220>
   <223> Oligonucleotide that binds to Streptococcus agalactiae
<400> 9
   tttcaccagc tgtattagaa gta 23
<210> 10
   <211> 23
   <212> DNA
   <213> Artificial Sequence
<220>
   <223> Oligonucleotide that binds to Streptococcus agalactiae
<400> 10
   gttccctgaa cattatcttt gat 23
<210> 11
   <211> 26
   <212> DNA
   <213> Artificial Sequence
<220>
   <223> Oligonucleotide that binds to Candida albicans
<400> 11
   caagaaggtt ggttacaacc caaaga 26
<210> 12
   <211> 26
   <212> DNA
   <213> Artificial Sequence
<220>
   <223> Oligonucleotide that binds to Candida albicans
<400> 12
   aggtcttacc agtaacttta ccggat 26
<210> 13
   <211> 22
   <212> DNA
   <213> Artificial Sequence
<220>
   <223> Oligonucleotide that binds to Enterococcus spp.
<400> 13
   tactgacaaa ccattcatga tg 22
<210> 14
   <211> 21
   <212> DNA
   <213> Artificial Sequence
<220>
   <223> Oligonucleotide that binds to Enterococcus spp.
<400> 14
   aacttcgtca ccaacgcgaa c 21
<210> 15
   <211> 20
   <212> DNA
   <213> Artificial Sequence
<220>
   <223> Oligonucleotide that binds to Neisseria spp.
<400> 15
   ctggcgcggt atggtcggtt 20
<210> 16
   <211> 22
   <212> DNA
   <213> Artificial Sequence
<220>
   <223> Oligonucleotide that binds to Neisseria spp.
<400> 16
   gccgacgttg gaagtggtaa ag 22
<210> 17
   <211> 25
   <212> DNA
   <213> Artificial Sequence
<220>
   <223> Oligonucleotide that binds to Staphylococcus spp.
<400> 17
   ccgtgttgaa cgtggtcaaa tcaaa 25
<210> 18
   <211> 25
   <212> DNA
   <213> Artificial Sequence
<220>
   <223> Oligonucleotide that binds to Staphylococcus spp.
<400> 18
   trtgtggtgt ratwgwrcca ggagc 25
<210> 19
   <211> 25
   <212> DNA
   <213> Artificial Sequence
<220>
   <223> Oligonucleotide that binds to Staphylococcus spp.
<400> 19
   acaacgtggw caagtwttag cwgct 25
<210> 20
   <211> 25
   <212> DNA
   <213> Artificial Sequence
<220>
   <223> Oligonucleotide that binds to Staphylococcus spp.
<400> 20
   accatttcwg taccttctgg taagt 25
<210> 21
   <211> 23
   <212> DNA
   <213> Artificial Sequence
<220>
   <223> Oligonucleotide that binds to Streptococcus spp.
<220>
   <221> modified_base
   <222> (12)..(12)
   <223> n = inosine
<400> 21
   gaaattgcag gnaaattgat tga 23
<210> 22
   <211> 23
   <212> DNA
   <213> Artificial Sequence
<220>
   <223> Oligonucleotide that binds to Streptococcus spp.
<220>
   <221> misc feature
   <222> (12)..(12)
   <223> n = inosine
<400> 22
   ttacgcatgg cntgactcat cat 23
<210> 23
   <211> 23
   <212> DNA
   <213> Artificial Sequence
<220>
   <223> Oligonucleotide that binds to several species (universal detection)
<220>
   <221> modified_base
   <222> (3)..(3)
   <223> n = inosine
<220>
   <221> modified base
   <222> (6)..(6)
   <223> n = inosine
<220>
   <221> modified base
   <222> (9)..(9)
   <223> n = inosine
<220>
   <221> modified base
   <222> (12)..(12)
   <223> n = inosine
<220>
   <221> modified base
   <222> (15)..(15)
   <223> n = inosine
<400> 23
   acnkknacng gngtngarat gtt 23
<210> 24
   <211> 23
   <212> DNA
   <213> Artificial Sequence
<220>
   <223> Oligonucleotide that binds to several species (universal detection)
<220>
   <221> misc feature
   <222> (6)..(6)
   <223> n = inosine
<220>
   <221> misc feature
   <222> (9)..(9)
   <223> n = inosine
<220>
   <221> misc feature
   <222> (12)..(12)
   <223> n = inosine
<220>
   <221> misc feature
   <222> (18)..(18)
   <223> n = inosine
<400> 24
   ayrttntcnc cnggcatnac cat 23
<210> 25
   <211> 10
   <212> DNA
   <213> Artificial Sequence
<220>
   <223> Oligonucleotide that binds to Staphylococcus saprophyticus (AP-PCR primer
   OPAD-9)
<400> 25
   tcgcttctcc 10
<210> 26
   <211> 600
   <212> DNA
   <213> Enterococcus faecium
<400> 26
   ttcttagaga cattgaatat gccttatgtc ggcgcaggcg tattgaccag tgcatgtgcc     60
   atggataaaa tcatgaccaa gtatatttta caagctgctg gtgtgccgca agttccttat    120
   gtaccagtac ttaagaatca atggaaagaa aatcctaaaa aagtatttga tcaatgtgaa    180
   ggttctttgc tttatccgat gtttgtcaaa cctgcgaata tgggttctag tgtcggcatt    240
   acaaaggcag aaaaccgaga agagctgcaa aatgctttag caacagccta tcagtatgat    300
   tctcgagcaa tcgttgaaca aggaattgaa gcgcgcgaaa tcgaagttgc tgtattagga    360
   aatgaagatg ttcggacgac tttgcctggc gaagtcgtaa aagacgtagc attctatgat    420
   tatgaagcca aatatatcaa taataaaatc gaaatgcaga ttccagccga agtgccggaa    480
   gaagtttatc aaaaagcgca agagtacgcg aagttagctt acacgatgtt aggtggaagc    540
   ggattgagcc ggtgcgattt ctttttgaca aataaaaatg aattattcct gaatgaatta    600
<210> 27
   <211> 1920
   <212> DNA
   <213> Listeria monocytogenes
<400> 27
   gtgggattaa acagatttat gcgtgcgatg atggtggttt tcattactgc caattgcatt     60
   acgattaacc ccgacataat atttgcagcg acagatagcg aagattctag tctaaacaca    120
   gatgaatggg aagaagaaaa aacagaagag caaccaagcg aggtaaatac gggaccaaga    180
   tacgaaactg cacgtgaagt aagttcacgt gatattaaag aactagaaaa atcgaataaa    240
   gtgagaaata cgaacaaagc agacctaata gcaatgttga aagaaaaagc agaaaaaggt    300
   ccaaatatca ataataacaa cagtgaacaa actgagaatg cggctataaa tgaagaggct    360
   tcaggagccg accgaccagc tatacaagtg gagcgtcgtc atccaggatt gccatcggat    420
   agcgcagcgg aaattaaaaa aagaaggaaa gccatagcat catcggatag tgagcttgaa    480
   agccttactt atccggataa accaacaaaa gtaaataaga aaaaagtggc gaaagagtca    540
   gttgcggatg cttctgaaag tgacttagat tctagcatgc agtcagcaga tgagtcttca    600
   ccacaacctt taaaagcaaa ccaacaacca tttttcccta aagtatttaa aaaaataaaa    660
   gatgcgggga aatgggtacg tgataaaatc gacgaaaatc ctgaagtaaa gaaagcgatt    720
   gttgataaaa gtgcagggtt aattgaccaa ttattaacca aaaagaaaag tgaagaggta    780
   aatgcttcgg acttcccgcc accacctacg gatgaagagt taagacttgc tttgccagag    840
   acaccaatgc ttcttggttt taatgctcct gctacatcag aaccgagctc attcgaattt    900
   ccaccaccac ctacggatga agagttaaga cttgctttgc cagagacgcc aatgcttctt    960
   ggttttaatg ctcctgctac atcggaaccg agctcgttcg aatttccacc gcctccaaca   1020
   gaagatgaac tagaaatcat ccgggaaaca gcatcctcgc tagattctag ttttacaaga   1080
   ggggatttag ctagtttgag aaatgctatt aatcgccata gtcaaaattt ctctgatttc   1140
   ccaccaatcc caacagaaga agagttgaac gggagaggcg gtagaccaac atctgaagaa   1200
   tttagttcgc tgaatagtgg tgattttaca gatgacgaaa acagcgagac aacagaagaa   1260
   gaaattgatc gcctagctga tttaagagat agaggaacag gaaaacactc aagaaatgcg   1320
   ggttttttac cattaaatcc gtttgctagc agcccggttc cttcgttaag tccaaaggta 1380
   tcgaaaataa gcgaccgggc tctgataagt gacataacta aaaaaacgcc atttaagaat 1440
   ccatcacagc cattaaatgt gtttaataaa aaaactacaa cgaaaacagt gactaaaaaa 1500
   ccaacccctg taaagaccgc accaaagcta gcagaacttc ctgccacaaa accacaagaa 1560
   accgtactta gggaaaataa aacacccttt atagaaaaac aagcagaaac aaacaagcag 1620
   tcaattaata tgccgagcct accagtaatc caaaaagaag ctacagagag cgataaagag 1680
   gaaatgaaac cacaaaccga ggaaaaaatg gtagaggaaa gcgaatcagc taataacgca 1740
   aacggaaaaa atcgttctgc tggcattgaa gaaggaaaac taattgctaa aagtgcagaa 1800
   gacgaaaaag cgaaggaaga accagggaac catacgacgt taattcttgc aatgttagct 1860
   attggcgtgt tctctttagg ggcgtttatc aaaattattc aattaagaaa aaataattaa 1920
<210> 28
   <211> 415
   <212> DNA
   <213> Neisseria meningitidis
<400> 28
   taccggtacg ctaaatattg gtgatgtatt ggatattatg atttgggaag cgccgccagc 60
   ggtattgttt ggtggtggcc tttcttcgat gggctcgggt agtgcgcaac aaaccaagtt 120
   gccggagcaa ctggtgacgg cacgtggtac ggtttctgtg ccgtttgttg gcgatatttc 180
   ggtggtcggt aaaacgcctg gtcaggttca ggaaattatt aaaggccgcc tgaaaaaaat 240
   ggccaatcag ccgcaagtga tggtgcgctt ggtgcagaat aatgcggcaa atgtatcggt 300
   gattcgcgca ggcaatagtg tgcgtatgcc gttgacggca gccggtgagc gtgtgttgga 360
   tgcggtggct gcggtaggtg gttcaacggc aaatgtgcag gatacgaatg tgcag 415
<210> 29
   <211> 438
   <212> DNA
   <213> Staphylococcus saprophyticus
<400> 29
   tcgcttctcc agaagaaatt ttagaaacat atctagaaaa tcccaaatta gataaaccgt 60
   ttatattatg tgaatacgca catgcaatgg gaaattcacc aggagatctt aatgcatatc 120
   aaacattaat tgaaaaatat gatagtttta ttggcggttt tgtttgggaa tggtgtgatc 180
   atagcattca ggttgggata aaggaaggta aaccaatttt tagatatggt ggagattttg 240
   gtgaggcctt acatgacggt aatttttgtg ttgatggtat tgtttcgcca gatcgaattc 300
   cacatgaagg ttattatgag tttaaacatg aacatagacc tttgagattg gttaacgaag 360
   aggattatcg gtttacattg aagaatcaat ttgattttac aaatgcggag gatagtttga 420
   ttgttgaggg agaagcga 438
<210> 30
   <211> 768
   <212> DNA
   <213> Streptococcus agalactiae
<400> 30
   atgaacgtta cacatatgat gtatctatct ggaactctag tggctggtgc attgttattt 60
   tcaccagctg tattagaagt acatgctgat caagtgacaa ctccacaagt ggtaaatcat 120
   gtaaatagta ataatcaagc ccagcaaatg gctcaaaagc ttgatcaaga tagcattcag 180
   ttgagaaata tcaaagataa tgttcaggga acagattatg aaaaaccggt taatgaggct 240
   attactagcg tggaaaaatt aaagacttca ttgcgtgcca accctgagac agtttatgat 300
   ttgaattcta ttggtagtcg tgtagaagcc ttaacagatg tgattgaagc aatcactttt 360
   tcaactcaac atttaacaaa taaggttagt caagcaaata ttgatatggg atttgggata 420
   actaagctag ttattcgcat tttagatcca tttgcttcag ttgattcaat taaagctcaa 480
   gttaacgatg taaaggcatt agaacaaaaa gttttaactt atcctgattt aaaaccaact 540
   gatagagcta ccatctatac aaaatcaaaa cttgataagg aaatctggaa tacacgcttt 600
   actagagata aaaaagtact taacgtcaaa gaatttaaag tttacaatac tttaaataaa 660
   gcaatcacac atgctgttgg agttcagttg aatccaaatg ttacggtaca acaagttgat 720
   caagagattg taacattaca agcagcactt caaacagcat taaaataa 768
<210> 31
   <211> 421
   <212> DNA
   <213> Neisseria meningitidis
<400> 31
   atgaaagtag gtttcgtcgg ctggcgcggt atggtcggtt cggttttgat gcagcgtatg 60
   aaagaagaaa acgacttcgc ccacattccc gaagcgtttt tctttaccac ttccaacgtc 120
   ggcggcgcac gccctgattt cggtcaggcg gctaaaacat tattggacgc gaacaacgtt 180
   gccgagctgg caaaaatgga catcatcgtt acctgccaag gcggcgacta caccaaatcc 240
   gtcttccaag ccctgcgcga cagcggctgg aacggctact ggattgacgc ggcatcctcg 300
   ctgcgtatga aagacgacgc gattatcgtc ctcgaccccg tcaaccgcaa cgtcatcgac 360
   aacggcctca aaaacggcgt gaaaaactac atcggcggca actgtaccgt ttccctgatg 420
   c 421
<210> 32
   <211> 213
   <212> DNA
   <213> Streptococcus gordonii
<400> 32
   ttcatagacg ctgagcacgc tttggatcca tcttacgcgg ctgctctagg tgtaaatatt 60
   gatgagctgt tgctatctca accagattct ggtgagcaag gtttagaaat tgcaggaaaa 120
   ttgattgact ctggggcagt tgatttagtt gtcatcgact ctgttgcagc tcttgtacca 180
   cgtgcggaaa tcgatggaga tatcggtgat agc 213
<210> 33
   <211> 692
   <212> DNA
   <213> Streptococcus mutans
<400> 33
   gggccggaat cttctggtaa gacaactgtc gctcttcatg ctgctgctca ggcgcaaaaa 60
   gatggcggta ttgccgcttt cattgatgca gaacatgccc ttgatccagc ctatgctgct 120
   gctcttggcg ttaatattga tgagcttttg ctttcacaac cagattcagg agaacagggt 180
   cttgaaattg cagggaaatt gattgattct ggcgctgttg atttagttgt tgttgactca 240
   gtggcagctt tagtaccacg tgcggagatt gacggagata ttggtaatag tcatgttggc 300
   ttacaagcac gcatgatgag tcaagcgatg cgtaaattat cagcttcaat caataaaaca 360
   aaaaccattg ctatttttat taatcaattg cgggaaaaag ttggtattat gtttggtaat 420
   ccagaaacaa cccctggcgg gcgtgccttg aagttttatt cttctgtgcg tcttgatgtc 480
   cgcggcaata ctcaaattaa aggaaccggg gaacaaaaag acagcaatat tggtaaagag 540
   accaaaatta aagttgttaa aaataaagtt gctccaccat ttaaggaagc ttttgtagaa 600
   attatatatg gtgaaggcat ttctcgtaca ggtgaattag ttaagattgc cagtgatttg 660
   ggaattatcc aaaaagctgg agcttggtac tc 692
<210> 34
   <211> 1204
   <212> DNA
   <213> Streptococcus pneumoniae
<400> 34
   atggcgaaaa aaccaaaaaa attagaagaa atttcaaaaa aatttggggc agaacgtgaa 60
   aaggccttga atgacgctct taaattgatt gagaaagact ttggtaaagg atcaatcatg 120
   cgtttgggtg aacgtgcgga gcaaaaggtg caagtgatga gctcaggttc tttagctctt 180
   gacattgccc ttggctcagg tggttatcct aagggacgta tcatcgaaat ctatggccca 240
   gagtcatctg gtaagacaac ggttgccctt catgcagttg cacaagcgca aaaagaaggt 300
   gggattgctg cctttatcga tgcggaacat gcccttgatc cagcttatgc tgcggccctt 360
   ggtgtcaata ttgacgaatt gctcttgtct caaccagact caggagagca aggtcttgag 420
   attgcgggaa aattgattga ctcaggtgca gttgatcttg tcgtagtcga ctcagttgct 480
   gcccttgttc ctcgtgcgga aattgatgga gatatcggag atagccatgt tggtttgcag 540
   gctcgtatga tgagccaggc catgcgtaaa cttggcgcct ctatcaataa aaccaaaaca 600
   attgccattt ttatcaacca attgcgtgaa aaagttggag tgatgtttgg aaatccagaa 660
   acaacaccgg gcggacgtgc tttgaaattc tatgcttcag tccgcttgga tgttcgtggt 720
   aatacacaaa ttaagggaac tggtgatcaa aaagaaacca atgtcggtaa agaaactaag 780
   attaaggttg taaaaaataa ggtagctcca ccgtttaagg aagccgtagt tgaaattatg 840
   tacggagaag gaatttctaa gactggtgag cttttgaaga ttgcaagcga tttggatatt 900
   atcaaaaaag caggggcttg gtattcttac aaagatgaaa aaattgggca aggttctgag 960
   aatgctaaga aatacttggc agagcaccca gaaatctttg atgaaattga taagcaagtc 1020
   cgttctaaat ttggcttgat tgatggagaa gaagtttcag aacaagatac tgaaaacaaa 1080
   aaagatgagc caaagaaaga agaagcagtg aatgaagaag ttccgcttga cttaggcgat 1140
   gaacttgaaa tcgaaattga agaataagct gttaaagcag tggagaaatc cgctactttt 1200
   tcga 1204
<210> 35
   <211> 981
   <212> DNA
   <213> Streptococcus pyogenes
<400> 35
   atgcgttcag gaagtctagc tcttgatatt gcttggatag ctggtggtta tcctaaagga 60
   cgtatcatcg aaatctatgg tccagagtct tccggtaaaa cgactgtggc tttacatgct 120
   gtagcacaag ctcaaaaaga aggtggaatc gcagccttta tcgatgccga gcatgcgctt 180
   gatccagctt atgctgctgc gcttggggtt aatattgatg aacttctctt gtctcaacca 240
   gattctggag aacaaggact tgaaattgca ggtaaattga ttgattctgg tgcggttgac 300
   ctggttgttg tcgattcagt agcagcttta gtgccacgtg ctgaaattga tggtgatatt 360
   ggcgatagcc atgtcggatt gcaagcacgt atgatgagtc aggccatgcg taaattatca 420
   gcttctatta ataaaacaaa aactatcgca atctttatca accaattgcg tgaaaaagtt 480
   ggtgtgatgt ttggaaatcc tgaaacaaca ccaggtggtc gagctttgaa attctatgct 540
   tctgttcggc tggatgtgcg tggaaacaac caaattaaag gaactggtga ccaaaagata 600
   gccagcattg gtaaggagac caaaatcaag gttgttaaaa acaaggtcgc tccgccattt 660
   aaggtagcag aagttgaaat catgtatggg gaaggtattt ctcgtacagg ggagcttgtg 720
   aaaattgctt ctgatttgga cattatccaa aaagcaggtg cttggttctc ttataatggt 780
   gagaagattg gccaaggttc tgaaaatgct aagcgttatt tggccgatca tccacaattg 840
   tttgatgaaa tcgaccgtaa agtacgtgtt aaatttggtt tgcttgaaga aagcgaagaa 900
   gaatctgcta tggcagtagc atcagaagaa accgatgatc ttgctttaga tttagataat 960
   ggtattgaaa ttgaagatta a 981
<210> 36
   <211> 312
   <212> DNA
   <213> Streptococcus salivarius
<400> 36
   gcgtatgcac gagctctagg tgttaatatc gatgagcttc ttttgtcgca gcctgattct 60
   ggtgagcaag gtctcgaaat tgcaggtaag ctgattgact ctggtgcagt ggatttagtt 120
   gttgttgact cagttgcggc cttcgtacca cgtgcagaaa ttgatggaga tagtggtgac 180
   agtcatgtag gacttcaagc gcgtatgatg agtcaagcca tgcgtaaact ttctgcatct 240
   attaataaaa caaaaacgat tgctatcttt attaaccagt tgcgtgaaaa agttggtatc 300
   atgtttggta ac 312
<210> 37
   <211> 20
   <212> DNA
   <213> Artificial Sequence
<220>
   <223> Oligonucleotide that binds to antibiotic resistance gene of
   Enterobacteriaceae,Pseudomonadaceae,Haemophilus spp.,Neisseria spp.
<400> 37
   ctatgtggcg cggtattatc 20
<210> 38
   <211> 20
   <212> DNA
   <213> Artificial Sequence
<220>
   <223> Oligonucleotide that binds to antibiotic resistance gene of
   Enterobacteriaceae,Pseudomonadaceae,Haemophilus spp.,Neisseria spp.
<400> 38
   cgcagtgtta tcactcatgg 20
<210> 39
   <211> 20
   <212> DNA
   <213> Artificial Sequence
<220>
   <223> Oligonucleotide that binds to antibiotic resistance gene of
   Enterobacteriaceae,Pseudomonadaceae,Haemophilus spp.,Neisseria spp.
<400> 39
   ctgaatgaag ccataccaaa 20
<210> 40
   <211> 20
   <212> DNA
   <213> Artificial Sequence
<220>
   <223> Oligonucleotide that binds to antibiotic resistance gene of
   Enterobacteriaceae, Pseudomonadaceae,Haemophilus spp.,Neisseria spp.
<400> 40
   atcagcaata aaccagccag 20
<210> 41
   <211> 20
   <212> DNA
   <213> Artificial Sequence
<220>
   <223> Oligonucleotide that binds to antibiotic resistance gene of Klebsiella
   spp.,and other Enterobacteriaceae
<400> 41
   ttaccatgag cgataacagc 20
<210> 42
   <211> 20
   <212> DNA
   <213> Artificial Sequence
<220>
   <223> Oligonucleotide that binds to antibiotic resistance gene of Klebsiella
   spp.,and other Enterobacteriaceae
<400> 42
   ctcattcagt tccgtttccc 20
<210> 43
   <211> 20
   <212> DNA
   <213> Artificial Sequence
<220>
   <223> Oligonucleotide that binds to antibiotic resistance gene of Klebsiella
   spp.,and other Enterobacteriaceae
<400> 43
   cagctgctgc agtggatggt 20
<210> 44
   <211> 20
   <212> DNA
   <213> Artificial Sequence
<220>
   <223> Oligonucleotide that binds to antibiotic resistance gene of Klebsiella
   spp.,and other Enterobacteriaceae
<400> 44
   cgctctgctt tgttattcgg 20
<210> 45
   <211> 20
   <212> DNA
   <213> Artificial Sequence
<220>
   <223> Oligonucleotide that binds to antibiotic resistance gene of Haemophilus
   spp.,Pasteurella spp.
<400> 45
   tacgccaaca tcgtggaaag 20
<210> 46
   <211> 20
   <212> DNA
   <213> Artificial Sequence
<220>
   <223> Oligonucleotide that binds to antibiotic resistance gene of Haemophilus
   spp.,Pasteurella spp.
<400> 46
   ttgaatttgg cttcttcggt 20
<210> 47
   <211> 20
   <212> DNA
   <213> Artificial Sequence
<220>
   <223> Oligonucleotide that binds to antibiotic resistance gene of Haemophilus
   spp.,Pasteurella spp.
<400> 47
   gggatacaga aacgggacat 20
<210> 48
   <211> 20
   <212> DNA
   <213> Artificial Sequence
<220>
   <223> Oligonucleotide that binds to antibiotic resistance gene of Haemophilus
   spp.,Pasteurella spp.
<400> 48
   taaatctttt tcaggcagcg 20
<210> 49
   <211> 25
   <212> DNA
   <213> Artificial Sequence
<220>
   <223> Oligonucleotide that binds to antibiotic resistance gene of
   Enterobacteriaceae,Pseudomonadaceae
<400> 49
   gatggtttga agggtttatt ataag 25
<210> 50
   <211> 25
   <212> DNA
   <213> Artificial Sequence
<220>
   <223> Oligonucleotide that binds to antibiotic resistance gene of
   Enterobacteriaceae,Pseudomonadaceae
<400> 50
   aatttagtgt gtttagaatg gtgat 25
<210> 51
   <211> 21
   <212> DNA
   <213> Artificial Sequence
<220>
   <223> Oligonucleotide that binds to antibiotic resistance gene of Enterococcus
   spp.
<400> 51
   acttcaacac ctgctgcttt c 21
<210> 52
   <211> 21
   <212> DNA
   <213> Artificial Sequence
<220>
   <223> Oligonucleotide that binds to antibiotic resistance gene of Enterococcus
   spp.
<400> 52
   tgaccacttt tatcagcaac c 21
<210> 53
   <211> 20
   <212> DNA
   <213> Artificial Sequence
<220>
   <223> Oligonucleotide that binds to antibiotic resistance gene of
   Enterobacteriaceae,Pseudomonadaceae
<400> 53
   ggcaatagtt gaaatgctcg 20
<210> 54
   <211> 20
   <212> DNA
   <213> Artificial Sequence
<220>
   <223> Oligonucleotide that binds to antibiotic resistance gene of
   Enterobacteriaceae,Pseudomonadaceae
<400> 54
   cagctgttac aacggactgg 20
<210> 55
   <211> 20
   <212> DNA
   <213> Artificial Sequence
<220>
   <223> Oligonucleotide that binds to antibiotic resistance gene of
   Enterobacteriaceae,Pseudomonadaceae
<400> 55
   tctatgatct cgcagtctcc 20
<210> 56
   <211> 20
   <212> DNA
   <213> Artificial Sequence
<220>
   <223> Oligonucleotide that binds to antibiotic resistance gene of
   Enterobacteriaceae,Pseudomonadaceae
<400> 56
   atcgtcaccg taatctgctt 20
<210> 57
   <211> 20
   <212> DNA
   <213> Artificial Sequence
<220>
   <223> Oligonucleotide that binds to antibiotic resistance gene of
   Enterobacteriaceae,Pseudomonadaceae
<400> 57
   cattctcgat tgctttgcta 20
<210> 58
   <211> 20
   <212> DNA
   <213> Artificial Sequence
<220>
   <223> Oligonucleotide that binds to antibiotic resistance gene of bacteria
<400> 58
   ccgaaatgct tctcaagata 20
<210> 59
   <211> 20
   <212> DNA
   <213> Artificial Sequence
<220>
   <223> Oligonucleotide that binds to antibiotic resistance gene of
   Enterobacteriaceae,Pseudomonadaceae
<400> 59
   ctggattatg gctacggagt 20
<210> 60
   <211> 20
   <212> DNA
   <213> Artificial Sequence
<220>
   <223> Oligonucleotide that binds to antibiotic resistance gene of
   Enterobacteriaceae,Pseudomonadaceae
<400> 60
   agcagtgtga tggtatccag 20
<210> 61
   <211> 20
   <212> DNA
   <213> Artificial Sequence
<220>
   <223> Oligonucleotide that binds to antibiotic resistance gene of
   Pseudomonadaceae
<400> 61
   gactcttgat gaagtgctgg 20
<210> 62
   <211> 20
   <212> DNA
   <213> Artificial Sequence
<220>
   <223> Oligonucleotide that binds to antibiotic resistance gene of
   Pseudomonadaceae
<400> 62
   ctggtctatt cctcgcactc 20
<210> 63
   <211> 20
   <212> DNA
   <213> Artificial Sequence
<220>
   <223> Oligonucleotide that binds to antibiotic resistance gene of
   Pseudomonadaceae
<400> 63
   tatgagaagg caggattcgt 20
<210> 64
   <211> 20
   <212> DNA
   <213> Artificial Sequence
<220>
   <223> Oligonucleotide that binds to antibiotic resistance gene of
   Pseudomonadaceae
<400> 64
   gctttctctc gaaggcttgt 20
<210> 65
   <211> 20
   <212> DNA
   <213> Artificial Sequence
<220>
   <223> Oligonucleotide that binds to antibiotic resistance gene of
   Enterobacteriaceae,Pseudomonadaceae
<400> 65
   gagttgctgt tcaatgatcc 20
<210> 66
   <211> 20
   <212> DNA
   <213> Artificial Sequence
<220>
   <223> Oligonucleotide that binds to antibiotic resistance gene of
   Enterobacteriaceae,Pseudomonadaceae
<400> 66
   gtgtttgaac catgtacacg 20
<210> 67
   <211> 20
   <212> DNA
   <213> Artificial Sequence
<220>
   <223> Oligonucleotide that binds to antibiotic resistance gene of Enterococcus
   spp.
<400> 67
   tgtagaggtc tagcccgtgt 20
<210> 68
   <211> 20
   <212> DNA
   <213> Artificial Sequence
<220>
   <223> Oligonucleotide that binds to antibiotic resistance gene of Enterococcus
   spp.
<400> 68
   acggggataa cgactgtatg 20
<210> 69
   <211> 20
   <212> DNA
   <213> Artificial Sequence
<220>
   <223> Oligonucleotide that binds to antibiotic resistance gene of Enterococcus
   spp.
<400> 69
   ataaagatga taggccggtg 20
<210> 70
   <211> 20
   <212> DNA
   <213> Artificial Sequence
<220>
   <223> Oligonucleotide that binds to antibiotic resistance gene of Enterococcus
   spp.
<400> 70
   tgctgtcata ttgtcttgcc 20
<210> 71
   <211> 20
   <212> DNA
   <213> Artificial Sequence
<220>
   <223> Oligonucleotide that binds to antibiotic resistance gene of Enterococcus
   spp.
<400> 71
   attatcttcg gcggttgctc 20
<210> 72
   <211> 20
   <212> DNA
   <213> Artificial Sequence
<220>
   <223> Oligonucleotide that binds to antibiotic resistance gene of Enterococcus
   spp.
<400> 72
   gactatcggc ttcccattcc 20
<210> 73
   <211> 20
   <212> DNA
   <213> Artificial Sequence
<220>
   <223> Oligonucleotide that binds to antibiotic resistance gene of Enterococcus
   spp.
<400> 73
   cgatagaagc agcaggacaa 20
<210> 74
   <211> 20
   <212> DNA
   <213> Artificial Sequence
<220>
   <223> Oligonucleotide that binds to antibiotic resistance gene of Enterococcus
   spp.
<400> 74
   ctgatggatg cggaagatac 20
<210> 75
   <211> 21
   <212> DNA
   <213> Artificial Sequence
<220>
   <223> Oligonucleotide that binds to antibiotic resistance gene of Enterococcus
   spp.
<400> 75
   gccttatgta tgaacaaatg g 21
<210> 76
   <211> 23
   <212> DNA
   <213> Artificial Sequence
<220>
   <223> Oligonucleotide that binds to antibiotic resistance gene of Enterococcus
   spp.
<400> 76
   gtgactttwg tgatcccttt tga 23
<210> 77
   <211> 20
   <212> DNA
   <213> Artificial Sequence
<220>
   <223> Oligonucleotide that binds to antibiotic resistance gene of
   Staphylococcus spp.
<400> 77
   tccaatcatt gcacaaaatc 20
<210> 78
   <211> 20
   <212> DNA
   <213> Artificial Sequence
<220>
   <223> Oligonucleotide that binds to antibiotic resistance gene of
   Staphylococcus spp.
<400> 78
   aattccctct atttggtggt 20
<210> 79
   <211> 20
   <212> DNA
   <213> Artificial Sequence
<220>
   <223> Oligonucleotide that binds to antibiotic resistance gene of
   Staphylococcus spp.
<400> 79
   tcccaagcca gtaaagctaa 20
<210> 80
   <211> 20
   <212> DNA
   <213> Artificial Sequence
<220>
   <223> Oligonucleotide that binds to antibiotic resistance gene of
   Staphylococcus spp.
<400> 80
   tggtttttca acttcttcca 20
<210> 81
   <211> 20
   <212> DNA
   <213> Artificial Sequence
<220>
   <223> Oligonucleotide that binds to antibiotic resistance gene of Enterococcus
   spp.
<400> 81
   tcatagaatg gatggctcaa 20
<210> 82
   <211> 20
   <212> DNA
   <213> Artificial Sequence
<220>
   <223> Oligonucleotide that binds to antibiotic resistance gene of Enterococcus
   spp.
<400> 82
   agctactatt gcaccatccc 20
<210> 83
   <211> 22
   <212> DNA
   <213> Artificial Sequence
<220>
   <223> Oligonucleotide that binds to antibiotic resistance gene of Enterococcus
   spp.,Staphylococcus spp.
<400> 83
   caataagggc ataccaaaaa tc 22
<210> 84
   <211> 22
   <212> DNA
   <213> Artificial Sequence
<220>
   <223> Oligonucleotide that binds to antibiotic resistance gene of Enterococcus
   spp.,Staphylococcus spp.
<400> 84
   ccttaacatt tgtggcatta tc 22
<210> 85
   <211> 22
   <212> DNA
   <213> Artificial Sequence
<220>
   <223> Oligonucleotide that binds to antibiotic resistance gene of Enterococcus
   spp.,Staphylococcus spp.
<400> 85
   ttgggaagat gaagttttta ga 22
<210> 86
   <211> 22
   <212> DNA
   <213> Artificial Sequence
<220>
   <223> Oligonucleotide that binds to antibiotic resistance gene of Enterococcus
   spp.,Staphylococcus spp.
<400> 86
   cctttactcc aataatttgg ct 22
<210> 87
   <211> 20
   <212> DNA
   <213> Artificial Sequence
<220>
   <223> Oligonucleotide that binds to antibiotic resistance gene of
   Staphylococcus spp.
<400> 87
   tttcatctat tcaggatggg 20
<210> 88
   <211> 20
   <212> DNA
   <213> Artificial Sequence
<220>
   <223> Oligonucleotide that binds to antibiotic resistance gene of
   Staphylococcus spp.
<400> 88
   ggagcaacat tctttgtgac 20
<210> 89
   <211> 20
   <212> DNA
   <213> Artificial Sequence
<220>
   <223> Oligonucleotide that binds to antibiotic resistance gene of
   Staphylococcus spp.
<400> 89
   tgtgcctgaa gaaggtattg 20
<210> 90
   <211> 20
   <212> DNA
   <213> Artificial Sequence
<220>
   <223> Oligonucleotide that binds to antibiotic resistance gene of
   Staphylococcus spp.
<400> 90
   cgtgttactt caccaccact 20
<210> 91
   <211> 23
   <212> DNA
   <213> Artificial Sequence
<220>
   <223> Oligonucleotide that binds to antibiotic resistance gene of
   Staphylococcus spp.
<400> 91
   tatcttatcg ttgagaaggg att 23
<210> 92
   <211> 22
   <212> DNA
   <213> Artificial Sequence
<220>
   <223> Oligonucleotide that binds to antibiotic resistance gene of
   Staphylococcus spp.
<400> 92
   ctacacttgg cttaggatga aa 22
<210> 93
   <211> 24
   <212> DNA
   <213> Artificial Sequence
<220>
   <223> Oligonucleotide that binds to antibiotic resistance gene of
   Staphylococcus spp.
<400> 93
   ctatctgatt gttgaagaag gatt 24
<210> 94
   <211> 24
   <212> DNA
   <213> Artificial Sequence
<220>
   <223> Oligonucleotide that binds to antibiotic resistance gene of
   Staphylococcus spp.
<400> 94
   gtttactctt ggtttaggat gaaa 24
<210> 95
   <211> 22
   <212> DNA
   <213> Artificial Sequence
<220>
   <223> Oligonucleotide that binds to antibiotic resistance gene of
   Staphylococcus spp.
<400> 95
   cttgttgatc acgataattt cc 22
<210> 96
   <211> 22
   <212> DNA
   <213> Artificial Sequence
<220>
   <223> Oligonucleotide that binds to antibiotic resistance gene of
   Staphylococcus spp.
<400> 96
   atcttttagc aaacccgtat tc 22
<210> 97
   <211> 27
   <212> DNA
   <213> Artificial Sequence
<220>
   <223> Oligonucleotide that binds to antibiotic resistance gene of
   Staphylococcus spp.
<400> 97
   aacaggtgaa ttattagcac ttgtaag 27
<210> 98
   <211> 27
   <212> DNA
   <213> Artificial Sequence
<220>
   <223> Oligonucleotide that binds to antibiotic resistance gene of
   Staphylococcus spp.
<400> 98
   attgctgtta atattttttg agttgaa 27
<210> 99
   <211> 19
   <212> DNA
   <213> Artificial Sequence
<220>
   <223> Oligonucleotide that binds to antibiotic resistance gene of
   Enterobacteriaceae,Pseudomonadaceae
<400> 99
   gtgatcgaaa tccagatcc 19
<210> 100
   <211> 19
   <212> DNA
   <213> Artificial Sequence
<220>
   <223> Oligonucleotide that binds to antibiotic resistance gene of
   Enterobacteriaceae,Pseudomonadaceae
<400> 100
   atcctcggtt ttctggaag 19
<210> 101
   <211> 19
   <212> DNA
   <<213> Artificial Sequence
<220>
   <223> Oligonucleotide that binds to antibiotic resistance gene of
   Enterobacteriaceae,Pseudomonadaceae
<400> 101
   ctggtcatac atgtgatgg 19
<210> 102
   <211> 19
   <212> DNA
   <213> Artificial Sequence
<220>
   <223> Oligonucleotide that binds to antibiotic resistance gene of
   Enterobacteriaceae,Pseudomonadaceae
<400> 102
   gatgttaccc gagagcttg 19
<210> 103
   <211> 20
   <212> DNA
   <213> Artificial Sequence
<220>
   <223> Oligonucleotide that binds to antibiotic resistance gene of
   Enterobacteriaceae,Pseudomonadaceae
<400> 103
   ttaagcgtgc ataataagcc 20
<210> 104
   <211> 20
   <212> DNA
   <213> Artificial Sequence
<220>
   <223> Oligonucleotide that binds to antibiotic resistance gene of
   Enterobacteriaceae,Pseudomonadaceae
<400> 104
   ttgcgattac ttcgccaact 20
<210> 105
   <211> 20
   <212> DNA
   <213> Artificial Sequence
<220>
   <223> Oligonucleotide that binds to antibiotic resistance gene of
   Enterobacteriaceae,Pseudomonadaceae
<400> 105
   tttactaagc ttgccccttc 20
<210> 106
   <211> 20
   <212> DNA
   <213> Artificial Sequence
<220>
   <223> Oligonucleotide that binds to antibiotic resistance gene of
   Enterobacteriaceae,Pseudomonadaceae
<400> 106
   aaaaggcagc aattatgagc 20
<210> 107
   <211> 29
   <212> DNA
   <213> Artificial Sequence
<220>
   <223> Oligonucleotide that binds to any bacterial species (tuf gene)
<220>
   <221> misc feature
   <222> (9)..(9)
   <223> n = inosine
<220>
   <221> misc feature
   <222> (12)..(12)
   <223> n = inosine
<220>
   <221> misc feature
   <222> (15)..(15)
   <223> n = inosine
<220>
   <221> misc feature
   <222> (18).. (18)
   <223> n = inosine
<220>
   <221> misc feature
   <222> (21)..(21)
   <223> n = inosine
<400> 107
   aayatgatna cnggngcngc ncaratgga 29
<210> 108
   <211> 23
   <212> DNA
   <213> Artificial Sequence
<220>
   <223> Oligonucleotide that binds to any bacterial species (tuf gene)
<220>
   <221> misc feature
   <222> (3)..(3)
   <223> n = inosine
<220>
   <221> misc feature
   <222> (6)..(6)
   <223> n = inosine
<220>
   <221> misc feature
   <222> (9)..(9)
   <223> n = inosine
<220>
   <221> misc feature
   <222> (12)..(12)
   <223> n = inosine
<400> 108
   ccnacngtnc knccrccytc rcg 23
<210> 109
   <211> 29
   <212> DNA
   <213> Artificial Sequence
<220>
   <223> Oligonucleotide that binds to any fungal species (tuf gene)
<220>
   <221> misc_feature
   <222> (6)..(6)
   <223> n = inosine
<220>
   <221> misc_feature
   <222> (12)..(12)
   <223> n = inosine
<220>
   <221> misc_feature
   <222> (15)..(15)
   <223> n = inosine
<220>
   <221> misc_feature
   <222> (18)..(18)
   <223> n = inosine
<400> 109
   carytnathg tngcngtnaa yaaratgga 29
<210> 110
   <211> 831
   <212> DNA
   <213> Enterobacteriaceae,Pseudomonadaceae
<400> 110
   atgaaaaaca caatacatat caacttcgct atttttttaa taattgcaaa tattatctac 60
   agcagcgcca gtgcatcaac agatatctct actgttgcat ctccattatt tgaaggaact 120
   gaaggttgtt ttttacttta cgatgcatcc acaaacgctg aaattgctca attcaataaa 180
   gcaaagtgtg caacgcaaat ggcaccagat tcaactttca agatcgcatt atcacttatg 240
   gcatttgatg cggaaataat agatcagaaa accatattca aatgggataa aacccccaaa 300
   ggaatggaga tctggaacag caatcataca ccaaagacgt ggatgcaatt ttctgttgtt 360
   tgggtttcgc aagaaataac ccaaaaaatt agattaaata aaatcaagaa ttatctcaaa 420
   gattttgatt atggaaatca agacttctct ggagataaag aaagaaacaa cggattaaca 480
   gaagcatggc tcgaaagtag cttaaaaatt tcaccagaag aacaaattca attcctgcgt 540
   aaaattatta atcacaatct cccagttaaa aactcagcca tagaaaacac catagagaac 600
   atgtatctac aagatctgga taatagtaca aaactgtatg ggaaaactgg tgcaggattc 660
   acagcaaata gaaccttaca aaacggatgg tttgaagggt ttattataag caaatcagga 720
   cataaatatg tttttgtgtc cgcacttaca ggaaacttgg ggtcgaattt aacatcaagc 780
   ataaaagcca agaaaaatgc gatcaccatt ctaaacacac taaatttata a 831
<210> 111
   <211> 846
   <212> DNA
   <213> Enterococcus spp.
<400> 111
   ttgaaaaagt taatattttt aattgtaatt gctttagttt taagtgcatg taattcaaac 60
   agttcacatg ccaaagagtt aaatgattta gaaaaaaaat ataatgctca tattggtgtt 120
   tatgctttag atactaaaag tggtaaggaa gtaaaattta attcagataa gagatttgcc 180
   tatgcttcaa cttcaaaagc gataaatagt gctattttgt tagaacaagt accttataat 240
   aagttaaata aaaaagtaca tattaacaaa gatgatatag ttgcttattc tcctatttta 300
   gaaaaatatg taggaaaaga tatcacttta aaagcactta ttgaggcttc aatgacatat 360
   agtgataata cagcaaacaa taaaattata aaagaaatcg gtggaatcaa aaaagttaaa 420
   caacgtctaa aagaactagg agataaagta acaaatccag ttagatatga gatagaatta 480
   aattactatt caccaaagag caaaaaagat acttcaacac ctgctgcttt cggtaagact 540
   ttaaataaac ttatcgcaaa tggaaaatta agcaaagaaa acaaaaaatt cttacttgat 600
   ttaatgttaa ataataaaag cggagatact ttaattaaag acggtgttcc aaaagactat 660
   aaggttgctg ataaaagtgg tcaagcaata acatatgctt ctagaaatga tgttgctttt 720
   gtttatccta agggccaatc tgaacctatt gttttagtca tttttacgaa taaagacaat 780
   aaaagtgata agccaaatga taagttgata agtgaaaccg ccaagagtgt aatgaaggaa 840
   ttttaa 846
<210> 112
   <211> 555
   <212> DNA
   <213> Pseudomonadaceae
<400> 112
   atgtccgcga gcaccccccc cataactctt cgcctcatga ccgagcgcga cctgccgatg 60
   ctccatgact ggctcaaccg gccgcacatc gttgagtggt ggggtggcga cgaagagcga 120
   ccgactcttg atgaagtgct ggaacactac ctgcccagag cgatggcgga agagtccgta 180
   acaccgtaca tcgcaatgct gggcgaggaa ccgatcggct atgctcagtc gtacgtcgcg 240
   ctcggaagcg gtgatggctg gtgggaagat gaaactgatc caggagtgcg aggaatagac 300
   cagtctctgg ctgacccgac acagttgaac aaaggcctag gaacaaggct tgtccgcgct 360
   ctcgttgaac tactgttctc ggaccccacc gtgacgaaga ttcagaccga cccgactccg 420
   aacaaccatc gagccatacg ctgctatgag aaggcaggat tcgtgcggga gaagatcatc 480
   accacgcctg acgggccggc ggtttacatg gttcaaacac gacaagcctt cgagagaaag 540
   cgcggtgttg cctaa 555
<210> 113
   <211> 732
   <212> DNA
   <213> Staphylococcus spp.
<400> 113
   atgaaccaga aaaaccctaa agacacgcaa aattttatta cttctaaaaa gcatgtaaaa      60
   gaaatattga atcacacgaa tatcagtaaa caagacaacg taatagaaat cggatcagga     120
   aaaggacatt ttaccaaaga gctagtcaaa atgagtcgat cagttactgc tatagaaatt     180
   gatggaggct tatgtcaagt gactaaagaa gcggtaaacc cctctgagaa tataaaagtg     240
   attcaaacgg atattctaaa attttccttc ccaaaacata taaactataa gatatatggt     300
   aatattcctt ataacatcag tacggatatt gtcaaaagaa ttacctttga aagtcaggct     360
   aaatatagct atcttatcgt tgagaaggga tttgcgaaaa gattgcaaaa tctgcaacga     420
   gctttgggtt tactattaat ggtggagatg gatataaaaa tgctcaaaaa agtaccacca     480
   ctatattttc atcctaagcc aagtgtagac tctgtattga ttgttcttga acgacatcaa     540
   ccattgattt caaagaagga ctacaaaaag tatcgatctt ttgtttataa gtgggtaaac     600
   cgtgaatatc gtgttctttt cactaaaaac caattccgac aggctttgaa gcatgcaaat     660
   gtcactaata ttaataaact atcgaaggaa caatttcttt ctattttcaa tagttacaaa     720
   ttgtttcact aa                                                         732
<210> 114
   <211> 738
   <212> DNA
   <213> Staphylococcus spp.
<400> 114
   atgaacaaaa atataaaata ttctcaaaac tttttaacga gtgaaaaagt actcaaccaa 60
   ataataaaac aattgaattt aaaagaaacc gataccgttt acgaaattgg aacaggtaaa 120
   gggcatttaa cgacgaaact ggctaaaata agtaaacagg taacgtctat tgaattagac 180
   agtcatctat tcaacttatc gtcagaaaaa ttaaaatcga atactcgtgt cactttaatt 240
   caccaagata ttctacagtt tcaattccct aacaaacaga ggtataaaat tgttgggaat 300
   attccttacc atttaagcac acaaattatt aaaaaagtgg tttttgaaag ccatgcgtct 360
   gacatctatc tgattgttga agaaggattc tacaagcgta ccttggatat tcaccgaaca 420
   ctagggttgc tcttgcacac tcaagtctcg attcagcaat tgcttaagct gccagcggaa 480
   tgctttcatc ctaaaccaag agtaaacagt gtcttaataa aacttacccg ccataccaca 540
   gatgttccag ataaatattg gaagctatat acgtactttg tttcaaaatg ggtcaatcga 600
   gaatatcgtc aactgtttac taaaaatcag tttcatcaag caatgaaaca cgccaaagta 660
   aacaatttaa gtaccgttac ttatgagcaa gtattgtcta tttttaatag ttatctatta 720
   tttaacggga ggaaataa 738
<210> 115
   <211> 735
   <212> DNA
   <213> Staphylococcus spp.
<400> 115
   atgaacgaga aaaatataaa acacagtcaa aactttatta cttcaaaaca taatatagat 60
   aaaataatga caaatataag attaaatgaa catgataata tctttgaaat cggctcagga 120
   aaagggcatt ttacccttga attagtacag aggtgtaatt tcgtaactgc cattgaaata 180
   gaccataaat tatgcaaaac tacagaaaat aaacttgttg atcacgataa tttccaagtt 240
   ttaaacaagg atatattgca gtttaaattt cctaaaaacc aatcctataa aatatttggt 300
   aatatacctt ataacataag tacggatata atacgcaaaa ttgtttttga tagtatagct 360
   gatgagattt atttaatcgt ggaatacggg tttgctaaaa gattattaaa tacaaaacgc 420
   tcattggcat tatttttaat ggcagaagtt gatatttcta tattaagtat ggttccaaga 480
   gaatattttc atcctaaacc tagagtgaat agctcactta tcagattaaa tagaaaaaaa 540
   tcaagaatat cacacaaaga taaacagaag tataattatt tcgttatgaa atgggttaac 600
   aaagaataca agaaaatatt tacaaaaaat caatttaaca attccttaaa acatgcagga 660
   attgacgatt taaacaatat tagctttgaa caattcttat ctcttttcaa tagctataaa 720
   ttatttaata agtaa 735
<210> 116
   <211> 1029
   <212> DNA
   <213> Enterococcus spp.
<400> 116
   atgaataaaa taaaagtcgc aattatcttc ggcggttgct cggaggaaca tgatgtgtcg     60
   gtaaaatccg caatagaaat tgctgcgaac attaatactg aaaaattcga tccgcactac    120
   atcggaatta caaaaaacgg cgtatggaag ctatgcaaga agccatgtac ggaatgggaa    180
   gccgatagtc tccccgccat attctccccg gataggaaaa cgcatggtct gcttgtcatg    240
   aaagaaagag aatacgaaac tcggcgtatt gacgtggctt tcccggtttt gcatggcaaa    300
   tgcggggagg atggtgcgat acagggtctg tttgaattgt ctggtatccc ctatgtaggc    360
   tgcgatattc aaagctccgc agcttgcatg gacaaatcac tggcctacat tcttacaaaa    420
   aatgcgggca tcgccgtccc cgaatttcaa atgattgaaa aaggtgacaa accggaggcg    480
   aggacgctta cctaccctgt ctttgtgaag ccggcacggt caggttcgtc ctttggcgta    540
   accaaagtaa acagtacgga agaactaaac gctgcgatag aagcagcagg acaatatgat    600
   ggaaaaatct taattgagca agcgatttcg ggctgtgagg tcggctgcgc ggtcatggga    660
   aacgaggatg atttgattgt cggcgaagtg gatcaaatcc ggttgagcca cggtatcttc    720
   cgcatccatc aggaaaacga gccggaaaaa ggctcagaga atgcgatgat tatcgttcca    780
   gcagacattc cggtcgagga acgaaatcgg gtgcaagaaa cggcaaagaa agtatatcgg    840
   gtgcttggat gcagagggct tgctcgtgtt gatctttttt tgcaggagga tggcggcatc    900
   gttctaaacg aggtcaatac cctgcccggt tttacatcgt acagccgcta tccacgcatg    960
   gcggctgccg caggaatcac gcttcccgca ctaattgaca gcctgattac attggcgata   1020
   gagaggtga                                                           1029
<210> 117
   <211> 1031
   <212> DNA
   <213> Enterococcus spp.
<400> 117
   atgaaaaaaa ttgccgtttt atttggaggg aattctccag aatactcagt gtcactaacc     60
   tcagcagcaa gtgtgatcca agctattgac ccgctgaaat atgaagtaat gaccattggc    120
   atcgcaccaa caatggattg gtattggtat caaggaaacc tcgcgaatgt tcgcaatgat     180
   acttggctag aagatcacaa aaactgtcac cagctgactt tttctagcca aggatttata     240
   ttaggagaaa aacgaatcgt ccctgatgtc ctctttccag tcttgcatgg gaagtatggc     300
   gaggatggct gtatccaagg actgcttgaa ctaatgaacc tgccttatgt tggttgccat     360
   gtcgctgcct ccgcattatg tatgaacaaa tggctcttgc atcaacttgc tgataccatg     420
   ggaatcgcta gtgctcccac tttgctttta tcccgctatg aaaacgatcc tgccacaatc     480
   gatcgtttta ttcaagacca tggattcccg atctttatca agccgaatga agccggttct     540
   tcaaaaggga tcacaaaagt aactgacaaa acagcgctcc aatctgcatt aacgactgct     600
   tttgcttacg gttctactgt gttgatccaa aaggcgatag cgggtattga aattggctgc     660
   ggcatcttag gaaatgagca attgacgatt ggtgcttgtg atgcgatttc tcttgtcgac     720
   ggtttttttg attttgaaga gaaataccaa ttaatcagcg ccacgatcac tgtcccagca     780
   ccattgcctc tcgcgcttga atcacagatc aaggagcagg cacagctgct ttatcgaaac     840
   ttgggattga cgggtctggc tcgaatcgat tttttcgtca ccaatcaagg agcgatttat     900
   ttaaacgaaa tcaacaccat gccgggattt actgggcact cccgctaccc agctatgatg     960
   gcggaagtcg ggttatccta cgaaatatta gtagagcaat tgattgcact ggcagaggag    1020
   gacaaacgat g                                                         1031
<210> 118
   <211> 809
   <212> DNA
   <213> Abiotrophia adiacens
<400> 118
   tggtgctatc ttagtagtat ctgcagctga tggtccaatg cctcaaacac gtgaacacat      60
   cttattatca cgtcaagtag gtgttcctta catcgttgta ttcttaaaca aagttgacat     120
   ggttgacgat gaagaattat tagaattagt agaaatggaa gttcgtgact tattatcaga     180
   atacgatttc ccaggcgatg acactccagt tgttgcaggt tctgctttac gcgctttaga     240
   aggcgacgct tcatacraag aaaaaatctt agaattaatg gctgctgttg acgaatacat     300
   tccaactcca gaacgygacg ttgacaaacc attcatgatg ccagttgaag acgtgttctc     360
   aatcacaggt cgtggtactg ttgctacagg tcgtgttgaa cgtggacaag ttcgtgttgg     420
   tgacgaagtt gaaatcgttg gtatttcaga agaaacttca aaaacaactg taactggtgt     480
   tgaaatgttc cgtaaattgt tagactacgc tgaagcaggg gataacattg gtacattatt     540
   acgtggtgtt acacgtgaca acatcgaacg tggacaagtt cttgctaaac caggaacaat 600
   cactccacat actaaattca aagctgaagt ttacgtatta actaaagaag aaggtggacg 660
   tcatactcca ttcttctcta actaccgtcc tcaattctac ttccgtacaa cagacatcac 720
   tggtgtttgt gtgttaccag aaggcgttga aatggtaatg cctggtgata acgtaactat 780
   ggaagttgaa ttaattcacc cagtagcga 809
<210> 119
   <211> 817
   <212> DNA
   <213> Abiotrophia defectiva
<400> 119
   cggcgcgatc ctcgttgtat ctgctgctga cggcccaatg ccacaaactc gtgaacacat 60
   cctcttgtct cgtcaagttg gtgttcctta catcgtagta ttcttgaaca aagttgacat 120
   ggttgacgac gaagaattgc tcgaattagt tgaaatggaa gttcgtgacc tcttgtctga 180
   atacgacttc ccaggcgacg acactccagt tatcgctggt tcagctttga aagctttaga 240
   aggcgacgct aactacgaag ctaaagtttt agaattgatg gaacaagttg atgcttacat 300
   tccagaacca gaacgtgaca ctgacaagcc attcatgatg ccagtcgaag acgtattctc 360
   tatcactggt cgtggtactg ttgcaactgg tcgtgttgaa cgtggtcaag ttcgcgttgg 420
   tgacgaagtt gaaatcgttg gtatcgaaga agaaacttct aagactaccg ttaccggtgt 480
   tgaaatgttc cgtaagttat tggattacgc tgaagctggg gacaacgttg gtaccttgtt 540
   acgtggtgta actcgtgacc aaatccaacg tggtcaagta ttatctaaac caggttcaat 600
   cactccgyac actaagttcg aagctgaagt gtacgtattg tctaaagaag aaggtggtcg 660
   tcacactcca ttcttctcta actaccgtcc acaattctac ttccgtacaa ctgacgtaac 720
   tggtgttgtt actttaccag aaggtactga aatggttatg ccaggcgaca acgtacaaat 780
   ggttgttgaa ttgatccacc caatcgcgat cgaagaa 817
<210> 120
   <211> 754
   <212> DNA
   <213> Candida albicans
<400> 120
   ctctgtcaaa tgggacaaaa acagatttga agaaatcatc aaggaaacct ccaacttcgt 60
   caagaaggtt ggttacaacc caaagactgt tccattcgtt ccaatctctg gttggaatgg 120
   tgacaacwtg attgaascat ccaccaactg tccatggtac aagggttggg aaaaggaaac    180
   caaatccggt aaagttactg gtaagacctt gttagaagct attgacgcta ttgaaccacc    240
   aaccagacca accgacaaac cattgagatt gccattrcaa gatgtttaca agatcggtgg    300
   tattggtact gtgccagtcg gtagagttga aactggtatc atcaaagccg gtatggtwgt    360
   tactttcgcc ccagctggtg ttaccactga agtcaartcc gttgaaatgc atcacgaaca    420
   attggctgaa ggtgttccag gtgacaatgt trgtttcaac gttaagaacr tttccgttaa    480
   agaaattaga agaggtaacg tttgtggtga ctccaagaac gatccaccaa agggttgtga    540
   ctctttcaat gcccaagtca ttgttttgaa ccatccaggt caaatctctg ctggttactc    600
   tccagtcttg gattgtcacr ctgcccacat tgcttgtaaa ttcgacrctt tggttgaaaa    660
   gattgacaga agaactggta agraattgga agaaaatcca aaattcgtca aatccggtga    720
   tgctgctatc gtcaagatgg tcccaaccaa acca                                754
<210> 121
   <211> 753
   <212> DNA
   <213> Candida glabrata
<400> 121
   tctgtcaagt gggatgaatc cagattcgct gaaatcgtta aggaaacctc caacttcatc     60
   aagaaggtcg gttacaaccc aaagactgtt ccattcgtcc caatctctgg ttggaacggt    120
   gacaacatga ttgaagccac caccaacgct tcctggtaca agggttggga aaaggaaacc    180
   aaggctggtg tcgtcaaggg taagaccttg ttggaagcca ttgacgctat cgaaccacca    240
   accagaccaa ctgacaagcc attgagattg ccattgcaag atgtctacaa gatcggtggt    300
   atcggtacgg tgccagtcgg tagagtcgaa accggtgtca tcaagccagg tatggttgtt    360
   accttcgccc cagctggtgt taccactgaa gtcaagtccg ttgaaatgca ccacgaacaa    420
   ttgactgaag gtttgccagg tgacaacgtt ggtttcaacg ttaagaacgt ttccgttaag    480
   gaaatcagaa gaggtaatgt ctgtggtgac tccaagaacg acccaccaaa ggctgctgct    540
   tctttcaacg ctaccgtcat tgtcttgaac cacccaggtc aaatctctgc tggttactct    600
   ccagttttgg actgtcacac cgcccacatt gcttgtaagt tcgaagaatt gttggaaaag    660
   aacgacagaa gatccggtaa gaagttggaa gactctccaa agttcttgaa gtccggtgac    720
   gctgctttgg ttaagttcgt tccatccaag cca                                 753
<210> 122
   <211> 752
   <212> DNA
   <213> Candida krusei
<400> 122
   ccgttaagtg ggatgaaaac agatttgaag aaattgtcaa ggaaacccaa aacttcatca      60
   agaaggttgg ttacaaccca aagactgttc cattcgttcc aatctctggt tggaatggtg     120
   acaacatgat tgaagcatcc accaactgtc catggtacaa gggttggact aaggaaacca     180
   aggcaggtgt tgttaagggt aagaccttat tagaagcaat cgatgctatt gaaccacctg     240
   tcagaccaac cgaaaagcca ttaagattac cattacaaga tgtttacaag attggtggta     300
   ttggtactgt gccagtcggt agagtcgaaa ccggtgtcat taagccaggt atggttgtca     360
   cttttgctcc agcaggtgtc accaccgaag tcaaatccgt tgaaatgcac catgaacaat     420
   tagaacaagg tgttccaggt gataacgttg gtttcaacgt taagaacgty tctgtcaagg     480
   atatcaagag aggtaacgtt tgtggtgact ccaagaacga cccaccaatg ggtgcagctt     540
   ctttcaatgc tcaagtcatt gtcttgaacc accctggtca aatttccgct ggttactctc     600
   cagtcttgga ttgtcacact gcccacattg catgtaagtt cgacgaatta atcgaaaaga     660
   ttgacagaag aactggtaag tctgttgaag accatccaaa gtcygtcaag tctggtgatg     720
   cagctatcgt caagatggtc ccaaccaagc ca                                   752
<210> 123
   <211> 754
   <212> DNA
   <213> Candida parapsilosis
<400> 123
   ctcagtcaaa tgggacaaga rcagatacga agaaattgtc aaggaaactt ccaacttcgt      60
   caagaaggtt ggttacaacc ctaaagctgt cccattcgtc ccaatctctg gttggaacgg     120
   tgacaatatg attgaaccat caaccaactg tccatggtac aagggttggg aaaaggaaac     180
   taaagctggt aaggttaccg gtaagacctt gttggaagct atcgatgcta tcgarccacc     240
   aaccagacca actgacaagc cattgagatt gccattgcaa gatgtctaca agattggtgg     300
   tattggaact gtgccagttg gtagagttga aaccggtatc atcaaggctg gtatggttgt     360
   tacttttgcc ccagctggtg ttaccactga agtcaagtcc gttgaaatgc accacgaaca     420
   attgactgaa ggtgtcccag gtgacaatgt tggtttcaac gtcaagaacg tttcagttaa     480
   ggaaatcaga agaggtaacg tytgtggtga ctccaagaac gatccaccaa agggatgtga     540
   ytccttcaat gctcaagtta ttgtcttgaa ccacccaggt caaatctctg ctggttactc     600
   accagtcttg gattgtcaca ctgcccacat tgcttgtaaa ttcgacactt tgattgaaaa     660
   gattgacaga agaaccggta agaaattgga agwtgaacca aaattcatca agtccggtga     720
   tgctgcyatc gtcaagatgg tcccaaccaa gcca                                 754
<210> 124
   <211> 753
   <212> DNA
   <213> Candida tropicalis
<400> 124
   tctgttaaat gggacaaraa cagatttgaa gaaattatca aggaaacytc taacttcgtc      60
   aagaaggttg gttacaaccc taaggctgtt ccattcgttc caatctcwgg ttggaatggt     120
   gacaacatga ttgaagcttc taccaactgt ccatggtaca agggttggga aaaagaaacc     180
   aaggctggta aggttaccgg taagactttg ttggaagcca ttgatgctat tgaaccacct     240
   tcaagaccaa ctgacaagcc attgagattg ccattgcaag atgtttacaa gattggtggt     300
   attggtactg tgccagtcgg tagagttgaa actggtgtca tcaaagccgg tatggttgtt     360
   actttygccc cagctggtgt taccactgaa gtcaaatccg tygaaatgca ccacgaacaa     420
   ttggctgaag gtgtcccagg tgacaatgtt ggtttcaacg ttaagaacgt ttctgttaaa     480
   gaaattagaa gaggtaacgt ttgtggtgac tccaagaacg atccaccaaa gggttgtgac     540
   tctttcaacg ctcaagttat tgtcttgaac cacccaggtc aaatytctgc tggttactct     600
   ccagtcttgg attgtcacac tgctcatatt gcttgtaaat tcgacacctt ggttgaaaag     660
   attgacagaa gaactggtaa gaaattggaa gaaaatccaa aattcgtcaa atccggtgat     720
   gctgctattg tcaagatggt tccaaccaaa cca                                   753
<210> 125
   <211> 814
   <212> DNA
   <213> Corynebacterium accolens
<400> 125
   cggcgctatc ctggttgttg ctgcaaccga tggcccgatg ccgcagaccc gcgagcacgt      60
   tctgcttgct cgccaggttg gcgttcctta catcctcgtt gcactgaaca agtgcgacat     120
   ggttgatgat gaggaaatca tcgagctcgt ggagatggag atctccgagc tgctcgcaga     180
   gcaggactac gatgaggaag ctcctatcgt tcacatctcc gctctgaagg cactcgaggg     240
   tgacgagaag tgggtacagt ccatcgttga cctgatggat gcctgcgaca actccatccc     300
   tgatccggag cgcgctaccg atcagccgtt cttgatgcct atcgaggaca tcttcaccat     360
   taccggccgc ggtaccgttg ttaccggccg tgttgagcgt ggtcgtctga acgtcaacga     420
   ggacgttgag atcatcggta tccaggagaa gtcccagaac accaccgtta ccggtatcga     480
   gatgttccgc aagatgatgg actacaccga ggctggcgac aactgtggtc tgcttctgcg     540
   tggtaccaag cgtgaggacg ttgagcgtgg ccaggttgtt atcaagccgg gcgcttacac     600
   ccctcacacc aagttcgagg gttccgtcta cgtcctgaag aaggaagagg gcggccgcca     660
   caccccgytc atgaacaact accgtcctca gttctacttc cgcaccaccg acgttaccgg     720
   tgttgtgaac ctgcctgagg gcaccgagat ggttatgcct ggcgacaacg ttgagatgtc     780
   tgttgagctc atccagcctg ttgctatgga cgag                                  814
<210> 126
   <211> 814
   <212> DNA
   <213> Corynebacterium diphtheriae
<400> 126
   cggcgcaatc ctcgttgttg ctgccaccga cggcccaatg cctcagaccc gtgagcacgt      60
   tctgctcgct cgccaggtcg gcgttcctta catcctcgtt gctctgaaca agtgcgacat     120
   ggttgatgat gaggaaatca tcgagctcgt cgagatggag atccrtgagc tgctcgctga     180
   gcaggattac gacgaagagg ctccaatcat ccacatctcc gcactgaagg ctcttgaggg     240
   cgacgagaag tggacccagt ccatcatcga cctcatgcag gcttgckatg attccatccc     300
   agacccagag cgtgagaccg acaagccatt cctcatgcct atcgaggaca tcttcaccat     360
   caccggccgc ggtaccgttg ttaccggccg tgttgagcgt ggctccctga aggtcaacga     420
   ggacgtcgag atcatcggta tccgcgagaa kgctaccacc accaccgtta ccggtatcga     480
   gatgttccgt aagcttctcg actacaccga ggctggcgac aactgtggtc tgcttctccg     540
   tggcgttaag cgcgaagacg ttgagcgtgg ccaggttgtt gttaagccag gcgcttacac     600
   ccctcacacc gagttcgagg gctctgtcta cgttctgtcc aaggacgagg gtggccgcca     660
   caccccattc ttcgacaact accgcccaca gttctacttc cgcaccaccg acgttaccgg     720
   tgttgtgaag cttcctgagg gcaccgagat ggtcatgcct ggcgacaacg tcgacatgtc     780
   cgtcaccctg atccagcctg tcgctatgga tgag                                  814
<210> 127
   <211> 814
   <212> DNA
   <213> Corynebacterium genitalium
<400> 127
   cggcgccatc ctggttgttg ctgcaaccga tggcccgatg ccgcagaccc gtgagcacgt      60
   tctgctggct cgccaggttg gcgttccgta catcctagtt gcactgaaca agtgcgacat     120
   ggttgatgat gaggagctgc tggagctcgt cgagatggag gtccgcgagc tgctggctga     180
   gcaggacttc gacgaggaag cacctgttgt tcacatctcc gcactgaagg ccctggaggg     240
   cgacgagaag tgggctaagc agatcctgga gctcatggag gcttgcgaca actccatccc     300
   ggatccggag cgcgagaccg acaagccgtt cctgatgccg gttgrggaca tcttcaccat     360
   taccggccgc ggtaccgttg ttaccggccg tgttgagcgt ggcgtcctga acctgaacga     420
   cgaggtcgag atcctgggca tccgcgagaa gtccaccaag accaccgtta cctccatcga     480
   gatgttcaac aagctgctgg acaccgcaga ggctggcgac aacgccgcac tgctgctgcg     540
   tggcctgaag cgcgaagatg ttgagcgtgg tcagatcgtt gctaagccgg gcgagtacac     600
   cccgcacacc gagttcgagg gctccgtcta cgttctgtcc aaggacgagg gtggccgcca     660
   caccccgttc ttcgacaact accgtccgca gttctatttc cgcaccaccg acgttaccgg     720
   tgttgtgaag ctgccggagg gcaccgagat ggttatgccg ggcgacaacg ttgacatgtc     780
   cgtcaccctg atccagccgg ttgctatgga cgag                                 814
<210> 128
   <211> 814
   <212> DNA
   <213> Corynebacterium jeikeium
<400> 128
   cggcgccatc ctggttgttg ccgcaaccga tggcccgatg ccgcagaccc gcgagcacgt      60
   tctgctggcy cgccaggttg gcgttccgta catcctggtt gcactgaaca agtgtgacat     120
   ggttgacgat gaggagctgc tggagctcgt cgagatggag gtccgcgagc tgctggctga     180
   gcaggacttc gacgaggaag ctccggttgt tcacatctcc gcactgaagg ccctggaggg     240
   cgacgagaag tgggctaacc agattctcga gctgatgcag gcttgcgacg agtctatccc     300
   ggatccggag cgcgagaccg acaagccgtt cctgatgccg gttgwggaca tcttcaccat     360
   taccggtcgc ggtaccgttg ttaccggccg tgttgagcgt ggcatcctga acctgaacga     420
   cgaggttgag atcctgggta tccgcgagaa gtcccagaag accaccgtta cctccatcga     480
   gatgttcaac aagctgctgg acaccgcaga ggctggcrac aacgctgcac tgctgctgcg     540
   tggtctgaag cgcgaggacg ttgagcgtgg ccagatcatc gctaagccgg gcgagtacac 600
   cccgcacacc gagttcgagg gctccgtcta cgttctgtcc aaggacgagg gcggccgcca 660
   caccccgttc ttcgacaact accgtccgca gttctacttc cgcaccaccg acgttaccgg 720
   tgttgtgaag ctgcctgagg gcaccgagat ggttatgccg ggcgacaacg tygacatgtc 780
   cgtcaccctg atccagccgg ttgctatgga cgag 814
<210> 129
   <211> 748
   <212> DNA
   <213> Corynebacterium pseudodiphtheriticum
<400> 129
   cggcgctatc ttggttgttg cagctaccga cggcccaatg ccacagactc gcgagcacgt 60
   tctgctggct cgccaggttg gcgttcctta catcctggtt gcactaaaca agtgcgacat 120
   ggttgacgac gaggaaatcc tcgagctcgt cgagatggag atccgcgaat tgctggctga 180
   ccaggaattc gacgaagaag ctccaatcgt tcacatctcc gcagtcggcg ccttggaagg 240
   cgaagagagg tgggttaacg ccatcgttga actgatggat gcttgtgacg agtcgatccc 300
   tgatccagac cgtgctaccg acaagccatt cctgatgcct atcgaggaca tcttcaccat 360
   taccggtcgt ggcaccgttg ttacgggtcg tgttgagcgt ggttccctga aggtcaacga 420
   agaagtcgag atcatcggca tcaaggaaaa gtcccagaag accaccatca ccggtatcga 480
   aatgttccgc aagatgctgg actacaccga ggccggcgac aacgctggtc tgctgcttcg 540
   cggtaccaag cgtgaagacg ttgagcgtgg acaggttatc gttgctccag gtgcttacag 600
   cacccacaag aagttcgaag gttccgtcta cgttctttcc aaggacgagg gcggccgcca 660
   caccccgttc ttcgacaact accgtcctca gttctacttc cgcaccaccg acgttaccgg 720
   tgttgttacc ctgcctgagg gcaccgag 748
<210> 130
   <211> 813
   <212> DNA
   <213> Corynebacterium striatum
<400> 130
   ggcgctatct tggttgttgc tgcaaccgat ggcccgrtgc cgcagacccg cgagcacgtt 60
   cttctggctc gccaggttgg cgttccttac atcctcgttg cactgaacaa gtgcgacatg 120
   gttgacgacg aggaaattat cgagctcgtc gagatggaga tccgcgaact gctcgcagag 180
   caggactacg atgaggaagc tccgatcgtt cacatctctg ctctgaaggc tcttgagggc    240
   grcgagaagt gggtacaggc tatcgttgac ctgatgcagg cttgcgatga ctccatcccg    300
   gatccggagc gcgagctgga caagccgttc ctgatgccaa tcgaggacat cttcaccatc    360
   accggccgcg gtaccgttgt tactggccgt gttgagcgtg gctccctgaa cgtcaacgag    420
   gacgttgaga tcatcggtat ccaggacarg tccatctcca ccaccgttac cggtatcgag    480
   atgytccgca agatgatgga ctacaccgag gctggcgaca actgtggtct gcttctgcgt    540
   ggtaccaagc gtgaagaggt tgagcgcggc caggttgtta ttaagccggg cgcttacacc    600
   cctcacaccc agttcgaggg ttccgtctac gtcctgaaga aggaagaggg cggccgccac    660
   accccgttca tggacaacta ccgtccgcag ttctacttcc gcaccaccga cgttaccggc    720
   gtcatcaagc tgcctgaggg caccgagatg gttatgcctg gcgacaacgt cgagatgtcy    780
   gtcgagctga tccagccggt cgctatggac gag                                  813
<210> 131
   <211> 817
   <212> DNA
   <213> Enterococcus avium
<400> 131
   cggagctatc ttagtagtat ctgctgctga tggccctatg cctcaaactc gtgaacacat     60
   cttgttatct cgtaacgttg gtgttcctta catcgttgta ttcttaaaca aaatggatat    120
   ggttgacgat gaagaattac ttgaattagt tgaaatggaa gttcgtgact tattaactga    180
   atacgacttc ccaggcgacg acactccagt tatcgcaggt tcagcgttga aagctttaga    240
   aggcgacgct tcatacgaag aaaaaatctt agaattaatg gctgctgttg acgaatatat    300
   cccaacacca gttcgtgata ctgacaaacc attcatgatg ccagtcgaag acgtattctc    360
   aatcactggt cgtggtactg ttgcaactgg tcgtgttgaa cgtggacaag ttcgcgttgg    420
   tgacgaagtt gaaatcgtag gtatcgctga cgaaactgct aaaacaactg ttacaggtgt    480
   tgaaatgttc cgtaaattgt tagactacgc tgaagcaggt gacaacatcg gtgctttgtt    540
   acgtggtgtt gcacgtgaag atatccaacg tggacaagta ttggctaaac cagcttcaat    600
   cactccacat acaaaattct ctgcagaagt ttatgttcta actaaagaag aaggtggacg    660
   tcatactcca ttcttcacta actaccgtcc tcagttctac ttccgtacaa ctgacgtaac    720
   tggtgtagtt gatctaccag aaggtactga aatggtwatg cctggggata acgtaactat    780
   ggaagttgaa ttgatycacc caatygcggt agaagac                              817
<210> 132
   <211> 817
   <212> DNA
   <213> Enterococcus faecalis
<400> 132
   cggagctatc ttagtagttt ctgctgctga tggtcctatg cctcaaacac gtgaacatat      60
   cttattatca cgtaacgttg gtgtaccata catcgttgta ttcttaaaca aaatggatat     120
   ggttgatgac gaagaattat tagaattagt agaaatggaa gttcgtgact tattatcaga     180
   atacgatttc ccaggcgatg atgttccagt tatcgcaggt tctgctttga aagctttaga     240
   aggcgacgag tcttatgaag aaaaaatctt agaattaatg gctgcagttg acgaatatat     300
   cccaactcca gaacgtgata ctgacaaacc attcatgatg ccagtcgaag acgtattctc     360
   aatcactgga cgtggtactg ttgctacagg acgtgttgaa cgtggtgaag ttcgcgttgg     420
   tgacgaagtt gaaatcgttg gtattaaaga cgaaacatct aaaacaacyg ttacaggtgt     480
   tgaaatgttc cgtaaattat tagactacgc tgaagcaggc gacaacmtcg gtgctttatt     540
   acgtggtgta gcacgtgaag atatcgaacg tggacaagta ttagctaaac cagctacaat     600
   cactccacac acaaaattca aagctgaagt atacgtatta tcaaaagaag aaggcggacg     660
   tcacactcca ttcttcacta actaccgtcc tcaattctac ttccgtacaa cagacgttac     720
   tggtgttgta gaattgccag aaggtactga aatggtaatg cctggtgata acgttgctat     780
   ggacgttgaa ttaattcacc caatcgctat cgaagac                              817
<210> 133
   <211> 774
   <212> DNA
   <213> Enterococcus faecium
<400> 133
   cggagctatc ttggtagttt ctgctgctga cggcccaatg cctcaaactc gtgaacacat      60
   cctattgtct cgtcaagttg gtgttcctta catcgttgta ttcttgaaca aagtagacat     120
   ggttgatgac gaagaattac tagaattagt tgaaatggaa gttcgtgacc tattaacaga     180
   atacraattc cctggtgrcg atgttcctgt agttgctgga tcagctttga aagctctaga     240
   aggcgacgct tcatacgaag aaaaaattct tgaattaatg gctgcagttg acgaatacat     300
   cccaactcca gaacgtgaca acgacaaacc attcatgatg ccagttgaag acgtgttctc     360
   aattactgga cgtggtactg ttgctacagg tcgtgttgaa cgtggacaag ttcgcgttgg     420
   tgacgaagtt gaagttgttg gtattgctga agaaacttca aaaacaacag ttactggtgt     480
   tgaaatgttc cgtaaattgt tagacyacgc tgaagctgga gacracattg gtgctttact 540
   acgtggtgtt gcacgtgaag acatccaacg tggacaagtt ttagctaaac caggtacaat 600
   cacacctcrt acaaaattct ctgcagaagt atacgtgttg acaaaagaag aaggtggacg 660
   tcatactcca ttcttcacta actaccgtcc acaattctac ttccgtacaa ctgacgtaac 720
   aggtgttgtt gaattaccag aaggaactga aatggtcatg cccggtgaca acgt 774
<210> 134
   <211> 809
   <212> DNA
   <213> Enterococcus gallinarum
<400> 134
   cggtgcgatc ttagtagtat ctgctgctga cggtcctatg cctcaaactc gtgaacacat 60
   cttgttatca cgtaacgttg gcgtaccata catcgttgtt ttcttgaaca aaatggatat 120
   ggttgaygac gaagaattgc tagaattagt tgaaatggaa gttcgtgacc tattgtctga 180
   atatgacttc ccaggcgacg atgttcctgt aatcgccggt tctgctttga aagctcttga 240
   aggagatcct tcatacgaag aaaaaatcat ggaattgatg gctgcagttg acgaatacgt 300
   tccaactcca gaacgtgata ctgacaaacc attcatgatg ccagtcgaag acgtattctc 360
   aatcactgga cgtggtactg ttgctacagg ccgtgttgaa cgtggacaag ttcgcgttgg 420
   tgatgaagta gaaatcgttg gtattgctga cgaaactgct aaaacaactg taacaggtgt 480
   tgaaatgttc cgtaaattgt tagactatgc tgaagcaggg gataacattg gtgcattgct 540
   acgtggggtt gctcgtgaag acatccaacg tggacaagta ttggctaaag ctggtacaat 600
   cacacctcat acaaaattca aagctgaagt ttatgttttg acaaaagaag aaggtggacg 660
   tcacactcca ttcttcacta actaccgtcc tcagttctac ttccgtacaa ctgacgtaac 720
   tggtgttgtt gaattaccag aaggaactga aatggtgatg cctggcgaca acgtgaccat 780
   cgacgttgaa ttgatrcacc caatcgctc 809
<210> 135
   <211> 823
   <212> DNA
   <213> Gardnerella vaginalis
<400> 135
   tggcgcaatc ctcgtggttg ctgctaccga cggtccaatg gctcagaccc gtgaacacgt 60
   cttgcttgct aagcaggtcg gcgttccaaa aattcttgtt gctttgaaca agtgcgatat 120
   ggttgacgac gaagagctta tcgatctcgt tgaagaagag gtccgtgacc tcctcgaaga    180
   aaacggcttc gatcgcgatt gcccagtcyt ccgtacttcc gcttacggcg ctttgcatga    240
   tgacgctcca gaccacgaca agtgggtaga gaccgtcaag gaactcatga aggctgttga    300
   cgagtacatc ccaaccccaa ctcacgatct tgacaagcca ttcttgatgc caatcgaaga    360
   tgtgttcacc atctccggtc gtggtyccgt tgtcaccggt cgtgttgagc gtggtaagct    420
   cccaatcaac accccagttg agatcgttgg tttgcgcgat acccagacca ccaccgtcac    480
   ctctatcgag accttccaca agcagatgga tgaggcagag gctggcgata acactggtct    540
   tcttctccgc ggtatcaacc gtaccgacgt tgagcgtggt caggttgtgg ctgctccagg    600
   ttctgtgact ccacacacca agttcgaagg cgaagtttac gtcttgacca aggacgaagg    660
   tggccgtcac tcgccattct tctccaacta ccgtccacag ttctacttcc gtaccaccga    720
   tgttactggc gttatcacct tgccagacgg catcgaaatg gttcagccag gcgatcacgc    780
   aaccttcact gttgagttga tccaggctat cgcaatggaa gag                      823
<210> 136
   <211> 817
   <212> DNA
   <213> Listeria innocua
<400> 136
   cggagctatc ttagtagtat ctgctgctga tggcccaatg ccacaaactc gtgaacatat     60
   cttactttca cgtcaagttg gtgttccata catcgttgta ttcatgaaca aatgtgacat    120
   ggttgacgat gaagaattac tagaattagt tgaaatggaa attcgtgatc tattaactga    180
   atatgaattc cctggcgatg acattcctgt aatcaaaggt tcagctctta aagcacttca    240
   aggtgaagct gactgggaag ctaaaattga cgagttaatg gaagctgtag attcttacat    300
   tccaactcca gaacgtgata ctgacaaacc attcatgatg ccagttgagg atgtattctc    360
   aatcactggt cgtggaacag ttgcaactgg acgtgttgaa cgtggacaag ttaaagttgg    420
   tgacgaagta gaagttatcg gtattgaaga agaaagcaaa aaagtagtag taactggagt    480
   agaaatgttc cgtaaattac tagactacgc tgaagctggc gacaacattg gcgcacttct    540
   acgtggtgtt gctcgtgaag atatccaacg tggtcaagta ttagctaaac caggttcgat    600
   tactccacac actaacttca aagctgaaac ttatgtttta actaaagaag aaggtggacg    660
   tcacactcca ttcttcaaca actaccgccc acaattctat ttccgtacta ctgacgtaac    720
   tggtattgtt acacttccag aaggtactga aatggtaatg cctggtgata acattgagct    780
   tgcagttgaa ctaattgcac caatcgctat cgaagac                              817
<210> 137
   <211> 818
   <212> DNA
   <213> Listeria ivanovii
<400> 137
   cggagctatc ttagtagtat ctgctgctga tggtccaatg ccacaaactc gtgaacatat      60
   tcttactttc acgtcaagtt ggtgttccat acatcgttgt attcatgaac aaatgtgaca     120
   tggttgacga tgaagaatta cttgaattag ttgaaatgga aattcgtgat ctattaactg     180
   aatatgaatt ccctggcgac gacattcctg taatcaaagg ttcagctctt aaagcacttc     240
   aaggtgaagc tgattgggaa gctaaaattg acgagttaat ggaagctgta gattcttaca     300
   ttccaactcc agaacgtgat actgacaaac cattcatgat gccagttgag gatgtattct     360
   caatcactgg tcgtggaaca gttgcaactg gacgtgttga acgtggacaa gttaaagttg     420
   gtgacgaagt agaagttatc ggtattgaag aagaaagcaa aaaagtagta gtaactggag     480
   tagaaatgtt ccgtaaatta ctagactacg ctgaagctgg cgacaacatt ggcgcacttc     540
   tacgtggtgt tgctcgtgaa gatatccaac gtggtcaagt attagctaaa ccaggttcga     600
   ttactccaca tactaacttc aaagctgaaa cttatgtttt aactaaagaa gaaggtggac     660
   gtcatactcc attcttcaac aactaccgcc cacaattcta tttccgtact actgacgtaa     720
   ctggtattgt tacacttcca gaaggtactg aaatggtaat gcctggtgat aacattgagc     780
   ttgcagttga actaattgca ccaatcgcta tcgaagac                            818
<210> 138
   <211> 817
   <212> DNA
   <213> Listeria monocytogenes
<400> 138
   cggagctatc ttagtagtat ctgctgctga tggcccaatg ccacaaactc gtgaacatat      60
   cttactttca cgtcaagttg gtgttccata catcgttgta ttcatgaaca aatgtgacat     120
   ggttgacgat gaagaattac tagaattagt tgaaatggaa attcgtgatc tattaactga     180
   atatgaattc cctggcgatg acattcctgt aatcaaaggt tcagctctta aagcacttca     240
   aggtgaagct gactgggaag ctaaaattga cgagttaatg gaagctgtag attcttacat     300
   tccaactccw gaacgtgata ctgacaaacc attcatgatg ccagttgagg atgtattctc     360
   aatcactggt cgtggaacag ttgcaactgg acgtgttgaa cgtggacaag ttaaagttgg     420
   tgacgaagta gaagttatcg gtatcgaaga agaaagcaaa aaagtagtag taactggagt 480
   agaaatgttc cgtaaattac tagactacgc tgaagctggc gacaacattg gcgcacttct 540
   acgtggtgtt gctcgtgaag atatccaacr tggtcaagta ttagctaaac caggttcgat 600
   tactccacac actaacttca aagctgaaac ttatgtttta actaaagaag aaggtggacg 660
   tcacactcca ttcttcaaca actaccgccc acaattctat ttccgtacta ctgacgtaac 720
   tggtattgtt acacttccag aaggtactga aatggtaayg cctggtgata acattgagct 780
   tgcagttgaa ctaattgcac caatcgctat cgaagac 817
<210> 139
   <211> 817
   <212> DNA
   <213> Listeria seeligeri
<400> 139
   cggagctatc ttagtagtat ctgctgctga tggcccaatg ccacaaactc gtgaacatat 60
   cttactttca cgtcaagttg gtgttccata catcgttgta ttcatgaaca aatgtgacat 120
   ggttgacgat gaagaattac ttgaattagt tgaaatggaa attcgtgatc tattaactga 180
   atatgaattc cctggtgatg acattcctgt aatcaaaggt tcagctctta aagcacttca 240
   aggtgaagct gactgggaag ctaaaattga cgagttaatg gaagctgtag attcttacat 300
   tccaactcca gaacgtgata ctgacaaacc attcatgatg ccagttgagg atgtattctc 360
   aatcactggt cgtggaactg ttgcaactgg acgtgttgaa cgtggacaag ttaaagttgg 420
   tgacgaagta gaagttatcg gtattgaaga agaaagcaaa aaagtaatag taactggagt 480
   agaaatgttc cgtaaattac tagactacgc tgaagctggc gacaacattg gcgcacttct 540
   acgtggtgtt gctcgtgaag atatccaacg tggtcaagta ttagctaaac caggttcgat 600
   tactccacat actaacttca aagctgaaac ttatgtttta actaaagaag aaggtggacg 660
   tcacactcca ttcttcaaca actaccgccc acaattctat ttccgtacta ctgacgtaac 720
   tggtattgtt acacttccag aaggtactga aatggtaatg cctggtgata acattgagct 780
   tgcagttgaa ctaattgcac caatcgctat cgaagac 817
<210> 140
   <211> 814
   <212> DNA
   <213> Staphylococcus aureus
<400> 140
   cggtggtatc ttagtagtat ctgctgctga cggtccaatg ccacaaactc gtgaacacat 60
   tcttttatca cgtaacgttg gtgtaccagc attagtagta ttcttaaaca aagttgacat 120
   ggttgacgat gaagaattat tagaattagt agaaatggaa gttcgtgact tattaagcga 180
   atatgacttc ccaggtgacg atgtacctgt aatcgctggt tcagcattar aagctttaga 240
   aggcgatgct caatacgaag aaaaaatctt agaattartg gaagctgtag atacttacat 300
   tccaactcca gaacgtgatt ctgacaaacc attcatgatg ccagttgagg acgtattctc 360
   aatcactggt cgtggtactg ttgctacagg ccgtgttgaa cgtggtcaaa tcaaagttgg 420
   tgaagaagtt gaaatcatcg gtttacatga cacatctaaa acaactgtta caggtgttga 480
   aatgttccgt aaattattag actacgctga agctggtgac aacattggtg cattattacg 540
   tggtgttgct cgtgaagacg tacaacgtgg tcaagtatta gctgctcctg gttcaattac 600
   accacatact gaattcaaag cagaagtata cgtattatca aaagacgaag gtggacgtca 660
   cactccattc ttctcaaact atcgtccaca attctatttc cgtactactg acgtaactgg 720
   tgttgttcac ttaccagaag gtactgaaat ggtaatgcct ggtgataacg ttgaaatgac 780
   agtagaatta atcgctccaa tcgcgattga agac 814
<210> 141
   <211> 814
   <212> DNA
   <213> Staphylococcus epidermidis
<400> 141
   cggcggtatc ttagttgtat ctgctgctga cggtccaatg ccacaaactc gtgaacacat 60
   cttattatca cgtaacgttg gtgtaccagc attagttgta ttcttaaaca aagttgacat 120
   ggtagacgac gaagaattat tagaattagt tgaaatggaa gttcgtgact tattaagcga 180
   atatgacttc ccaggtgacg atgtacctgt aatcgctggt tctgcattaa aagcattaga 240
   aggcgatgct gaatacgaac aaaaaatctt agacttaatg caagcagttg atgattacat 300
   tccaactcca gaacgtgatt ctgacaaacc attcatgatg ccagttgagg acgtattctc 360
   aatcactggt cgtggtactg ttgctacagg ccgtgttgaa cgtggtcaaa tcaaagtwgg 420
   tgaagaagtt gaaatcatcg gtatgcacga aacttctaaa acaactgtta ctggtgtaga 480
   aatgttccgt aaattattag actacgctga agctggtgac aacatcggtg ctttattacg 540
   tggtgttgca cgtgaagacg tacaacgtgg tcaagtatta gctgctcctg gttctattac 600
   accacacaca aaattcaaag ctgaagtata cgtattatct aaagatgaag gtggacgtca 660
   cactccattc ttcactaact atcgcccaca attctatttc crtactactg acgtaactgg 720
   tgttgtaaac ttaccagaag gtacagaaat ggttatgcct ggcgacaacg ttgaaatgac 780
   agttgaatta atcgctccaa tcgctatcga agac 814
<210> 142
   <211> 817
   <212> DNA
   <213> Staphylococcus saprophyticus
<400> 142
   cggagctatc ttagtagtat ctgctgctga tggcccaatg ccacaaactc gtgaacacat 60
   tcttttatca cgtracgttg gtgytccagc attagttgta ttcttaaaca aagttgacat 120
   ggttgacgay gaagaattat tagaattrgt agaaatggaa gttcgtgrct tattaagcga 180
   atatgacttc ccaggtgacg atgtacctgt aatctctggt tctgcattaa aagctttaga 240
   aggcgacgct gactatgagc aaaaaatctt agacttaatg caagctgttg atgactycat 300
   tccaacacca gaacgtgatt ctgacaaacc attcatgatg ccagttgagg acgtattctc 360
   aatcactggt cgtggtactg ttgctacagg ccgtgttgaa cgtggtcaaa tcaaagtcgg 420
   tgaagaaatc garatcatcg gtatgcaaga agaatcaagc aaaacaactg ttactggtgt 480
   agaaatgttc cgtaaattat tagactacgc tgaagctggt gacaacattg gtgcattatt 540
   acgtggtgtt tcacgtgatg atgtacaacg tggtcaagtt ttagctgctc ctggtactat 600
   cacaccacat acaaaattca aagcggatgt ttacgtttta tctaaagatg aaggtggtcg 660
   tcatacgcca ttcttcacta actaccgccc acaattctat ttccgtacta ctgacgtaac 720
   tggtgttgtt aacttaccag aaggtactga aatggttatg cctggcgata acgttgaaat 780
   ggatgttgaa ttaatttctc caatcgctat tgaagac 817
<210> 143
   <211> 817
   <212> DNA
   <213> Staphylococcus simulans
<400> 143
   cggcggtatc ttagtagtat ctgctgcaga tggtccaatg ccacaaactc gtgaacacat 60
   cttattatca cgtaacgttg gtgtaccagc tttagttgta ttcttaaaca aagctgacat 120
   ggttgacgac gaagaattat tagaattagt tgaaatggaa gttcgtgact tattatctga 180
   atacgacttc cctggtgacg atgtaccagt tatcgttggt tctgcattaa aagctttaga 240
   aggcgaccca gaatacgaac aaaaaatctt agacttaatg caagctgtag atgactacat 300
   cccaactcca gaacgtgact ctgataaacc attcatgatg ccagttgagg acgtattctc 360
   aatcactggt cgtggtactg tagcaacagg ccgtgttgaa cgtggtcaaa tcaaagtcgg 420
   tgaagaagtt gaaatcatcg gtatcactga agaaagcaag aaaacaacag ttacaggtgt 480
   agaaatgttc cgtaaattat tagactacgc tgaagctggt gacaacatcg gtgctttatt 540
   acgtggtgtt gcacgtgaag acgtacaacg tggacaagta ttagcagctc ctggctctat 600
   tactccacac acaaaattca aagctgatgt ttacgtttta tctaaagaag aaggtggacg 660
   tcatactcca ttcttcacta actaccgccc acaattctac ttccgtacta ctgacgtaac 720
   tggcgttgtt cacttaccag aaggtactga aatggttatg cctggcgata acgtagaaat 780
   gactgttgaa ttgatcgctc caatcgcgat tgaagac 817
<210> 144
   <211> 817
   <212> DNA
   <213> Streptococcus agalactiae
<400> 144
   cggagctatc cttgtagttg cttcaactga tggaccaatg ccacaaactc gtgagcacat 60
   ccttctttca cgtcaagttg gtgttaaaca ccttatcgta ttcatgaaca aagttgacct 120
   tgttgatgat gaagaattgc ttgaattggt tgaaatggaa attcgtgacc ttctttcaga 180
   atacgacttc ccaggtgatg accttccagt tatccaaggt tcagctctta aagcacttga 240
   aggcgacgaa aaatacgaag acatcatcat ggaattgatg agcactgttg atgagtacat 300
   tccagaacca gaacgtgata ctgacaaacc tttacttctt ccagttgaag atgtattctc 360
   aatcactgga cgtggtacag ttgcttcagg acgtatcgac cgtggtactg ttcgtgtcaa 420
   cgacgaagtt gaaatcgttg gtattaaaga agatatccaa aaagcagttg ttactggtgt 480
   tgaaatgttc cgtaaacaac ttgacgaagg tcttgcaggg gacaacgttg gtgttcttct 540
   tcgtggtgtt caacgtgatg aaatcgaacg tggtcaagtt cttgctaaac caggttcaat 600
   caacccacac actaaattta aaggtgaagt ttacatcctt tctaaagaag aaggtggacg 660
   tcatactcca ttcttcaaca actaccgtcc acaattctac ttccgtacaa ctgacgtaac 720
   aggttcaatc gaacttccag caggaacaga aatggttatg cctggtgata acgttactat 780
   cgaagttgaa ttgattcacc caatcgccgt agaacaa 817
<210> 145
   <211> 817
   <212> DNA
   <213> Streptococcus pneumoniae
<400> 145
   cggagctatc cttgtagtag cttcaactga cggaccaatg ccacaaactc gtgagcacat 60
   ccttctttca cgtcaggttg gtgttaaaca ccttatcgtc ttcatgaaca aagttgactt 120
   ggttgacgac gaagaattgc ttgaattggt tgaaatggaa atccgtgacc tattgtcaga 180
   atacgacttc ccaggtgacg atcttccagt tatccaaggt tcagcactta aagctcttga 240
   aggtgactct aaatacgaag acatcgttat ggaattgatg aacacagttg atgagtatat 300
   cccagaacca gaacgtgaca ctgacaaacc attgcttctt ccagtcgagg acgtattctc 360
   aatcactgga cgtggtacag ttgcttcagg acgtatcgac cgtggtatcg ttaaagtcaa 420
   cgacgaaatc gaaatcgttg gtatcaaaga agaaactcra aaagcagttg ttactggtgt 480
   tgaaatgttc cgtaaacaac ttgacgaagg tcttgctgga gataacgtag gtgtccttct 540
   tcgtggtgtt caacgtgatg aaatcgaacg tggacaagtt atcgctaaac caggttcaat 600
   caacccacac actaaattca aaggtgaagt ctacatcctt actaaagaag aaggtggacg 660
   tcacactcca ttcttcaaca actaccgtcc acaattctac ttccgtacta ctgacgttac 720
   aggttcaatc gaacttccag caggtactga aatggtaatg cctggtgata acgtgacaat 780
   cgacgttgag ttgattcacc caatcgccgt agaacaa 817
<210> 146
   <211> 817
   <212> DNA
   <213> Streptococcus salivarius
<400> 146
   cggtgcgatc cttgtagtag catctactga cggaccaatg ccacaaactc gtgagcacat 60
   ccttctttca cgtcaggttg gtgttaaaca ccttatcgtc ttcatgaaca aagttgactt 120
   ggttgacgat gaagaattgc ttgaattggt tgaaatggaa atccgtgacc ttctttcaga 180
   atacgatttc ccaggtgatg acattccagt tatccaaggt tcagctctta aagctcttga 240
   aggtgattct aaatacgaag acatcatcat ggacttgatg aacactgttg acgaatacat 300
   cccagaacca gaacgtgaca ctgacaaacc attgttgctt ccagtcgaag acgtattctc 360
   aatcactggt cgtggtactg ttgcttcagg acgtatcgac cgtggtgttg ttcgtgtcaa 420
   tgacgaagtt gaaatcgttg gtcttaaaga agacatccaa aaagcagttg ttactggtgt 480
   tgaaatgttc cgtaaacaac ttgacgragg tattgccgga gataacgtcg gtgttcttct 540
   tcgtggtatc caacgtgatg aaatcgaacg tggtcaagta ttggctgcac ctggttcaat 600
   caacccacac actaaattca aaggtgaagt ttacatcctt tctaaagaag aaggtggacg 660
   tcacactcca ttcttcaaca actaccgtcc acagttctac ttccgtacaa ctgacgtaac 720
   aggttcaatc gaacttcctg caggtactga aatggttatg cctggtgata acgtgactat 780
   cgacgttgag ttgatccacc caatcgccgt tgaacaa 817
<210> 147
   <211> 897
   <212> DNA
   <213> Agrobacterium tumefaciens
<400> 147
   aacatgatca ccggtgctgc cgagatggac ggcgcgatcc tggtttgctc ggctgccgac 60
   ggcccgatgc cacagacccg cgagcacatc ctgcttgccc gtcaggtggg cgttccggcc 120
   atcgtcgtgt tcctcaacaa ggtcgaccag gttgacgacg ccgagcttct cgagctcgtc 180
   gagcttgaag ttcgcgaact tctgtcgtcc tacgacttcc cgggcgacga tatcccgatc 240
   atcaagggtt cggcacttgc tgctcttgaa gattctgaca agaagatcgg tgaagacgcg 300
   atccgcgagc tgatggctgc tgtcgacgcc tacatcccga cgcctgagcg tccgatcgac 360
   cagccgttcc tgatgccgat cgaagacgtg ttctcgatct cgggtcgtgg tacggttgtg 420
   acgggtcgcg ttgagcgcgg tatcgtcaag gttggtgaag aagtcgaaat cgtcggcatc 480
   cgtccgacct cgaagacgac tgttaccggc gttgaaatgt tccgcaagct gctcgaccag 540
   ggccaggccg gcgacaacat cggtgcactc gttcgcggcg ttacccgtga cggcgtcgag 600
   cgtggtcaga tcctgtgcaa gccgggttcg gtcaagccgc acaagaagtt catggcagaa 660
   gcctacatcc tgacgaagga agaaggcggc cgtcatacgc cgttcttcac gaactaccgt 720
   ccgcagttct acttccgtac gactgacgtt accggtatcg tttcgcttcc tgaaggcacg 780
   gaaatggtta tgcctggcga caacgtcact gttgaagtcg agctgatcgt tccgatcgcg 840
   atggaagaaa agctgcgctt cgctatccgc gaaggcggcc gtaccgtcgg cgccggc 897
<210> 148
   <211> 885
   <212> DNA
   <213> Bacillus subtilis
<400> 148
   atgatcactg gtgctgcgca aatggacgga gctatccttg tagtatctgc tgctgatggc 60
   ccaatgccac aaactcgtga gcacatcctt ctttctaaaa acgttggtgt accatacatc 120
   gttgtattct taaacaaatg cgacatggta gacgacgaag agcttcttga actagttgaa 180
   atggaagttc gcgatcttct tagcgaatac gacttccctg gtgatgatgt accagttgtt 240
   aaaggttctg ctcttaaagc tcttgaagga gacgctgagt gggaagctaa aatcttcgaa 300
   cttatggatg cggttgatga gtacatccca actccagaac gcgacactga aaaaccattc 360
   atgatgccag ttgaggacgt attctcaatc actggtcgtg gtacagttgc tactggccgt 420
   gtagaacgcg gacaagttaa agtcggtgac gaagttgaaa tcatcggtct tcaagaagag 480
   aacaagaaaa caactgttac aggtgttgaa atgttccgta agcttcttga ttacgctgaa 540
   gctggtgaca acattggtgc ccttcttcgc ggtgtatctc gtgaagaaat ccaacgtggt 600
   caagtacttg ctaaaccagg tacaatcact ccacacagca aattcaaagc tgaagtttac 660
   gttctttcta aagaagaggg tggacgtcat actccattct tctctaacta ccgtcctcag 720
   ttctacttcc gtacaactga cgtaactggt atcatccatc ttccagaagg cgtagaaatg 780
   gttatgcctg gagataacac tgaaatgaac gttgaactta tttctacaat cgctatcgaa 840
   gaaggaactc gtttctctat tcgtgaaggc ggacgtactg ttggt 885
<210> 149
   <211> 882
   <212> DNA
   <213> Bacteroides fragilis
<400> 149
   atggttactg gtgctgctca gatggacggt gctatcattg tagttgctgc tactgatggt 60
   ccgatgcctc agactcgtga gcacatcctt ttggctcgtc aggtaaacgt tccgaagctg 120
   gttgtattca tgaacaagtg cgatatggtt gaagatgctg agatgttgga acttgttgaa 180
   atggaaatga gagaattgct ttcattctat gatttcgacg gtgacaatac tccgatcatt 240
   cagggttctg ctcttggtgc attgaacggc gtagaaaaat gggaagacaa agtaatggaa 300
   ctgatggaag ctgttgatac ttggattcca ctgcctccgc gcgatgttga taaacctttc 360
   ttgatgccgg tagaagacgt gttctctatc acaggtcgtg gtactgtagc tacaggtcgt 420
   atcgaaactg gtgttatcca tgtaggtgat gaaatcgaaa tcctcggttt gggtgaagat 480
   aagaaatcag ttgtaacagg tgttgaaatg ttccgcaaac ttctggatca gggtgaagct 540
   ggtgacaacg taggtctgtt gcttcgtggt gttgacaaga acgaaatcaa acgtggtatg 600
   gttctttgta aaccgggtca gattaaacct cactctaaat tcaaagcaga ggtttatatc 660
   ctgaagaaag aagaaggtgg tcgtcacact ccattccata acaaatatcg tcctcagttc 720
   tacctgcgta ctatggactg tacaggtgaa atcactcttc cggaaggaac tgaaatggta 780
   atgccgggtg ataacgtaac tatcactgta gagttgatct atccggttgc actgaacatc 840
   ggtcttcgtt tcgctatccg cgaaggtgga cgtacagtag gt 882
<210> 150
   <211> 888
   <212> DNA
   <213> Borrelia burgdorferi
<400> 150
   aatatgatta caggagcagc tcaaatggat gcagcgatac ttttagttgc tgctgatagt 60
   ggtgctgagc ctcaaacaaa agagcatttg cttcttgctc aaagaatggg aataaagaaa 120
   ataatagttt ttttaaataa attggactta gcagatcctg aacttgttga gcttgttgaa 180
   gttgaagttt tagaacttgt tgaaaaatat ggcttttcag ctgatactcc aataatcaaa 240
   ggttcagctt ttggggctat gtcaaatcca gaagatcctg aatctacaaa atgcgttaaa 300
   gaacttcttg aatctatgga taattatttt gatcttccag aaagagatat tgacaagcca 360
   tttttgcttg ctgttgaaga tgtattttct atttcaggaa gaggcactgt tgctactggg 420
   cgtattgaaa gaggtattat taaagttggt caagaagttg aaatagttgg aattaaagaa 480
   accagaaaaa ctactgttac tggtgttgaa atgttccaga aaattcttga gcaaggtcaa 540
   gcaggggata atgttggtct tcttttgaga ggcgttgata aaaaagacat tgagaggggg 600
   caagttttgt cagctccagg tacaattact ccacacaaga aatttaaagc ttcaatttat 660
   tgtttgacta aagaagaagg cggtaggcac aagccatttt tcccagggta tagaccacag 720
   ttctttttta gaacaaccga tgttactgga gttgttgctt tagagggcaa agaaatggtt 780
   atgcctggtg ataatgttga tattattgtt gagctgatct cttcaatagc tatggataag 840
   aatgtagaat ttgctgttcg agaaggtgga agaaccgttg cttcagga 888
<210> 151
   <211> 894
   <212> DNA
   <213> Brevibacterium linens
<400> 151
   aacatgatca ccggtgccgc tcagatggac ggtgcgatcc tcgtcgtcgc cgctaccgac 60
   ggaccgatgc cccagacccg tgagcacgtg ctgctcgcgc gtcaggtcgg cgttccctac 120
   atcgtcgtgg ctctgaacaa gtccgacatg gtcgatgacg aggagctcct cgagctcgtc 180
   gaattcgagg tccgcgacct gctctcgagc caggacttcg acggagacaa cgctccggtc     240
   attccggtgt ccgctctcaa ggcgctggaa ggcgacgaga agtgggtcaa gagcgttcag     300
   gatctcatgg ctgccgtcga tgacaacgtt ccggagccgg agcgcgatgt cgacaagccg     360
   ttcctcatgc ccgtcgagga cgtcttcacg atcaccggtc gtggaaccgt cgtcaccggt     420
   cgtgtcgagc gcggcgtgct cctgcctaac gacgaaatcg aaatcgtcgg catcaaggag     480
   aagtcgtcca agacgactgt caccgctatc gagatgttcc gcaagaccct gccggatgcc     540
   cgtgcaggtg agaacgtcgg tctgctcctc cgcggcacca agcgcgagga tgttgagcgc     600
   ggtcaggtca tcgtgaagcc gggttcgatc accccgcaca ccaagttcga ggctcaggtc     660
   tacatcctga gcaaggacga gggcggacgt cacaacccgt tctactcgaa ctaccgtccg     720
   cagttctact tccggaccac ggacgtcacc ggtgtcatca cgctgcccga gggcaccgag     780
   atggtcatgc ccggcgacaa caccgatatg tcggtcgagc tcatccagcc gatcgctatg     840
   gaggaccgcc tccgcttcgc aatccgcgaa ggtggccgca ccgtcggcgc cggt           894
<210> 152
   <211> 888
   <212> DNA
   <213> Burkholderia cepacia
<400> 152
   atgatcacgg gcgcagcgca gatggacggc gcgatcctgg tttgctcggc agcagacggc      60
   ccgatgccgc aaacgcgtga gcacatcctg ctggcgcgtc aggttggtgt tccgtacatc     120
   atcgtgttcc tgaacaagtg cgacagtgtg gacgacgctg aactgctcga gctggtcgag     180
   atggaagttc gcgaactcct gtcgaagtac gacttcccgg gcgacgacac gccgatcgtg     240
   aagggttcgg ccaagctggc gctggaaggc gacacgggcg agctgggcga agtggcgatc     300
   atgagcctgg cagacgcgct ggacacgtac atcccgacgc cggagcgtgc agttgacggc     360
   gcgttcctga tgccggtgga agacgtgttc tcgatctcgg gccgtggtac ggtggtgacg     420
   ggtcgtgtcg agcgcggcat cgtgaaggtc ggcgaagaaa tcgaaatcgt cggtatcaag     480
   ccgacggtga agacgacctg cacgggcgtt gaaatgttcc gcaagctgct ggaccaaggt     540
   caggcaggcg acaacgtcgg tatcctgctg cgcggcacga agcgtgaaga cgtggagcgt     600
   ggccaggttc tggcgaagcc gggttcgatc acgccgcaca cgcacttcac ggctgaagtg     660
   tacgtgctga gcaaggacga aggcggccgt cacacgccgt tcttcaacaa ctaccgtccg     720
   cagttctact tccgtacgac ggacgtgacg ggctcgatcg agctgccgaa ggacaaggaa     780
   atggtgatgc cgggcgacaa cgtgtcgatc acggtgaagc tgattgctcc gatcgcgatg 840
   gaagaaggtc tgcgcttcgc aatccgtgaa ggcggccgta cggtcggc 888
<210> 153
   <211> 891
   <212> DNA
   <213> Chlamydia trachomatis
<400> 153
   aacatgatca ccggtgcggc tcaaatggac ggggctattc tagtagtttc tgcaacagac 60
   ggagctatgc ctcaaactaa agagcatatt cttttggcaa gacaagttgg ggttccttac 120
   atcgttgttt ttctcaataa aattgacatg atttccgaag aagacgctga attggtcgac 180
   ttggttgaga tggagttggc tgagcttctt gaagagaaag gatacaaagg gtgtccaatc 240
   atcagaggtt ctgctctgaa agctttggaa ggagatgctg catacataga gaaagttcga 300
   gagctaatgc aagccgtcga tgataatatc cctactccag aaagagaaat tgacaagcct 360
   ttcttaatgc ctattgagga cgtgttctct atctccggac gaggaactgt agtaactgga 420
   cgtattgagc gtggaattgt taaagtttcc gataaagttc agttggtcgg tcttagagat 480
   actaaagaaa cgattgttac tggggttgaa atgttcagaa aagaactccc agaaggtcgt 540
   gcaggagaga acgttggatt gctcctcaga ggtattggta agaacgatgt ggaaagagga 600
   atggttgttt gcttgccaaa cagtgttaaa cctcatacac agtttaagtg tgctgtttac 660
   gttctgcaaa aagaagaagg tggacgacat aagcctttct tcacaggata tagacctcaa 720
   ttcttcttcc gtacaacaga cgttacaggt gtggtaactc tgcctgaggg agttgagatg 780
   gtcatgcctg gggataacgt tgagtttgaa gtgcaattga ttagccctgt ggctttagaa 840
   gaaggtatga gatttgcgat tcgtgaaggt ggtcgtacaa tcggtgctgg a 891
<210> 154
   <211> 891
   <212> DNA
   <213> Escherichia coli
<400> 154
   aacatgatca ccggtgctgc gcagatggac ggcgcgatcc tggtagttgc tgcgactgac 60
   ggcccgatgc cgcagactcg tgagcacatc ctgctgggtc gtcaggtagg cgttccgtac 120
   atcatcgtgt tcctgaacaa atgcgacatg gttgatgacg aagagctgct ggaactggtt 180
   gaaatggaag ttcgtgaact tctgtctcag tacgacttcc cgggcgacga cactccgatc 240
   gttcgtggtt ctgctctgaa agcgctggaa ggcgacgcag agtgggaagc gaaaatcctg 300
   gaactggctg gcttcctgga ttcttacatt ccggaaccag agcgtgcgat tgacaagccg 360
   ttcctgctgc cgatcgaaga cgtattctcc atctccggtc gtggtaccgt tgttaccggt 420
   cgtgtagaac gcggtatcat caaagttggt gaagaagttg aaatcgttgg tatcaaagag 480
   actcagaagt ctacctgtac tggcgttgaa atgttccgca aactgctgga cgaaggccgt 540
   gctggtgaga acgtaggtgt tctgctgcgt ggtatcaaac gtgaagaaat cgaacgtggt 600
   caggtactgg ctaagccggg caccatcaag ccgcacacca agttcgaatc tgaagtgtac 660
   attctgtcca aagatgaagg cggccgtcat actccgttct tcaaaggcta ccgtccgcag 720
   ttctacttcc gtactactga cgtgactggt accatcgaac tgccggaagg cgtagagatg 780
   gtaatgccgg gcgacaacat caaaatggtt gttaccctga tccacccgat cgcgatggac 840
   gacggtctgc gtttcgcaat ccgtgaaggc ggccgtaccg ttggcgcggg c 891
<210> 155
   <211> 891
   <212> DNA
   <213> Fibrobacter succinogenes
<400> 155
   aacatggtga ctggtgctgc tcagatggac ggcgctatcc tcgttgttgc cgctactgac 60
   ggtccgatgc cgcagactcg cgaacacatc cttctcgctc accaggttgg cgtgccgaag 120
   atcgtcgtgt tcatgaacaa gtgcgacatg gttgacgatg ctgaaattct cgacctcgtc 180
   gaaatggaag ttcgcgaact cctctccaag tatgacttcg acggtgacaa caccccgatc 240
   atccgtggtt ccgctctcaa ggccctcgaa ggcgatccgg aataccagga caaggtcatg 300
   gaactcatga acgcttgcga cgaatacatc ccgctcccgc agcgcgatac cgacaagccg 360
   ttcctcatgc cgatcgaaga cgtgttcacg attactggcc gcggcactgt cgctactggc 420
   cgtatcgaac gcggtgtcgt tcgcttgaac gacaaggttg aacgtatcgg tctcggtgaa 480
   accaccgaat acgtcatcac cggtgttgaa atgttccgta agctcctcga cgacgctcag 540
   gcaggtgaca acgttggtct cctcctccgt ggtgctgaaa agaaggacat cgtccgtggc 600
   atggttctcg cagctccgaa gtctgtcact ccgcacaccg aatttaaggc tgaaatctac 660
   gttctcacga aggacgaagg tggccgtcac acgccgttca tgaatggcta ccgtccgcag 720
   ttctacttcc gcaccaccga cgttactggt acgatccagc tcccggaagg tgtcgaaatg 780
   gttactccgg gtgacacggt cacgatccac gtgaacctca tcgctccgat cgctatggaa 840
   aagcagctcc gcttcgctat ccgtgaaggt ggacgtactg ttggtgctgg c 891
<210> 156
   <211> 894
   <212> DNA
   <213> Flavobacterium ferrugineum
<400> 156
   aacatgatca ccggtgctgc ccagatggac ggtgctatct tagttgtggc tgcatcagac      60
   ggtcctatgc ctcaaacaaa agaacacatc ctgcttgctg cccaggtagg tgtacctaaa     120
   atggttgtgt ttctgaataa agttgacctc gttgacgacg aagagctcct ggagctggtt     180
   gagatcgagg ttcgcgaaga actgactaaa cgcggtttcg acggcgacaa cactccaatc     240
   atcaaaggtt ccgctacagg cgccctcgct ggtgaagaaa agtgggttaa agaaattgaa     300
   aacctgatgg acgctgttga cagctacatc ccactgcctc ctcgtccggt tgatctgccg     360
   ttcctgatga gcgtagagga cgtattctct atcactggtc gtggtactgt tgctaccggt     420
   cgtatcgagc gtggccgtat caaagttggt gagcctgttg agatcgtagg tctgcaggag     480
   tctcccctga actctaccgt tacaggtgtt gagatgttcc gcaaactcct cgacgaaggt     540
   gaagctggtg ataacgccgg tctcctcctc cgtggtgttg aaaaaacaca gatccgtcgc     600
   ggtatggtaa tcgttaaacc cggttccatc actccgcaca cggacttcaa aggcgaagtt     660
   tacgtactga gcaaagacga aggtggccgt cacactccat tcttcaacaa ataccgtcct     720
   caattctact tccgtacaac tgacgttaca ggtgaagtag aactgaacgc aggaacagaa     780
   atggttatgc ctggtgataa caccaacctg accgttaaac tgatccaacc gatcgctatg     840
   gaaaaaggtc tgaaattcgc gatccgcgaa ggtggccgta ccgtaggtgc agga           894
<210> 157
   <211> 891
   <212> DNA
   <213> Haemophilus influenzae
<400> 157
   aatatgatta ctggtgcggc acaaatggat ggtgctattt tagtagtagc agcaacagat      60
   ggtcctatgc cacaaactcg tgaacacatc ttattaggtc gccaagtagg tgttccatac     120
   atcatcgtat tcttaaacaa atgcgacatg gtagatgacg aagagttatt agaattagtc     180
   gaaatggaag ttcgtgaact tctatctcaa tatgacttcc caggtgacga tacaccaatc     240
   gtacgtggtt cagcattaca agcgttaaac ggcgtagcag aatgggaaga aaaaatcctt     300
   gagttagcaa accacttaga tacttacatc ccagaaccag aacgtgcgat tgaccaaccg     360
   ttccttcttc caatcgaaga tgtgttctca atctcaggtc gtggtactgt agtaacaggt 420
   cgtgtagaac gaggtattat ccgtacaggt gatgaagtag aaatcgtcgg tatcaaagat 480
   acagcgaaaa ctactgtaac gggtgttgaa atgttccgta aattacttga cgaaggtcgt 540
   gcaggtgaaa acatcggtgc attattacgt ggtaccaaac gtgaagaaat cgaacgtggt 600
   caagtattag cgaaaccagg ttcaatcaca ccacacactg acttcgaatc agaagtgtac 660
   gtattatcaa aagatgaagg tggtcgtcat actccattct tcaaaggtta ccgtccacaa 720
   ttctatttcc gtacaacaga cgtgactggt acaatcgaat taccagaagg cgtggaaatg 780
   gtaatgccag gcgataacat caagatgaca gtaagcttaa tccacccaat tgcgatggat 840
   caaggtttac gtttcgcaat ccgtgaaggt ggccgtacag taggtgcagg c 891
<210> 158
   <211> 906
   <212> DNA
   <213> Helicobacter pylori
<400> 158
   aacatgatca ccggtgcggc gcaaatggac ggagcgattt tggttgtttc tgcagctgat 60
   ggccctatgc ctcaaactag ggagcatatc ttattgtctc gtcaagtagg cgtgcctcac 120
   atcgttgttt tcttaaacaa acaagacatg gtagatgacc aagaattgtt agaacttgta 180
   gaaatggaag tgcgcgaatt gttgagcgcg tatgaatttc ctggcgatga cactcctatc 240
   gtagcgggtt cagctttaag agctttagaa gaagcaaagg ctggtaatgt gggtgaatgg 300
   ggtgaaaaag tgcttaaact tatggctgaa gtggatgcct atatccctac tccagaaaga 360
   gacactgaaa aaactttctt gatgccggtt gaagatgtgt tctctattgc gggtagaggg 420
   actgtggtta caggtaggat tgaaagaggc gtggtgaaag taggcgatga agtggaaatc 480
   gttggtatca gacctacaca aaaaacgact gtaaccggtg tagaaatgtt taggaaagag 540
   ttggaaaaag gtgaagccgg cgataatgtg ggcgtgcttt tgagaggaac taaaaaagaa 600
   gaagtggaac gcggtatggt tctatgcaaa ccaggttcta tcactccgca caagaaattt 660
   gagggagaaa tttatgtcct ttctaaagaa gaaggcggga gacacactcc attcttcacc 720
   aattaccgcc cgcaattcta tgtgcgcaca actgatgtga ctggctctat cacccttcct 780
   gaaggcgtag aaatggttat gcctggcgat aatgtgaaaa tcactgtaga gttgattagc 840
   cctgttgcgt tagagttggg aactaaattt gcgattcgtg aaggcggtag gaccgttggt 900
   gctggt 906
<210> 159
   <211> 891
   <212> DNA
   <213> Micrococcus luteus
<400> 159
   aacatgatca ccggcgccgc tcagatggac ggcgcgatcc tcgtggtcgc cgctaccgac      60
   ggcccgatgg cccagacccg tgagcacgtg ctcctggccc gccaggtcgg cgtgccggcc     120
   ctgctcgtgg ccctgaacaa gtcggacatg gtggaggacg aggagctcct cgagcgtgtc     180
   gagatggagg tccggcagct gctgtcctcc aggagcttcg acgtcgacga ggccccggtc     240
   atccgcacct ccgctctgaa ggccctcgag ggcgaccccc agtgggtcaa gtccgtcgag     300
   gacctcatgg atgccgtgga cgagtacatc ccggacccgg tgcgcgacaa ggacaagccg     360
   ttcctgatgc cgatcgagga cgtcttcacg atcaccggcc gtggcaccgt ggtgaccggt     420
   cgcgccgagc gcggcaccct gaagatcaac tccgaggtcg agatcgtcgg catccgcgac     480
   gtgcagaaga ccactgtcac cggcatcgag atgttccaca agcagctcga cgaggcctgg     540
   gccggcgaga actgcggtct gctcgtgcgc ggtctgaagc gcgacgacgt cgagcgcggc     600
   caggtgctgg tggagccggg ctccatcacc ccgcacacca acttcgaggc gaacgtctac     660
   atcctgtcca aggacgaggg tgggcgtcac accccgttct actcgaacta ccgcgcgcag     720
   ttctacttcc gcaccaccga cgtcaccggc gtcatcacgc tgcccgaggg caccgagatg     780
   gtcatgcccg gcgacaccac cgagatgtcg gtcgagctca tccagccgat cgccatggag     840
   gagggcctcg gcttcgccat ccgcgagggt ggccgcaccg tgggctccgg c              891
<210> 160
   <211> 891
   <212> DNA
   <213> Mycobacterium tuberculosis
<400> 160
   aacatgatca ccggcgccgc gcagatggac ggtgcgatcc tggtggtcgc cgccaccgac      60
   ggcccgatgc cccagacccg cgagcacgtt ctgctggcgc gtcaagtggg tgtgccctac     120
   atcctggtag cgctgaacaa ggccgacgca gtggacgacg aggagctgct cgaactcgtc     180
   gagatggagg tccgcgagct gctggctgcc caggaattcg acgaggacgc cccggttgtg     240
   cgggtctcgg cgctcaaggc gctcgagggt gacgcgaagt gggttgcctc tgtcgaggaa     300
   ctgatgaacg cggtcgacga gtcgattccg gacccggtcc gcgagaccga caagccgttc     360
   ctgatgccgg tcgaggacgt cttcaccatt accggccgcg gaaccgtggt caccggacgt     420
   gtggagcgcg gcgtgatcaa cgtgaacgag gaagttgaga tcgtcggcat tcgcccatcg 480
   accaccaaga ccaccgtcac cggtgtggag atgttccgca agctgctcga ccagggccag 540
   gcgggcgaca acgttggttt gctgctgcgg ggcgtcaagc gcgaggacgt cgagcgtggc 600
   caggttgtca ccaagcccgg caccaccacg ccgcacaccg agttcgaagg ccaggtctac 660
   atcctgtcca aggacgaggg cggccggcac acgccgttct tcaacaacta ccgtccgcag 720
   ttctacttcc gcaccaccga cgtgaccggt gtggtgacac tgccggaggg caccgagatg 780
   gtgatgcccg gtgacaacac caacatctcg gtgaagttga tccagcccgt cgccatggac 840
   gaaggtctgc gtttcgcgat ccgcgagggt ggccgcaccg tgggcgccgg c 891
<210> 161
   <211> 891
   <212> DNA
   <213> Mycoplasma genitalium
<400> 161
   aatatgatca caggtgctgc acaaatggat ggagctattc tagttgtttc agcaactgat 60
   agtgtgatgc cccaaacccg cgagcacatc ttacttgccc gccaagtagg ggttcctaaa 120
   atggtagttt ttctaaacaa gtgtgatatt gctagtgatg aagaggtaca agaacttgtt 180
   gctgaagaag tacgtgatct gttaacttcc tatggttttg atggtaagaa cactcctatt 240
   atttatggct cagctttaaa agcattggaa ggtgatccaa agtgggaggc taagatccat 300
   gatttgatta aagcagttga tgaatggatt ccaactccta cacgtgaagt agataaacct 360
   ttcttattag caattgaaga tacgatgacc attactggta gaggtacagt tgttacagga 420
   agagttgaaa gaggtgaact caaagtaggt caagaagttg aaattgttgg tttaaaacca 480
   attagaaaag cagttgttac tggaattgaa atgttcaaaa aggaacttga ttcagcaatg 540
   gctggtgaca atgctggggt attattacgt ggtgttgaac gtaaagaagt tgaaagaggt 600
   caagttttag caaaaccagg ctctattaaa ccgcacaaga aatttaaagc tgagatctat 660
   gctttaaaga aagaagaagg tggtagacac actggttttt taaacggtta ccgtcctcaa 720
   ttctatttcc gtaccactga tgtaactggt tctattgctt tagctgaaaa tactgaaatg 780
   gttctacctg gtgataatgc ttctattact gttgagttaa ttgctcctat cgcttgtgaa 840
   aaaggtagta agttctcaat tcgtgaaggt ggtagaactg taggggcagg c 891
<210> 162
   <211> 891
   <212> DNA
   <213> Neisseria gonorrhoeae
<400> 162
   aacatgatta ccggcgccgc acaaatggac ggtgcaatcc tggtatgttc tgctgccgac 60
   ggccctatgc cgcaaacccg cgaacacatc ctgctggccc gtcaagtagg cgtaccttac 120
   atcatcgtgt tcatgaacaa atgcgacatg gtcgacgatg ccgagctgtt ccaactggtt 180
   gaaatggaaa tccgcgacct gctgtccagc tacgacttcc ccggcgacga ctgcccgatc 240
   gtacaaggtt ccgcactgaa agccttggaa ggcgatgccg cttacgaaga aaaaatcttc 300
   gaactggcta ccgcattgga cagatacatc ccgactcccg agcgtgccgt ggacaaacca 360
   ttcctgctgc ctatcgaaga cgtgttctcc atttccggcc gcggtaccgt agtcaccggc 420
   cgtgtagagc gaggtatcat ccacgttggt gacgagattg aaatcgtcgg tctgaaagaa 480
   acccaaaaaa ccacctgtac cggcgttgaa atgttccgca aactgctgga cgaaggtcag 540
   gcgggcgaca acgtaggcgt attgctgcgc ggtaccaaac gtgaagacgt agaacgcggt 600
   caggtattgg ccaaacgggg tactatcact cctcacacca agttcaaagc agaagtgtac 660
   gtattgagca aagaagaggg cggcccccat accccgtttt tcgccaacta ccgtccccaa 720
   ttctacttcc gtaccactga cgtaaccggc acgattactt tggaaaaagg tgtggaaatg 780
   gtaatgccgg gtgagaacgt aaccattact gtagaactga ttgcgcctat cgctatggaa 840
   gaaggtctgc gctttgcgat tcgcgaaggc ggccgtaccg tgggtgccgg c 891
<210> 163
   <211> 891
   <212> DNA
   <213> Rickettsia prowazekii
<400> 163
   aatatgataa ctggtgccgc tcagatggat ggtgctatat tagtagtttc tgctgctgat 60
   ggtcctatgc ctcaaactag agaacatata ttactggcaa aacaggtagg tgtacctgct 120
   atggtagtat ttttgaataa agtagatatg gtagatgatc ctgacctatt agaattagtt 180
   gagatggaag taagagaatt attatcaaaa tatggtttcc ctggtaatga aatacctatt 240
   attaaaggtt ctgcacttca agctttagaa ggaaaacctg aaggtgaaaa agctattaat 300
   gagttaatga atgcagtaga tacgtatata cctcagccta tagagctaca agataaacct 360
   tttttaatgc caatagagga tgtattttct atttcaggca gaggtaccgt tgtaactggt 420
   agagtggagt caggcataat taaggtgggt gaagaaattg aaatagtagg tctaaaaaat 480
   acgcaaaaaa cgacttgtac aggtgtagaa atgttcagaa aattacttga tgaaggacaa 540
   tctggagata atgtcggtat attactacgt ggtacaaaaa gagaagaagt agaaagagga 600
   caagtacttg caaaacctgg gagcataaaa ccgcatgata aatttgaagc tgaagtgtat 660
   gtgcttagta aagaggaagg tggacgtcat accccattta ctaatgatta tcgcccacag 720
   ttctatttta gaacaacaga tgttaccggc acaataaaat tgccttctga taagcagatg 780
   gttatgcctg gagataatgc tactttttca gtagaattaa ttaagccgat tgctatgcaa 840
   gaagggttaa aattctctat acgtgaaggt ggtagaacag taggagccgg t 891
<210> 164
   <211> 891
   <212> DNA
   <213> Salmonella typhimurium
<400> 164
   aacatgatca ccggtgctgc tcagatggac ggcgcgatcc tggttgttgc tgcgactgac 60
   ggcccgatgc cgcagacccg tgagcacatc ctgctgggtc gtcaggtagg cgttccgtac 120
   atcatcgtgt tcctgaacaa atgcgacatg gttgatgacg aagagctgct ggaactggtt 180
   gagatggaag ttcgcgaact gctgtctcag tacgacttcc cgggcgacga cactccgatc 240
   gttcgtggtt ctgctctgaa agcgctggaa ggcgacgcag agtgggaagc gaaaatcatc 300
   gaactggctg gcttcctgga ttcttatatt ccggaaccag agcgtgcgat tgacaagccg 360
   ttcctgctgc cgatcgaaga cgtattctcc atctccggtc gtggtaccgt tgttaccggt 420
   cgtgtagagc gcggtatcat caaagtgggc gaagaagttg aaatcgttgg tatcaaagag 480
   actcagaagt ctacctgtac tggcgttgaa atgttccgca aactgctgga cgaaggccgt 540
   gccggtgaga acgtaggtgt tctgctgcgt ggtatcaaac gtgaagaaat cgaacgtggt 600
   caggtactgg ctaagccggg caccatcaag ccgcacacca agttcgaatc tgaagtgtac 660
   attctgtcca aagatgaagg cggccgtcat actccgttct tcaaaggcta ccgtccgcag 720
   ttctacttcc gtactactga cgtgactggt accatcgaac tgccggaagg cgtagagatg 780
   gtaatgccgg gcgacaacat caaaatggtt gttaccctga tccacccgat cgcgatggac 840
   gacggtctgc gtttcgcaat ccgtgaaggc ggccgtaccg ttggcgcggg c 891
<210> 165
   <211> 881
   <212> DNA
   <213> Shewanella putida
<400> 165
   atgatcactg gtgctgcaca gatggacggc gcgattctgg tagtcgcttc aacagacggt      60
   ccaatgccac agactcgtga gcacatcctg ctttctcgtc aggttggcgt accattcatc     120
   atcgtattca tgaacaaatg tgacatggta gatgacgaag agctgttaga gctagttgag     180
   atggaagtgc gtgaactgtt atcagaatac gatttcccag gtgatgactt accggtaatc     240
   caaggttcag ctctgaaagc gctagaaggc gagccagagt gggaagcaaa aatccttgaa     300
   ttagcagcgg cgctggattc ttacattcca gaaccacaac gtgacatcga taagccgttc     360
   ctactgccaa tcgaagacgt attctcaatt tcaggccgtg gtacagtagt aacaggtcgt     420
   gttgagcgtg gtattgtacg cgtaggcgac gaagttgaaa tcgttggtgt acgtgcgaca     480
   actaagacaa cgtgtactgg tgtagaaatg ttccgtaaac tgcttgacga aggtcgtgca     540
   ggtgagaact gtggtatttt gttacgtggt actaagcgtg atgacgtaga acgtggtcaa     600
   gtattagcga agccaggttc aatcaaccca cacactactt ttgaatcaga agtttacgta     660
   ctgtcaaaag aagaaggtgg tcgtcacacg ccattcttca aaggctaccg tccacagttc     720
   tacttccgta caactgacgt aaccggtact atcgaactgc cagaaggcgt agagatggta     780
   atgccaggcg ataacatcaa gatggtagtg acactgattt gcccaatcgc gatggacgaa     840
   ggtttacgct tcgcaatccg tgaaggcggt cgtacagtgg t                         881
<210> 166
   <211> 897
   <212> DNA
   <213> Stigmatella aurantiaca
<400> 166
   aacatgatca cgggcgcggc gcagatggac ggagcgattc tggtggtgtc cgcggccgac      60
   ggcccgatgc cccagacgcg tgagcacatc ctgctggcca ggcaggtggg cgtgccctac     120
   atcgtcgtct tcctgaacaa ggtggacatg ctggacgatc cggagctgcg cgagctggtg     180
   gagatggagg tgcgcgacct gctcaagaag tacgagttcc cgggcgacag catccccatc     240
   atccctggca gcgcgctcaa ggcgctggag ggagacacca gcgacatcgg cgagggagcg     300
   atcctgaagc tgatggcggc ggtggacgag tacatcccga cgccgcagcg tgcgacggac     360
   aagccgttcc tgatgccggt ggaagacgtg ttctccatcg caggccgagg aacggtggcg     420
   acgggccgag tggagcgcgg caagatcaag gtgggcgagg aagtggagat cgtggggatc     480
   cgtccgacgc agaagacggt catcacgggg gtggagatgt tccgcaagct gctggacgag     540
   ggcatggcgg gagacaacat cggagcgctg ctgcgaggcc tgaagcgcga ggacctggag 600
   cgtgggcagg tgctggcgaa ctgggggagc atcaacccgc acacgaagtt caaggcgcag 660
   gtgtacgtgc tgtcgaagga agagggaggg cggcacacgc cgttcttcaa gggataccgg 720
   ccgcagttct acttccggac gacggacgtg accggaacgg tgaagctgcc ggacaacgtg 780
   gagatggtga tgccgggaga caacatcgcc atcgaggtgg agctcattac tccggtcgcc 840
   atggagaagg agctgccgtt cgccatccgt gagggtggcc gcacggtggg cgccggc 897
<210> 167
   <211> 894
   <212> DNA
   <213> Streptococcus pyogenes
<400> 167
   aacatgatca ctggtgccgc tcaaatggac ggagctatcc ttgtagttgc ttcaactgat 60
   ggaccaatgc cacaaactcg tgagcacatc cttctttcac gtcaggttgg tgttaaacac 120
   cttatcgtgt tcatgaacaa agttgacctt gttgatgacg aagagttgct tgaattagtt 180
   gagatggaaa ttcgtgacct tctttcagaa tacgatttcc caggtgatga ccttccagtt 240
   atccaaggtt cagctcttaa agctcttgaa ggcgacacta aatttgaaga catcatcatg 300
   gaattgatgg atactgttga ttcatacatt ccagaaccag aacgcgacac tgacaaacca 360
   ttgcttcttc cagtcgaaga cgtattctca attacaggtc gtggtacagt tgcttcagga 420
   cgtatcgacc gtggtactgt tcgtgtcaac gacgaaatcg aaatcgttgg tatcaaagaa 480
   gaaactaaaa aagctgttgt tactggtgtt gaaatgttcc gtaaacaact tgacgaaggt 540
   cttgcaggag acaacgtagg tatccttctt cgtggtgttc aacgtgacga aatcgaacgt 600
   ggtcaagtta ttgctaaacc aagttcaatc aacccacaca ctaaattcaa aggtgaagta 660
   tatatccttt ctaaagacga aggtggacgt cacactccat tcttcaacaa ctaccgtcca 720
   caattctact tccgtacaac tgacgtaaca ggttcaatcg aacttccagc aggtacagaa 780
   atggttatgc ctggtgataa cgtgacaatc aacgttgagt tgatccaccc aatcgccgta 840
   gaacaaggta ctactttctc aatccgtgaa ggtggacgta ctgttggttc aggt 894
<210> 168
   <211> 897
   <212> DNA
   <213> Thiobacillus cuprinus
<400> 168
   aacatgatca ccggtgcggc ccagatggac ggcgccatcc tggtcgtgtc cgccgccgac 60
   ggccccatgc cccaaacccg cgagcacatc ctgctggcgc gtcaggtggg cgtgccctac 120
   atcatcgtgt tcctcaacaa gtgcgacatg gtcgacgacg ccgagctgct cgaactcgtc 180
   gagatggaag tgcgcgagct gctgtccaag tacgacttcc ccggtgacga cacccccatc 240
   atcaagggct cggccaagct ggccctcgaa ggcgacaagg gcgaactggg cgaaggcgcc 300
   attctcaagc tggccgaggc cctggacacc tacatcccca cgcccgagcg ggccgtcgac 360
   ggcgcgttcc tcatgcccgt ggaagacgtg ttctccatct ccgggcgcgg cacggtggtc 420
   accgggcgtg tggagcgcgg catcatcaag gtcggcgagg aaatcgagat tgtcggcctc 480
   aagcccaccc tcaagaccac ctgcaccggc gtggaaatgt tcaggaagct gctcgaccag 540
   ggccaggccg gcgacaacgt cggcatcttg ctgcgcggca ccaagcgcga ggaagtcgag 600
   cgcggccagg tgctgtgcaa acccggctcg atcaagcccc acacccactt caccgccgag 660
   gtgtacgtgc tgagcaagga cgagggcggc cgccacaccc ccttcttcaa caactaccgc 720
   ccgcagttct acttccgcac caccgacgtc accggcgcca tcgaactgcc caaggacaag 780
   gaaatggtca tgcccggcga taatgtgagc atcaccgtca agctcatcgc ccccatcgcc 840
   atggaagaag gcctgcgctt cgccatccgc gaaggcggcc gcaccgtcgg cgccggc 897
<210> 169
   <211> 894
   <212> DNA
   <213> Treponema pallidum
<400> 169
   aatatgatca cgggtgctgc gcagatggac ggtggtattc tcgtcgtgtc tgcgcctgac 60
   ggcgttatgc cacagacgaa ggagcatctt ctgctcgccc gtcaggttgg tgttccctcc 120
   atcattgttt ttttgaacaa ggttgatttg gttgatgatc ctgagttgct agagctggtg 180
   gaagaagagg tgcgtgatgc gcttgctgga tatgggtttt cgcgtgagac gcctatcgtc 240
   aaggggtctg cgtttaaagc tctgcaggat ggcgcttccc cggaggatgc agcttgtatt 300
   gaggaactgc ttgcggccat ggattcctac tttgaagacc cagtgcgtga cgacgcaaga 360
   cctttcttgc tctctatcga ggatgtgtac actatttctg ggcgtggtac cgttgtcacg 420
   gggcgcatcg aatgtggggt aattagtctg aatgaagagg tcgagatcgt cgggattaag 480
   cccactaaga aaacagtggt tactggcatt gagatgttta ataagttgct tgatcaggga 540
   attgcaggtg ataacgtggg gctgcttttg cgcggggtgg ataaaaaaga ggttgagcgc 600
   ggtcaggtgc tttctaagcc cggttctatt aagccacaca ccaagtttga ggcgcagatc 660
   tacgtgctct ctaaggaaga gggtggccgt cacagtcctt tttttcaagg ttatcgtccg 720
   cagttttatt ttagaactac tgacattacc ggtacgattt ctcttcctga aggggtagac 780
   atggtgaagc cgggggataa caccaagatt ataggtgagc tcatccaccc gatagctatg 840
   gacaagggtc tgaagcttgc gattcgtgaa ggggggcgca ctattgcttc tggt 894
<210> 170
   <211> 891
   <212> DNA
   <213> Ureaplasma urealyticum
<400> 170
   aatatgatta caggggcagc acaaatggat ggagcaattt tagttattgc tgcatctgat 60
   ggggttatgg ctcaaactaa agaacatatt ttattagcac gtcaagttgg tgttccaaaa 120
   atcgttgttt tcttaaacaa atgtgatttc atgacagatc cagatatgca agatcttgtt 180
   gaaatggaag ttcgtgaatt attatctaaa tatggatttg atggcgataa cacaccagtt 240
   attcgtggtt caggtcttaa ggctttagaa ggagatccag tttgagaagc aaaaattgat 300
   gaattaatgg acgcagttga ttcatgaatt ccattaccag aacgtagtac tgacaaacca 360
   ttcttattag caattgaaga tgtattcaca atttcaggac gtggtacagt agtaactgga 420
   cgtgttgaac gtggtgtatt aaaagttaat gatgaggttg aaattgttgg tctaaaagac 480
   actcaaaaaa ctgttgttac aggaattgaa atgtttagaa aatcattaga tcaagctgaa 540
   gctggtgata atgctggtat tttattacgt ggtattaaaa aagaagatgt tgaacgtggt 600
   caagtacttg taaaaccagg atcaattaaa cctcaccgta cttttactgc taaagtttat 660
   attcttaaaa aagaagaagg tggacgtcat acacctattg tttcaggata ccgtccacaa 720
   ttctatttta gaacaacaga tgtaacaggt gctatttcat tacctgctgg tgttgatttg 780
   gttatgccag gtgatgacgt tgaaatgact gtagaattaa ttgctccagt tgcgattgaa 840
   gatggatcta aattctcaat ccgtgaaggt ggtaaaactg taggtcatgg t 891
<210> 171
   <211> 909
   <212> DNA
   <213> Wolinella succinogenes
<400> 171
   aacatgatta caggtgctgc tcaaatggat ggcgcgattc ttgttgtttc tgcggcggat 60
   ggccccatgc cccaaactag ggagcacatt cttctttctc gacaagtagg cgttccttac 120
   atcgtggttt tcttgaacaa agaagatatg gttgatgacg ctgagcttct tgagcttgtt 180
   gaaatggaag ttagagaact tcttagcaac tacgacttcc ctggagatga cactcctatc 240
   gttgcaggtt ccgctcttaa agctcttgaa gaggctaacg accaggaaaa tgttggcgag 300
   tggggcgaga aagtattgaa gcttatggct gaggttgacc gatatattcc tacgcctgag 360
   cgagatgtgg ataagccttt ccttatgcct gttgaagacg tattctccat cgcgggtcgt 420
   ggaaccgttg tgacaggaag aattgaaaga ggcgtggtta aagtcggtga cgaagtagaa 480
   atcgttggta tccgaaacac acaaaaaaca accgtaactg gcgttgagat gttccgaaaa 540
   gagctcgaca agggtgaggc gggtgacaac gttggtgttc ttttgagagg caccaagaaa 600
   gaagatgttg agagaggtat ggttctttgt aaaataggtt ctatcactcc tcacactaac 660
   tttgaaggtg aagtttacgt tctttccaaa gaggaaggcg gacgacacac tccattcttc 720
   aatggatacc gacctcagtt ctatgttaga actacagacg ttaccggttc tatctctctt 780
   cctgagggcg tagagatggt tatgcctggt gacaacgtta agatcaatgt tgagcttatc 840
   gctcctgtag ccctcgaaga gggaacacga ttcgcgatcc gtgaaggtgg tcgaaccgtt 900
   ggtgcgggt 909
<210> 172
   <211> 26
   <212> DNA
   <213> Artificial Sequence
<220>
   <223> Oligonucleotide that binds to any fungal species (tuf gene)
<220>
   <221> misc_feature
   <222> (6)..(6)
   <223> n = inosine
<220>
   <221> misc_feature
   <222> (12)..(12)
   <223> n = inosine
<220>
   <221> misc_feature
   <222> (18)..(18)
   <223> n = inosine
<400> 172
   tartcngtra angcytcnac rcacat 26
<210> 173
   <211> 21
   <212> DNA
   <213> Artificial Sequence
<220>
   <223> Oligonucleotide that binds to antibiotic resistance gene of Enterococcus
   spp.
<400> 173
   tctttagcag aacaggatga a 21
<210> 174
   <211> 20
   <212> DNA
   <213> Artificial Sequence
<220>
   <223> Oligonucleotide that binds to antibiotic resistance gene of Enterococcus
   spp.
<400> 174
   gaataattcc atatcctccg 20